# EUROPEAN PATENT APPLICATION

(11) **EP 1 541 557 A1**
(43) Date of publication of application: **15.06.2005**
(21) Application number: 03788085.3
(22) Date of filing: 12.08.2003
(51) Int. Cl.: C07D 213/75, A01N 43/40

(54) **PHENYLPYRIDINE COMPOUND AND BACTERICIDAL COMPOSITION CONTAINING THE SAME**

(30) Priority: 19.08.2002 JP 2002237942
(71) Applicant: Sumitomo Chemical Company, Limited, Tokyo 104-8260 (JP)
(72) Inventor: KOMORI, Takashi, Toyonaka-shi, Osaka 561-0802 (JP); SAKAGUCHI, Hiroshi, Toyonaka-shi, Osaka 561-0802 (JP)
(74) Representative: Henkel, Feiler & Hänzel
(86) International application number: PCT/JP2003/010246
(87) International publication number: WO 2004/016594

(57) **Abstract**

A phenylpyridine compound represented by the formula has an excellent controlling activity against plant diseases: 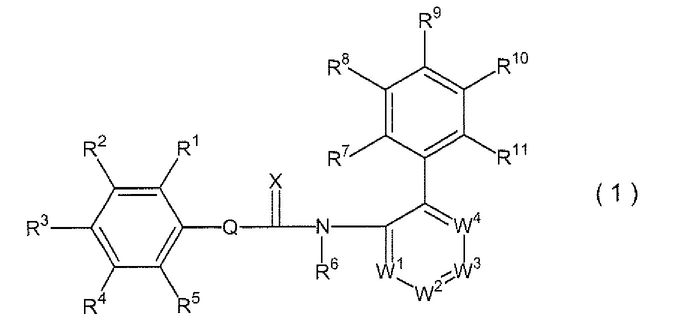 [, wherein, in the formula, R¹, R², R³, R⁴ and R⁵ independently represent a hydrogen atom, a halogen atom and the like; R⁶ represents a hydrogen atom or a C1-C3 alkyl group; R⁷, R⁸ and R¹¹ independently represent a hydrogen atom, a halogen atom and the like; R⁹ and R¹⁰ independently represent a hydroxyl group and the like; W¹―W²=W³ ―W⁴ represents N―CH=CH―CH and the like; X represents an oxygen atom and the like; and Q represents a (C1-C6 alkoxy)methylene and the like].

## Description

### TECHNICAL FIELD

The present invention relates a phenylpyridine compound and a fungicidal composition comprising the same.

### BACKGROUND ART

Various compounds having controlling activity against plant diseases are conventionally developed and provided in practical use as an active ingredient of the fungicidal composition, however sometimes their controlling activity are not always enough.

### DISCLOSURE OF THE INVENTION

The inventor (s) of the present invention, after intensively studying, has found that a phenylpyridine compound (1) represented by the following formula has an excellent controlling activity against plant diseases and accomplished the present invention.

The present invention provides:
[Invention 1] a phenylpyridine compound (1) 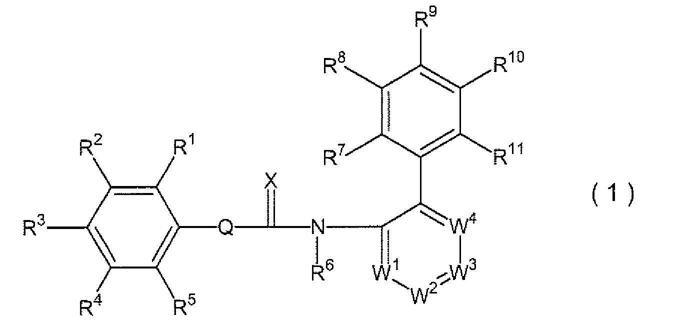 [, wherein, in the formula, R¹, R², R³, R⁴ and R⁵ independently represent a hydrogen atom, a halogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C1-C6 alkylthio group, a C1-C6 haloalkylthio group, a C3-C6 cycloalkyl group, a C3-C6 cycloalkoxy group or a cyano group; both of R² and R³ may be combined to represent a trimethylene, a tetramethylene or ―CH=CH―CH=CH―;
   R⁶ represents a hydrogen atom or a C1-C3 alkyl group; R⁷, R⁸ and R¹¹ independently represent a hydrogen atom, a halogen atom or a C1-C3 alkyl group;
   R⁹ and R¹⁰ independently represent a hydroxyl group, a halogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C2-C6 cyanoalkyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group, a C1-C6 alkylthio group, a C1-C6 haloalkylthio group, a C3-C6 cycloalkyl group, a C3-C6 cycloalkoxy group, a nitro group, a benzyl group or a cyano group; W¹―W²=W³ ―W⁴ represents N―CR²¹=CR²²―CR²³, CR²⁴―N=CR²⁵―CR²⁶, CR²⁷―CR²⁸=N― CR²⁹ or CR³⁰―CR³¹=CR³²―N
   {in which R²¹, R²², R²³, R²⁴, R²⁵, R²⁶, R²⁷, R²⁸, R²⁹, R³⁰, R³¹ and R³² independently represent a hydrogen atom, a halogen atom, a C1-C3 alkyl group, a C1-C3 alkoxy group, a C1-C3 haloalkyl group}:
   X represents an oxygen atom or a sulfur atom;
   Q represents a group illustrated by the following formulas of Q1 or Q2 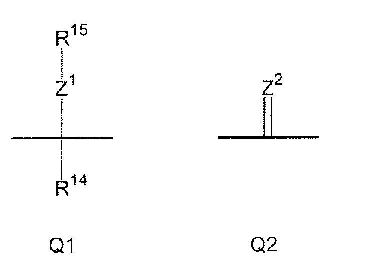 {in which R¹⁴ represents a hydrogen atom or a C1-C3 alkyl group, R¹⁵ represents a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group, a C3-C6 cycloalkyl group, a (C1-C6 alkyl)carbonyl group, a (C1-C6 haloalkyl)carbonyl group, a (C1-C6 alkoxy)carbonyl group, a (C1-C6 haloalkoxy) carbonyl group, a (C3-C6 alkenyloxy) carbonyl group, a (C3-C6 haloalkenyloxy)carbonyl group, a (C3-C6 alkynyloxy)carbonyl group, a (C3-C6 haloalkynyloxy) carbonyl group or a C1-C3 alkylsulfonyl group,
   Z¹ represents an oxygen atom or a sulfur atom,
   Z² represents an oxygen atom, NOR¹⁶ (in which R¹⁶ represents a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group), CR¹⁷R¹⁸ (in which R¹⁷ represents a halogen atom, a C1-C6 alkyl group, a C1-C6 haloalkyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group or a C3-C6 cycloalkyloxy group and R¹⁸ represents a hydrogen atom, a halogen atom, a C1-C6 alkyl group or a C1-C6 haloalkyl group) or NNR¹⁹R²⁰ (in which R¹⁹ and R²⁰ independently represent a hydrogen atom, a C1-C6 alkyl group, a C1-C6 haloalkyl group, a C3-C6 alkenyl group, a C3-C6 haloalkenyl group, a C3-C6 alkynyl group or a C3-C6 cycloalkyl group)}];
   (referred to as "the compound of the present invention" hereinafter.);
[Invention 2] the phenylpyridine compound (1) according to Invention 1, wherein X is an oxygen atom;
[Invention 3] the phenylpyridine compound (1) according to any one of Invention 1 or 2, wherein R⁶ is a hydrogen atom;
[Invention 4] the phenylpyridine compound (1) according to any one of Invention 1 to 3, wherein Q is Q1, R¹⁴ is a hydrogen atom and Z¹ is an oxygen atom;
[Invention 5] the phenylpyridine compound (1) according to Invention 4, wherein R¹⁵ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group;
[Invention 6] the phenylpyridine compound (1) according to any one of Invention 1 to 3, wherein Q is Q2 and Z² is NOR¹⁶ (in which R¹⁶ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group);
[Invention 7] the phenylpyridine compound (1) according to any one of Invention 1 to 6, wherein R¹, R⁴ and R⁵ are hydrogen atoms;
[Invention 8] the phenylpyridine compound (1) according to any one of Invention 1 to 6, wherein R¹, R⁴ and R⁵ are hydrogen atoms and R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group;
[Invention 9] the phenylpyridine compound (1) according to any one of Invention 1 to 6, wherein R¹, R², R⁴ and R⁵ are hydrogen atoms;
[Invention 10] the phenylpyridine compound (1) according to any one of Invention 1 to 9, wherein each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group;
[Invention 11] the phenylpyridine compound (1) according to any one of Invention 1 to 9, wherein each of R⁹ and R¹⁰ is a C1-C4 alkoxy group;
[Invention 12] the phenylpyridine compound (1) according to any one of Invention 1 to 9, wherein R⁹ and R¹⁰ are methoxy groups;
[Invention 13] the phenylpyridine compound (1) according to any one of Invention 1 to 12, wherein R⁷, R⁸ and R¹¹ are hydrogen atoms;
[Invention 14] the phenylpyridine compound (1) according to any one of claim 1 to 13, wherein W¹―W²=W³―W⁴ is N―CH=CH―CH, CH-N=CH-CH, CH―CH=N―CH or CH-CH=CH-N;
[Invention 15] a fungicidal composition comprising the phenylpyridine compound (1) according to any one of Invention 1 to 14 and a carrier; and
[Invention 16] a method for controlling plant diseases comprising applying of the phenylpyridine compound (1) according to any one of Invention 1 to 14 for plants or soils.

In the representation of R¹, R², R³, R⁴ and R⁵, the halogen atom includes a fluorine atom, a chlorine atom, a bromine atom and the like;
the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a fluoromethyl group, a difluoromethyl group, a trifluoromethyl group, a chlorodifluoromethyl group, a chlorofluoromethyl group, a bromodifluoromethyl group, a trichloromethyl group, a dichlorobromomethyl group, a pentafluoroethyl group, a 2,2,2-trifluoroethyl group, a 1,1-difluoroethyl group, a 2,2-difluoroethyl group, a 2-fluoroethyl group, a 6,6,6-trifluorohexyl group and the like;
the C2-C6 alkenyl group includes a vinyl group, a 1-propenyl group, a 2-propenyl group, a 1-methyl-1-propenyl group, a 1-methyl-2-propenyl group, a 2-methyl-1-propenyl group, a 2-methyl-2-propenyl group, a 1-butenyl group, a 2-butenyl group, a 3-butenyl group, a 1-hexenyl group, a 2-hexenyl group, a 5-hexenyl group and the like;
the C2-C6 haloalkenyl group includes a 1-chlorovinyl group, a 2-chlorovinyl group, a 2,2-dichlorovinyl group, a 2,2-difluorovinyl group, a 1,2-dichlorovinyl group, a 3,3-dichloro-2-propenyl group, a 3,3-difluoro-2-propenyl group and the like;
the C2-C6 alkynyl group includes an ethynyl group, a 2-propynyl group, a 3-butynyl group, a 3-hexynyl group, a 5-hexynyl group and the like;
the C2-C6 haloalkynyl group includes a 2-chloroethynyl group, a 2-bromoethynyl group, a 3-chloro-2-propynyl group, a 3-bromo-2-propynyl group, a 6-chloro-5-hexynyl group and the like; the C1-C6 alkoxy group includes a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group and the like;
the C1-C6 haloalkoxy group includes a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, a 2,2-dichlorohexyloxy group and the like;
the C3-C6 alkenyloxy group includes a 2-propenyloxy group, a 1-methyl-2-propenyloxy group, a 2-methyl-2-propenyloxy group, a 2-butenyloxy group, a 3-butenyloxy group, a 2-hexenyloxy group, a 5-hexenyloxy group and the like;
the C3-C6 haloalkenyloxy group includes a 3,3-dichloro-2-propenyloxy group, a 3,3-difluoro-2-propenyloxy group, a 3,3-dibromo-2-propenyloxy group, a 2,3-dichloropropenyloxy group, a 6-fluoro-2-hexenyloxy group, a 2,2-dichloro-5-hexenyloxy group and the like;
the C3-C6 alkynyloxy group includes a 2-propynyloxy group, a 1-methyl-2-propynyloxy group, a 2-butynyloxy group, a 3-butynyloxy group, a 2-hexynyloxy group, a 5-hexynyloxy group and the like;
the C3-C6 haloalkynyloxy group includes a 3-chloro-2-propynyloxy group, a 3-bromo-2-propynyloxy group, a 3-iodo-2-propynyloxy group, a 6-chloro-5-hexynyloxy group and the like;
the C1-C6 alkylthio group includes a methylthio group, an ethylthio group, a propylthio group, an isopropylthio group, a butylthio group, an isobutylthio group, a sec-butylthio group, a
tert-butylthio group, a pentylthio group, an isopentylthio group, a hexylthio group and the like;
the C1-C6 haloalkylthio group includes a trifluoromethylthio group, a difluoromethylthio group, a bromodifluoromethylthio group, a chlorodifluoromethylthio group, a fluoromethylthio group, a 2,2,2-trifluoroethylthio group, a 1,1,2,2-tetrafluoroethylthio group, a 5-chloropentylthio group, a 4-fluoroisopentylthio group, a 2,2-dichlorohexylthio group and the like;
the C3-C6 cycloalkyl group includes a cyclopropyl group, a cyclopentyl group, a cyclohexyl group and the like; and
the C3-C6 cycloalkoxy group includes a cyclopropoxy group, a cyclopentyloxy group, a cyclohexyloxy group and the like.

In the representation of R⁶, the C1-C3 alkyl group includes a methyl group, an ethyl group, a propyl group and the like.

In the representation of R⁷, R⁸ and R¹¹, the halogen atom includes a fluorine atom, a chlorine atom, a bromine atom and the like.
the C1-C3 alkyl group includes a methyl group, an ethyl group, a propyl group and the like.

In the representation of R⁹ and R¹⁰, the halogen atom includes a fluorine atom, a chlorine atom, a bromine atom and the like; the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a fluoromethyl group, a difluoromethyl group, a trifluoromethyl group, a chlorodifluoromethyl group, a chlorofluoromethyl group, a bromodifluoromethyl group, a trichloromethyl group, a dichlorobromomethyl group, a pentafluoroethyl group, a 2,2,2-trifluoroethyl group, a 2,2-difluoroethyl group, a 2-fluoroethyl group, a 6,6,6-trifluorohexyl group and the like; the C2-C6 alkenyl group includes a vinyl group, a 1-propenyl group, a 2-propenyl group, a 1-methyl-1-propenyl group, a 1-methyl-2-propenyl group, a 2-methyl-1-propenyl group, a 2-methyl-2-propenyl group, a 1-butenyl group, a 2-butenyl group, a 3-butenyl group, a 1-hexenyl group, a 2-hexenyl group, a 5-hexenyl group and the like;
the C2-C6 haloalkenyl group includes a 1-chlorovinyl group, a 2-chlorovinyl group, a 2,2-dichlorovinyl group, a 2,2-difluorovinyl group, a 1,2-dichlorovinyl group, a 3, 3-dichloropropenyl group, a 3,3-difluoropropenyl group and the like;
the C2-C6 alkynyl group includes an ethynyl group, a 2-propynyl group, a 3-butynyl group, a 5-hexynyl group and the like;
the C2-C6 haloalkynyl group includes a 2-chloroethynyl group, a 2-bromoethynyl group, and a 6-chloro-5-hexynyl group and the like;
the C2-C6 cyanoalkyl group includes a cyanomethyl group, a 1-cyanoethyl group, a 2-cyanoethyl group and the like;
the C1-C6 alkoxy group includes a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group and the like;
the C1-C6 haloalkoxy group includes a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, a 2,2-dichlorohexyloxy group and the like;
the C3-C6 alkenyloxy group includes a 2-propenyloxy group, a 1-methyl-2-propenyloxy group, a 2-methyl-2-propenyloxy group, a 2-butenyloxy group, a 3-butenyloxy group, a 2-hexenyloxy group, a 5-hexenyloxy group and the like;
the C3-C6 haloalkenyloxy group includes a 3-chloro-2-propynyloxy group, a 3-bromo-2-propynyloxy group, a 3-iodo-2-propynyloxy group, a 6-chloro-5-hexenyloxy group and the like;
the C3-C6 alkynyloxy group includes a 2-propynyloxy group, a 1-methyl-2-propynyloxy group, a 2-butynyloxy group, a 3-butynyloxy group, a 2-hexynyloxy group, a 5-hexynyloxy group and the like;
the C3-C6 haloalkynyloxy group includes a 3-chloro-2-propynyloxy group, a 3-bromo-2-propynyloxy group, a 3-iodo-2-propynyloxy group, a 6-chloro-5-hexynyloxy group and the like;
the C2-C6 cyanoalkyloxy group includes a cyanomethyloxy group, a 1-cyanoethyloxy group, a 2-cyanoethyloxy group and the like;
the C1-C6 alkylthio group includes a methylthio group, an ethylthio group, a propylthio group, an isopropylthio group, a butylthio group, an isobutylthio group, a sec-butylthio group, a
tert-butylthio group, a pentylthio group, an isopentylthio group, a hexylthio group and the like;
the C1-C6 haloalkylthio group includes a trifluoromethylthio group, a difluoromethylthio group, a bromodifluoromethylthio group, a chlorodifluoromethylthio group, a fluoromethylthio group, a 2,2,2-trifluoroethylthio group, a 1,1,2,2-tetrafluoroethylthio group, a 5-chloropentylthio group, a 4-f luoroisopentylthio group, a 2,2-dichlorohexylthio group and the like;
the C3-C6 cycloalkyl group includes a cyclopropyl group, a cyclopentyl group, a cyclohexyl group and the like; and
the C3-C6 cycloalkyloxy group includes a cyclopropyloxy group, a cyclopentyloxy group, a cyclohexyloxy group and the like.

In the representation of R²¹, R²², R²³, R²⁴, R²⁵, R²⁶, R²⁷, R²⁸, R²⁹, R³⁰, R³¹ and R³², the halogen atom includes a fluorine atom, a chlorine atom, a bromine atom and the like;
the C1-C3 alkyl group includes a methyl group, an ethyl group, a propyl group and the like;
the C1-C3 alkoxy group includes a methoxy group, an ethoxy group, a propoxy group and the like; and
the C1-C3 haloalkyl group includes a fluoromethyl group, a difluoromethyl group, a trifluoromethyl group, a chloromethyl group, a bromomethyl group, a 2,2,2-trifluoroethyl group, a trichloromethyl group and the like.

In the representation of R¹⁴, the C1-C3 alkyl group includes a methyl group, an ethyl group, a propyl group and the like.

In the representation of R¹⁵, the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a fluoromethyl group, a difluoromethyl group, a trifluoromethyl group, a trichloromethyl group, a chlorofluoromethyl group, a bromodifluoromethyl group, a 2-fluoroethyl group, a 2,2-difluoroethyl group, a 2, 2,2-trifluoroethyl group, a 6, 6, 6-trifluorohexyl group and the like;
the C3-C6 alkenyl group includes a 2-propenyl group, a 1-methyl-2-propenyl group, a 1,1-dimethyl-2-propenyl group, a 2-methyl-2-propenyl group, a 2-butenyl group, a 3-butenyl group, a 2-hexenyl group, a 5-hexenyl group and the like;
the C3-C6 haloalkenyl group includes a 3,3-dichloro-2-propenyl group, a 2,3-dichloro-2-propenyl group, a 3,3-difluoro-2-propenyl group and the like;
the C3-C6 alkynyl group includes a 2-propynyl group, a 1-methyl-2-propynyl group, a 1,1-dimethyl-2-propynyl group, a 2-butynyl group, a 3-butynyl group, a 2-hexynyl group, a 5-hexynyl group and the like;
the C3-C6 haloalkynyl group includes a 3-chloro-2-propynyl group, a 3-bromo-2-propynyl group, a 6-chloro-5-hexynyl group and the like;
the C3-C6 cycloalkyl group includes a cyclopropyl group, a cyclopentyl group, a cyclohexyl group and the like;
the (C1-C6 alkyl)carbonyl group includes an acetyl group, an ethylcarbonyl group, a propylcarbonyl group, an isopropylcarbonyl group, a butylcarbonyl group, a isobutylcarbonyl group, a sec-butylcarbonyl group, a tert-butylcarbonyl group, a pentylcarbonyl group, an isopentylcarbonyl group, a hexylcarbonyl group and the like;
the (C1-C6 haloalkyl) carbonyl group includes a chloroacetyl group, a bromoacetyl group, a difluoroacetyl group, a trifluoroacetyl group, a trichloroacetyl group, a chlorofluoroacetyl group, a bromodifluoroacetyl group, a 2-fluoroethylcarbonyl group, a 2,2-difluoroethylcarbonyl group, a 2,2,2-trifluoroethylcarbonyl group, a 6,6,6-trifluorohexylcarbonyl group and the like;
the (C1-C6 alkoxy) carbonyl group includes a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group, a tert-butoxycarbonyl group, a pentyloxycarbonyl group, an isopentyloxycarbonyl group, a hexyloxycarbonyl group and the like;
the (C1-C6 haloalkoxy)carbonyl group includes a 2,2,2-trifluoroethoxycarbonyl group, a 1,1,2,2-tetrafluoroethoxycarbonyl group, a 5-chloropentyloxycarbonyl group, a 2,2-dichlorohexyloxycarbonyl group and the like;
the (C3-C6 alkenyloxy)carbonyl group includes a 2-propenyloxycarbonyl group, a 1-methyl-2-propenyloxycarbonyl group, a 2-methyl-2-propenyloxycarbonyl group, a 2-butenyloxycarbonyl group, a 3-butenyloxycarbonyl group, a 2-hexenyloxycarbonyl group, a 5-hexenyloxy group carbonyl and the like;
the (C3-C6 haloalkenyloxy)carbonyl group includes a 3-chloro-2-propynyloxycarbonyl group, a 3-bromo-2-propynyloxycarbonyl group, a 3-iodo-2-propynyloxycarbonyl group, a 6-chloro-5-hexenyloxycarbonyl group and the like;
the (C3-C6 alkynyloxy)carbonyl group includes a 2-propynyloxycarbonyl group, a 1-methyl-2-propynyloxycarbonyl group, a 2-butynyloxycarbonyl group, a 3-butynyloxycarbonyl group, a 2-hexynyloxycarbonyl group, a 5-hexynyloxycarbonyl group and the like;
the (C3-C6 haloalkynyloxy)carbonyl group includes a 3-chloro-2-propynyloxycarbonyl group, a 3-bromo-2-propynyloxycarbonyl group, a 3-iodo-2-propynyloxycarbonyl group, a 6-chloro-5-hexynyloxycarbonyl group and the like; and
the C1-C3 alkylsulfonyl group includes a methanesulfonyl group, an ethanesulfonyl group, or the like.

In the representation of R¹⁶, the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a fluoromethyl group, a difluoromethyl group, a 2-fluoroethyl group, a 2, 2-difluoroethyl group, a 2,2,2-trifluoroethyl group, a 6, 6, 6-trif luorohexyl group and the like;
the C3-C6 alkenyl group includes a 2-propenyl group, a 1-methyl-2-propenyl group, a 2-methyl-2-propenyl group, a 2-butenyl group, a 3-butenyl group, a 2-hexenyl group, a 5-hexenyl group and the like;
the C3-C6 haloalkenyl group includes a 2,3-dichloropropenyl group, a 3,3-dichloropropenyl group, a 3,3-difluoropropenyl group and the like;
the C3-C6 alkynyl group includes a 2-propynyl group, a 1-methyl-2-propynyl group, a 1,1-dimethyl-2-propynyl group, a 2-butynyl group, a 3-butynyl group, a 2-hexynyl group, a 5-hexynyl group and the like;
the C3-C6 haloalkynyl group includes a 3-chloro-2-propynyl group, a 3-bromo-2-propynyl group, a 6-chloro-5-hexynyl group and the like; and
the C3-C6 cycloalkyl group includes a cyclopropyl group, a cyclobutyl group, a cyclopentyl group, a cyclohexyl group and the like.

In the representation of R¹⁷, the halogen atom includes a fluorine atom, a chlorine atom, a bromine atom and the like; the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a fluoromethyl group, a difluoromethyl group, a trifluoromethyl group, a chlorofluoromethyl group, a bromodifluoromethyl group, a 2-fluoroethyl group, a 2,2-difluoroethyl group, a 2,2,2-trifluoroethyl group, a 6,6,6-trifluorohexyl group and the like;
the C1-C6 alkoxy group includes a methoxy group, an ethoxy group, a propoxy group, an isopropoxy group, a butoxy group, an isobutoxy group, a sec-butoxy group, a tert-butoxy group, a pentyloxy group, an isopentyloxy group, a hexyloxy group and the like;
the C1-C6 haloalkoxy group includes a trifluoromethoxy group, a difluoromethoxy group, a bromodifluoromethoxy group, a chlorodifluoromethoxy group, a fluoromethoxy group, a 2,2,2-trifluoroethoxy group, a 1,1,2,2-tetrafluoroethoxy group, a 5-chloropentyloxy group, a 4-fluoroisopentyloxy group, a 2,2-dichlorohexyloxy group and the like;
the C3-C6 alkenyloxy group includes a 2-propenyloxy group, a 1-methyl-2-propenyloxy group, a 2-methyl-2-propenyloxy group, a 2-butenyloxy group, a 3-butenyloxy group, a 2-hexenyloxy group, a 5-hexenyloxy group and the like;
the C3-C6 haloalkenyloxy group includes a 3, 3-dichloro-2-propynyloxy group, a 3-chloro-2-propynyloxy group, a 3-bromo-2-propynyloxy group, a 3-iodo-2-propynyloxy group, a 6-chloro-5-hexenyloxy group and the like;
the C3-C6 alkynyloxy group includes a 2-propynyloxy group, a 1-methyl-2-propynyloxy group, a 2-butynyloxy group, a 3-butynyloxy group, a 2-hexynyloxy group, a 5-hexynyloxy group and the like; and
the C3-C6 haloalkynyloxy group includes a 3-chloro-2-propynyloxy group, a 3-bromo-2-propynyloxy group, a 3-iodo-2-propynyloxy group, a 6-chloro-5-hexynyloxy group and the like;
the C3-C6 cycloalkyloxy group includes a cyclopropyloxy group, a cyclobutyloxy group, a cyclopentyloxy group, a cyclohexyloxy group and the like.

In the representation of R¹⁸, the halogen atom includes a fluorine atom, a chlorine atom, a bromine atom and the like; the C1-C6 alkyl group includes a methyl group, an ethyl group, a propyl group, an isopropyl group, a butyl group, a isobutyl group, a sec-butyl group, a tert-butyl group, a pentyl group, an isopentyl group, a hexyl group and the like; and
the C1-C6 haloalkyl group includes a trifluoromethyl group, a chlorofluoromethyl group, a bromodifluoromethyl group, a 2-fluoroethyl group, a 2,2-difluoroethyl group, a 2,2,2-trifluoroethyl group, a 6,6,6-trifluorohexyl group and the like.

In the representation of R¹⁹ and R²⁰, the C1-C6 alkyl group includes a methyl group, an ethyl group, a propyl group, an isopropyl group, a butyl group, an isobutyl group, a sec-butyl group, a tert-butyl group, a pentyl group, an isopentyl group, a hexyl group and the like;
the C1-C6 haloalkyl group includes a 2-fluoroethyl group, a 2,2-difluoroethyl group, a 2,2,2-trifluoroethyl group, a 6,6,6-trifluorohexyl group and the like;
the C3-C6 alkenyl group includes a 2-propenyl group, a 1-methyl-2-propenyl group, a 2-methyl-2-propenyl group, a 2-butenyl group, a 3-butenyl group, a 2-hexenyl group, a 5-hexenyl group and the like;
the C3-C6 haloalkenyl group includes a 3,3-dichloro-2-propynyl group, a 3-chloro-2-propynyl group, a 3-bromo-2-propynyl group, a 3-iodo-2-propynyl group, a 6-chloro-5-hexenyl group and the like; the C3-C6 alkynyl group includes a 2-propynyl group, a 1-methyl-2-propynyl group, a 2-butynyl group, a 3-butynyl group, a 2-hexynyl group, a 5-hexynyl group and the like; and
the C3-C6 cycloalkyl group includes a cyclopropyl group, a cyclopentyl group, a cyclohexyl group and the like.

The embodiment of the compound of the present invention includes, for example, the following compounds:
a phenylpyridine compound represented by the formula (1-1); 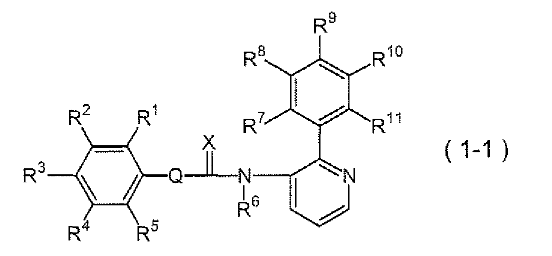
a phenylpyridine compound represented by the formula (1-2); 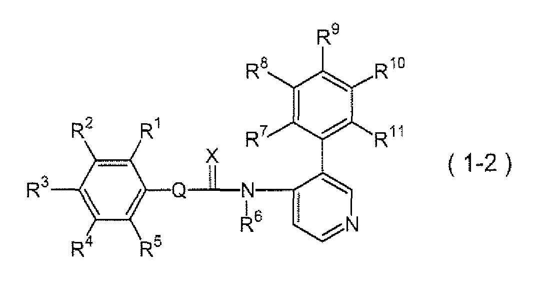
a phenylpyridine compound represented by the formula (1-3); 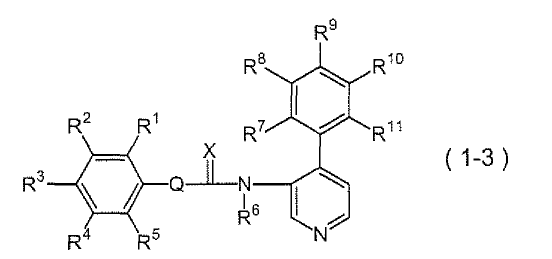
a phenylpyridine compound represented by the formula (1-4); 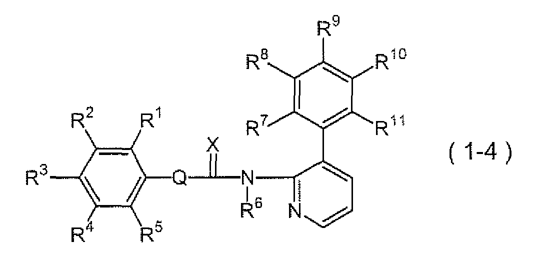
a phenylpyridine compound represented by any of the formula (1-1) to (1-4) wherein X is an oxygen atom;
a phenylpyridine compound represented by any of the formula (1-1) to (1-4) wherein R⁶ is a hydrogen atom;
a phenylpyridine compound represented by any of the formula (1-1) to (1-4) wherein R¹, R², R⁴ and R⁵ are a hydrogen atom;
a phenylpyridine compound represented by any of the formula (1-1) to (1-4) wherein R⁹ and R¹⁰ are a C1-C4 alkoxy group;
a phenylpyridine compound represented by any of the formula (1-1) to (1-4) wherein R⁹ and R¹⁰ are methoxy groups;
a phenylpyridine compound represented by any of the formula (1-1) to (1-4) wherein X is an oxygen atom and R⁶ is a hydrogen atom;
a phenylpyridine compound represented by any of the formula (1-1) to (1-4) wherein X is an oxygen atom, and R¹, R², R⁴ and R⁵ are hydrogen atoms;
a phenylpyridine compound represented by any of the formula (1-1) to (1-4) wherein X is an oxygen atom, and R⁹ and R¹⁰ are C1-C4 alkoxy groups;
a phenylpyridine compound represented by any of the formula (1-1) to (1-4) wherein X is an oxygen atom, and R⁹ and R¹⁰ are methoxy groups;
a phenylpyridine compound represented by any of the formula (1-1) to (1-4) wherein X is an oxygen atom, R⁶ is a hydrogen atom, and R¹, R², R⁴ and R⁵ are hydrogen atoms;
a phenylpyridine compound represented by any of the formula (1-1) to (1-4) wherein X is an oxygen atom, R⁶ is a hydrogen atom, and R⁹ and R¹⁰ are C1-C4 alkoxy groups;
a phenylpyridine compound represented by any of the formula (1-1) to (1-4) wherein X is an oxygen atom, R⁶ is a hydrogen atom, and R⁹ and R¹⁰ are methoxy groups;
a phenylpyridine compound represented by any of the formula (1-1) to (1-4) wherein R⁶ is a hydrogen atom, and R¹, R², R⁴ and R⁵ are hydrogen atoms;
a phenylpyridine compound represented by any of the formula (1-1) to (1-4) wherein R⁶ is a hydrogen atom, and R⁹ and R¹⁰ are C1-C4 alkoxy groups;
a phenylpyridine compound represented by any of the formula (1-1) to (1-4) wherein R⁶ is a hydrogen atom, and R⁹ and R¹⁰ are methoxy groups;
a phenylpyridine compound represented by any of the formula (1-1) to (1-4) wherein R¹, R², R⁴ and R⁵ are hydrogen atoms, and R⁹ and R¹⁰ are C1-C4 alkoxy groups;
a phenylpyridine compound represented by any of the formula (1-1) to (1-4) wherein R¹, R², R⁹ and R⁵ are hydrogen atoms, and R⁹ and R¹⁰ are methoxy groups;
a phenylpyridine compound represented by any of the formula (1-1) to (1-4) wherein R¹, R⁴ and R⁵ are hydrogen atoms, R⁶ is a hydrogen atom, R⁷, R⁸ and R¹¹ are hydrogen atoms, and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group;
a phenylpyridine compound represented by any of the formula (1-1) to (1-4) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms, and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group;
a phenylpyridine compound represented by any of the formula (1-1) to (1-4) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms, and R⁹ and R¹⁰ are C1-C6 alkoxy groups;
a phenylpyridine compound represented by the formula (1-5); 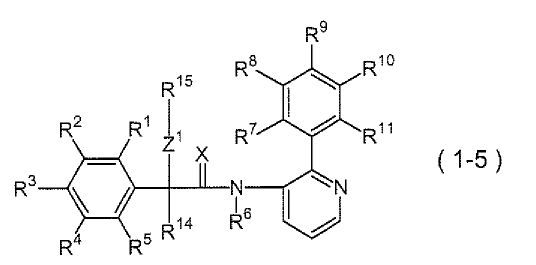
a phenylpyridine compound represented by the formula (1-6); 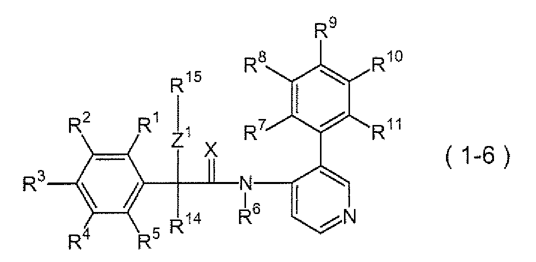
a phenylpyridine compound represented by the formula (1-7); 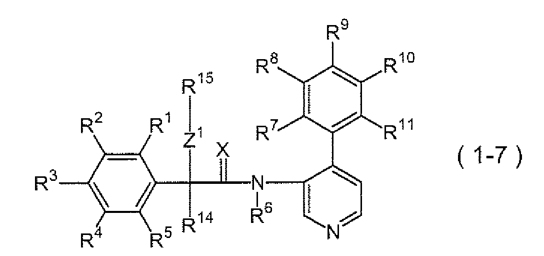
a phenylpyridine compound represented by the formula (1-8); 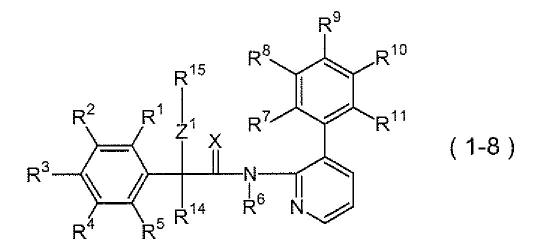
a phenylpyridine compound represented by any of the formula (1-5) to (1-8) wherein X is an oxygen atom;
a phenylpyridine compound represented by any of the formula (1-5) to (1-8) wherein R⁶ is a hydrogen atom;
a phenylpyridine compound represented by any of the formula (1-5) to (1-8) wherein R¹⁴ is a hydrogen atom and Z¹ is an oxygen atom; a phenylpyridine compound represented by any of the formula (1-5) to (1-8) wherein R¹⁴ is a hydrogen atom, Z¹ is an oxygen atom and R¹⁵ is a 2-propynyl group;
a phenylpyridine compound represented by any of the formula (1-5) to (1-8) wherein R¹, R², R⁴ and R⁵ are hydrogen atoms;
a phenylpyridine compound represented by any of the formula (1-5) to (1-8) wherein R⁹ and R¹⁰ are C1-C4 alkoxy groups;
a phenylpyridine compound represented by any of the formula (1-5) to (1-8) wherein R⁹ and R¹⁰ are methoxy groups;
a phenylpyridine compound represented by any of the formula (1-5) to (1-8) wherein X is an oxygen atom and R⁶ is a hydrogen atom; a phenylpyridine compound represented by any of the formula (1-5) to (1-8) wherein X is an oxygen atom, R¹⁴ is a hydrogen atom and Z¹ is an oxygen atom;
a phenylpyridine compound represented by any of the formula (1-5) to (1-8) wherein X is an oxygen atom, R¹⁴ is a hydrogen atom, Z¹ is an oxygen atom and R¹⁵ is a 2-propynyl group;
a phenylpyridine compound represented by any of the formula (1-5) to (1-8) wherein X is an oxygen atom and R¹, R², R⁴ and R⁵ are hydrogen atoms;
a phenylpyridine compound represented by any of the formula (1-5) to (1-8) wherein X is an oxygen atom and R⁹ and R¹⁰ are C1-C4 alkoxy groups;
a phenylpyridine compound represented by any of the formula (1-5) to (1-8) wherein X is an oxygen atom, and R⁹ and R¹⁰ are methoxy groups;
a phenylpyridine compound represented by any of the formula (1-5) to (1-8) wherein X is an oxygen atom, R⁶ is a hydrogen atom, R¹⁴ is a hydrogen atom, and Z¹ is an oxygen atom;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, and R¹⁵ is a 2-propynyl group;
a phenylpyridine compound represented by any of the formula (1-5) to (1-8) wherein X is an oxygen atom, R⁶ is a hydrogen atom, and R¹, R², R⁴ and R⁵ are hydrogen atoms;
a phenylpyridine compound represented by any of the formula (1-5) to (1-8) wherein X is an oxygen atom, R⁶ is a hydrogen atom, and R⁹ and R¹⁰ are C1-C4 alkoxy groups;
a phenylpyridine compound represented by any of the formula (1-5) to (1-8) wherein X is an oxygen atom, R⁶ is a hydrogen atom, and R⁹ and R¹⁰ are methoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, and R¹, R², R⁴ and R⁵ are hydrogen atoms;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹⁵ is a 2-propynyl group, and R¹, R², R⁴ and R⁵ are hydrogen atoms;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, and R⁹ and R¹⁰ are C1-C4 alkoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹⁵ is a 2-propynyl group, and R⁹ and R¹⁰ are C1-C4 alkoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, and R⁹ and R¹⁰ are methoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹⁵ is a 2-propynyl group, and R⁹ and R¹⁰ are methoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹⁵ is a 2-propynyl group, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹, R², R⁴ and R⁵ are hydrogen atoms, and R⁹ and R¹⁰ are methoxy groups;
a phenylpyridine compound represented by any of the formula (1-5) to (1-8) wherein X is an oxygen atom, R⁶ is a hydrogen atom, R¹⁴ is a hydrogen atom, Z¹ is an oxygen atom, R¹⁵ is a 2-propynyl group, R¹, R², R⁴ and R⁵ are hydrogen atoms, and R⁹ and R¹⁰ are methoxy groups;
a phenylpyridine compound represented by any of the formula (1-5) to (1-8) wherein R⁶ is a hydrogen atom, R¹⁴ is a hydrogen atom, and Z¹ is an oxygen atom;
a phenylpyridine compound represented by any of the formula (1-5) to (1-8) wherein R⁶ is a hydrogen atom, R¹⁴ is a hydrogen atom, Z¹ is an oxygen atom, and R¹⁵ is a 2-propynyl group;
a phenylpyridine compound represented by any of the formula (1-5) to (1-8) wherein R⁶ is a hydrogen atom, and R¹, R², R⁴ and R⁵ are hydrogen atoms;
a phenylpyridine compound represented by any of the formula (1-5) to (1-8) wherein R⁶ is a hydrogen atom, and R⁹ and R¹⁰ are C1-C4 alkoxy groups;
a phenylpyridine compound represented by any of the formula (1-5) to (1-8) wherein R⁶ is a hydrogen atom, and R⁹ and R¹⁰ are methoxy groups;
a phenylpyridine compound represented by any of the formula (1-5) to (1-8) wherein R⁶ is a hydrogen atom, R¹⁴ is a hydrogen atom, Z¹ is an oxygen atom, and R¹, R², R⁴ and R⁵ are hydrogen atoms;
a phenylpyridine compound represented by any of the formula (1-5) to (1-8) wherein R⁶ is a hydrogen atom, R¹⁴ is a hydrogen atom, Z¹ is an oxygen atom, R¹⁵ is a 2-propynyl group, and R¹, R², R⁴ and R⁵ are hydrogen atoms;
a phenylpyridine compound represented by any of the formula (1-5) to (1-8) wherein R⁶ is a hydrogen atom, R¹⁴ is a hydrogen atom, Z¹ is an oxygen atom, and R⁹ and R¹⁰ are C1-C4 alkoxy groups;
a phenylpyridine compound represented by any of the formula (1-5) to (1-8) wherein R⁶ is a hydrogen atom, R¹⁴ is a hydrogen atom, Z¹ is an oxygen atom, R¹⁵ is a 2-propynyl group, and R⁹ and R¹⁰ are C1-C4 alkoxy groups;
a phenylpyridine compound represented by any of the formula (1-5) to (1-8) wherein R⁶ is a hydrogen atom, R¹⁴ is a hydrogen atom, Z¹ is an oxygen atom, and R⁹ and R¹⁰ are methoxy groups;
a phenylpyridine compound represented by any of the formula (1-5) to (1-8) wherein R⁶ is a hydrogen atom, R¹⁴ is a hydrogen atom, Z¹ is an oxygen atom, R¹⁵ is a 2-propynyl group, and R⁹ and R¹⁰ are methoxy groups;
a phenylpyridine compound represented by any of the formula (1-5) to (1-8) wherein R⁶ is a hydrogen atom, R¹⁴ is a hydrogen atom, Z¹ is an oxygen atom, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein R⁶ is a hydrogen atom, R¹⁴ is a hydrogen atom, Z¹ is an oxygen atom, R²⁵ is a 2-propynyl group, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein R⁶ is a hydrogen atom, R¹⁴ is a hydrogen atom, Z¹ is an oxygen atom, R¹, R², R⁴ and R⁵ are hydrogen atoms, and R⁹ and R¹⁰ are methoxy groups;
a phenylpyridine compound represented by any of the formula (1-5) to (1-8) wherein R⁶ is a hydrogen atom, R¹⁴ is a hydrogen atom, Z¹ is an oxygen atom, R¹⁵ is a 2-propynyl group, R¹, R², R⁴ and R⁵ are hydrogen atoms, and R⁹ and R¹⁰ are methoxy groups;
a phenylpyridine compound represented by any of the formula (1-5) to (1-8) wherein R¹⁴ is a hydrogen atom, Z¹ is an oxygen atom, and R¹, R², R⁴ and R⁵ are hydrogen atoms;
a phenylpyridine compound represented by any of the formula (1-5) to (1-8) wherein R¹⁴ is a hydrogen atom, Z¹ is an oxygen atom, R¹⁵ is a 2-propynyl group, and R¹, R², R⁴ and R⁵ are hydrogen atoms;
a phenylpyridine compound represented by any of the formula (1-5) to (1-8) wherein R¹⁴ is a hydrogen atom, Z¹ is an oxygen atom, and R⁹ and R¹⁰ are C1-C4 alkoxy groups;
a phenylpyridine compound represented by any of the formula (1-5) to (1-8) wherein R¹⁴ is a hydrogen atom, Z¹ is an oxygen atom, R¹⁵ is a 2-propynyl group, and R⁹ and R¹⁰ are C1-C4 alkoxy groups;
a phenylpyridine compound represented by any of the formula (1-5) to (1-8) wherein R¹⁴ is a hydrogen atom, Z¹ is an oxygen atom, and R⁹ and R¹⁰ are methoxy groups;
a phenylpyridine compound represented by any of the formula (1-5) to (1-8) wherein R¹⁴ is a hydrogen atom, Z¹ is an oxygen atom, R¹⁵ is a 2-propynyl group, and R⁹ and R¹⁰ are methoxy groups;
a phenylpyridine compound represented by any of the formula (1-5) to (1-8) wherein R¹⁴ is a hydrogen atom, Z¹ is an oxygen atom, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein R¹⁴ is a hydrogen atom, Z¹ is an oxygen atom, R¹⁵ is a 2-propynyl group, R¹, R², R⁴ and R⁵ are hydrogen atoms, and R⁹ and R¹⁰ are C1-C4 alkoxy groups;
a phenylpyridine compound represented by any of the formula (1-5) to (1-8) wherein R¹⁴ is a hydrogen atom, Z¹ is an oxygen atom, R¹, R², R⁴ and R⁵ are hydrogen atoms, and R⁹ and R¹⁰ are methoxy groups;
a phenylpyridine compound represented by any of the formula (1-5) to (1-8) wherein R¹⁴ is a hydrogen atom, Z¹ is an oxygen atom, R¹⁵ is a 2-propynyl group, R¹, R², R⁴ and R⁵ are hydrogen atoms, and R⁹ and R¹⁰ are methoxy groups;
a phenylpyridine compound represented by any of the formula (1-5) to (1-8) wherein R¹, R², R⁴ and R⁵ are hydrogen atoms, and R⁹ and R¹⁰ are C1-C4 alkoxy groups;
a phenylpyridine compound represented by any of the formula (1-5) to (1-8) wherein R¹, R², R⁴ and R⁵ are hydrogen atoms, and R⁹ and R¹⁰ are methoxy groups;
a phenylpyridine compound represented by any of the formula (1-5) to (1-8) wherein R¹, R⁴ and R⁵ are a hydrogen atom, R⁶ is a hydrogen atom, R⁷, R⁸ and R¹¹ are hydrogen atoms, and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group, R¹⁴ is a hydrogen atom, Z¹ is an oxygen atom, R¹⁵ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group;
a phenylpyridine compound represented by any of the formula (1-5) to (1-8) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms, and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6
cyanoalkyloxy group or a C3-C6 cycloalkoxy group, R¹⁴ is a hydrogen atom, Z¹ is an oxygen atom, R¹⁵ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group;
a phenylpyridine compound represented by any of the formula (1-5) to (1-8) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms, and R⁹ and R¹⁰ are C1-C6 alkoxy groups, R¹⁴ is a hydrogen atom, Z¹ is an oxygen atom, R¹⁵ is a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group:
   a phenylpyridine compound represented by the formula (1-9); 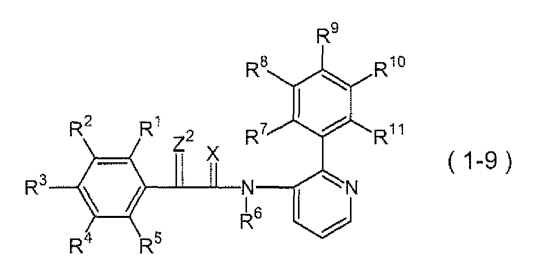
   a phenylpyridine compound represented by the formula (1-10); 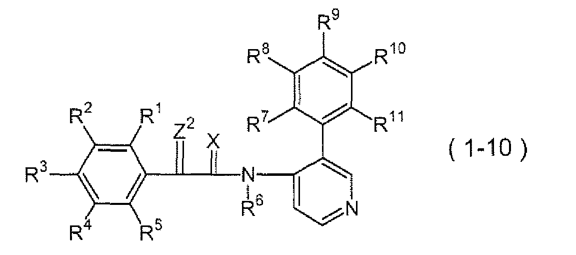
   a phenylpyridine compound represented by the formula (1-11); 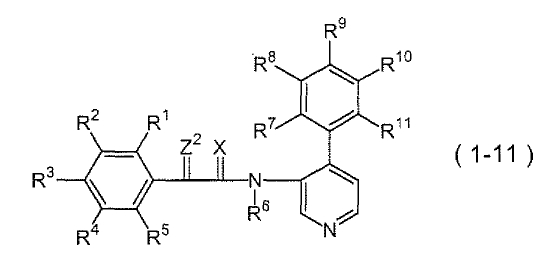
   a phenylpyridine compound represented by the formula (1-12); 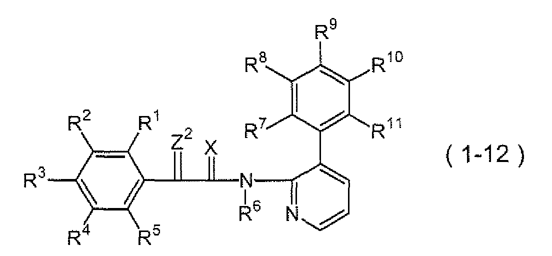
   a phenylpyridine compound represented by any of the formula (1-9) to (1-12) wherein X is an oxygen atom;
   a phenylpyridine compound represented by any of the formula (1-9) to (1-12) wherein R⁶ is a hydrogen atom;
   a phenylpyridine compound represented by any of the formula (1-9) to (1-12) wherein Z² is NOR¹⁶;
   a phenylpyridine compound represented by any of the formula (1-9) to (1-12) wherein R¹, R², R⁴ and R⁵ are hydrogen atoms;
   a phenylpyridine compound represented by any of the formula (1-9) to (1-12) wherein R⁹ and R¹⁰ are C1-C4 alkoxy groups;
   a phenylpyridine compound represented by any of the formula (1-9) to (1-12) wherein R⁹ and R¹⁰ are methoxy groups;
   a phenylpyridine compound represented by any of the formula (1-9) to (1-12) wherein X is an oxygen atom and R⁶ is a hydrogen atom;
   a phenylpyridine compound represented by any of the formula (1-9) to (1-12) wherein X is an oxygen atom and Z² is NOR¹⁶;
   a phenylpyridine compound represented by any of the formula (1-9) to (1-12) wherein X is an oxygen atom, and R¹, R², R⁴ and R⁵ are hydrogen atoms;
   a phenylpyridine compound represented by any of the formula (1-9) to (1-12) wherein X is an oxygen atom, and R⁹ and R¹⁰ are C1-C4 alkoxy groups;
   a phenylpyridine compound represented by any of the formula (1-9) to (1-12) wherein X is an oxygen atom, and R⁹ and R¹⁰ are methoxy groups;
   a phenylpyridine compound represented by any of the formula (1-9) to (1-12) wherein X is an oxygen atom, R⁶ is a hydrogen atom, and Z² is NOR¹⁶;
   a phenylpyridine compound represented by any of the formula (1-9) to (1-12) wherein X is an oxygen atom, R⁶ is a hydrogen atom, and R¹, R², R⁴ and R⁵ are hydrogen atoms;
   a phenylpyridine compound represented by any of the formula (1-9) to (1-12) wherein X is an oxygen atom, R⁶ is a hydrogen atom, and R⁹ and R¹⁰ are C1-C4 alkoxy groups;
   a phenylpyridine compound represented by any of the formula (1-9) to (1-12) wherein X is an oxygen atom, R⁶ is a hydrogen atom, and R⁹ and R¹⁰ are methoxy groups;
   a phenylpyridine compound represented by any of the formula (1-9) to (1-12) wherein X is an oxygen atom, R⁶ is a hydrogen atom, Z² is NOR¹⁶, and R¹, R², R⁴ and R⁵ are hydrogen atoms;
   a phenylpyridine compound represented by any of the formula (1-9) to (1-12) wherein X is an oxygen atom, R⁶ is a hydrogen atom, Z² is NOR¹⁶, and R⁹ and R¹⁰ are C1-C4 alkoxy groups;
   a phenylpyridine compound represented by any of the formula (1-9) to (1-12) wherein X is an oxygen atom, R⁶ is a hydrogen atom, Z² is NOR¹⁶, and R⁹ and R¹⁰ are methoxy groups;
   a phenylpyridine compound represented by any of the formula (1-9) to (1-12) wherein R⁶ is a hydrogen atom, and Z² is NOR¹⁶;
   a phenylpyridine compound represented by any of the formula (1-9) to (1-12) wherein R⁶ is a hydrogen atom, and R¹, R², R⁴ and R⁵ are hydrogen atoms;
   a phenylpyridine compound represented by any of the formula (1-9) to (1-12) wherein R⁶ is a hydrogen atom, and R⁹ and R¹⁰ are C1-C4 alkoxy groups;
   a phenylpyridine compound represented by any of the formula (1-9) to (1-12) wherein R⁶ is a hydrogen atom, and R⁹ and R¹⁰ are methoxy groups;
   a phenylpyridine compound represented by any of the formula (1-9) to (1-12) wherein R⁶ is a hydrogen atom, Z² is NOR¹⁶, and R¹, R², R⁴ and R⁵ are hydrogen atoms;
   a phenylpyridine compound represented by any of the formula (1-9) to (1-12) wherein R⁶ is a hydrogen atom, Z² is NOR¹⁶, and R⁹ and R¹⁰ are C1-C4 alkoxy groups;
   a phenylpyridine compound represented by any of the formula (1-9) to (1-12) wherein R⁶ is a hydrogen atom, Z² is NOR¹⁶, and R⁹ and R¹⁰ are methoxy groups;
   a phenylpyridine compound represented by any of the formula (1-9) to (1-12) wherein R⁶ is a hydrogen atom, Z² is NOR¹⁶, R¹, R², R⁴ and R⁵ are hydrogen atoms, and R⁹ and R¹⁰ are C1-C4 alkoxy groups;
   a phenylpyridine compound represented by any of the formula (1-9) to (1-12) wherein R⁶ is a hydrogen atom, Z² is NOR¹⁶, R¹, R², R⁴ and R⁵ are hydrogen atoms, and R⁹ and R¹⁰ are methoxy groups;
   a phenylpyridine compound represented by any of the formula (1-9) to (1-12) wherein Z² is NOR¹⁶, and R¹, R², R⁴ and R⁵ are hydrogen atoms;
   a phenylpyridine compound represented by any of the formula (1-9) to (1-12) wherein Z² is NOR¹⁶, and R⁹ and R¹⁰ are C1-C4 alkoxy groups;
   a phenylpyridine compound represented by any of the formula (1-9) to (1-12) wherein Z² is NOR¹⁶, and R⁹ and R¹⁰ are methoxy groups;
   a phenylpyridine compound represented by any of the formula (1-9) to (1-12) wherein Z² is NOR¹⁶, R¹, R², R⁴ and R⁵ are hydrogen atoms, and R⁹ and R¹⁰ are C1-C4 alkoxy groups;
   a phenylpyridine compound represented by any of the formula (1-9) to (1-12) wherein Z² is NOR¹⁶, R¹, R², R⁴ and R⁵ are hydrogen atoms, and R⁹ and R¹⁰ are methoxy groups;
   a phenylpyridine compound represented by any of the formula (1-9) to (1-12) wherein R¹, R², R⁴ and R⁵ are hydrogen atoms, and R⁹ and R¹⁰ are C1-C4 alkoxy groups;
   a phenylpyridine compound represented by any of the formula (1-9) to (1-12) wherein R¹, R², R⁴ and R⁵ are hydrogen atoms, and R⁹ and R¹⁰ are methoxy groups;
   a phenylpyridine compound represented by any of the formula (1-9) to (1-12) wherein R¹, R⁴ and R⁵ are hydrogen atoms, R⁶ is a hydrogen atom, R⁷, R⁸ and R¹¹ are hydrogen atoms and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group, and Z² is NOR¹⁶;
   a phenylpyridine compound represented by any of the formula (1-9) to (1-12) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms and each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group, and Z² is NOR¹⁶; and
   a phenylpyridine compound represented by any of the formula (1-9) to (1-12) wherein R¹, R⁴ and R⁵ are hydrogen atoms, R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group, R⁶ is a hydrogen atom, R⁷, R⁸ and R¹¹ are hydrogen atoms and R⁹ and R¹⁰ are C1-C6 alkoxy groups, and Z² is NOR¹⁶.

Methods for producing the compounds of the present invention are described hereinafter.

The compound of the present invention can be produced, for example, according to the following (Production Method 1) to (Production Method 12). In these production methods, protecting groups may be, if necessary, applied to protect functional groups from chemical reactions.

### (Production Method 1)

The compound (4) among the compounds of the present invention can be produced by subjecting the compound (2) to reaction with the compound (3); 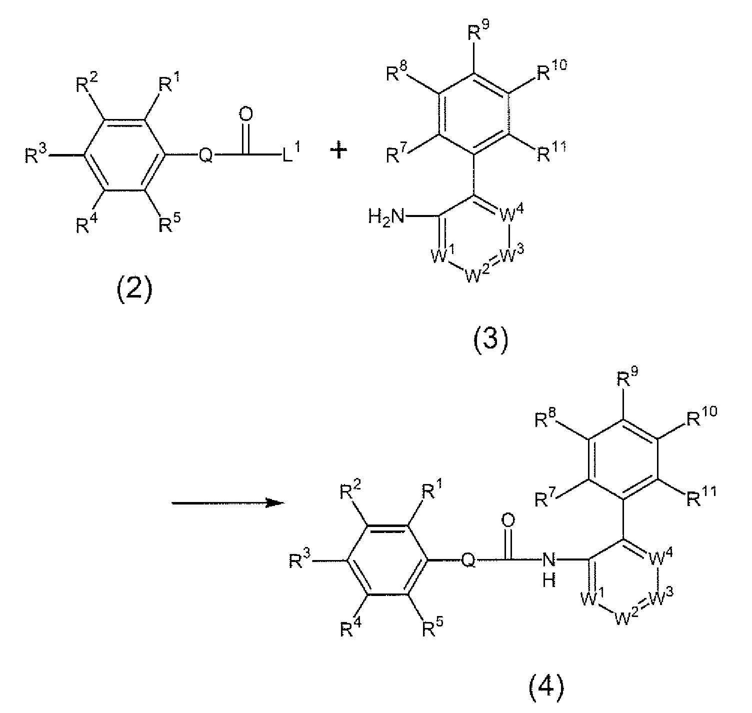 (, wherein, in the formula, L¹ represents a chlorine atom or a bromine atom, R¹, R², R³, R⁴, R⁵, R⁷, R⁸, R⁹, R¹⁰, R¹¹, W¹―W²=W³―W⁴ and Q represent same meaning defined in [Invention 1].)

Said reaction is carried out in the presence of a base and usually in a solvent.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the compound (3) is usually from 0.5 to 2 moles per 1 mole of the compound (2) and that to the base is 10 moles or less.

The reaction time is usually in the range of from 0.1 to 24 hours, and the reaction temperature is in the range of from 0 to 150 °C.

After completion of the reaction, the compound (4) can be isolated by being subjectedtopost treatment procedure exemplified below.
(1) A method to extract reaction mixture with an organic solvent, followed by the organic layer being dried and concentrated
(2) A method to filtrate reaction mixture, after addition with an organic solvent, if necessary, followed by the filtrate being concentrated
The isolated compound of the present invention represented by the formula (4) can be purified by a technique such as chromatography, recrystallization and the like.

### (Production Method 2)

The compound (6) among the compounds of the present invention can be produced by subjecting the compound (5) to reaction with 2,4-bis(4-methoxyphenyl)-1,3-dithia-2,4-diphosphetane-2,4-dis ulfide (,which is referred to as "Lawesson's reagent" hereinafter); 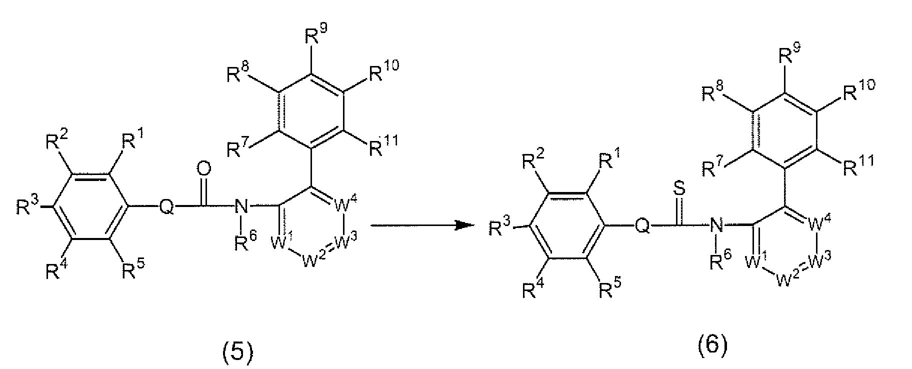 (, wherein, in the formula, R¹, R², R³, R⁴, R⁵, R⁶, R⁷, R⁸, R⁹, R¹⁰, R¹¹, W¹―W²=W³―W⁴ and Q represent same meaning defined in [Invention 1].)

Said reaction is usually carried out in a solvent.

The solvent used for the reaction includes ethers such as 1,4-dioxane, tetrahydrofuran, ethyleneglycoldimethylether, t-butylmethylethers and the like; aliphatic hydrocarbons such as hexane, heptane and the like; aromatic hydrocarbons such as toluene, xylene and the like; halogenated hydrocarbons such as chlorobenzene and the like; organic nitriles such as acetonitrile, butyronitrile and the like; sulfoxides such as dimethylsulfoxide and the like; and the mixture thereof.

Lawesson's reagent is usually used in the molar ratio of from 1 to 10 moles per 1 mole of the compound (5).

The reaction time is usually in the range of from 0.5 to 24 hours, and the reaction temperature is in the range of from 30 to 150 °C.

After completion of the reaction, the reaction mixture is subjected to post treatment procedure such as extracting with organic solvent, subsequently drying and concentrating the organic layer, to isolate the compound (6). The isolated compound (6) can be purified by a technique such as chromatography, recrystallization and the like.

### (Production Method 3)

The compound (9) among the compounds of the present invention can be produced by subjecting the compound (7) to reaction with the compound (8); 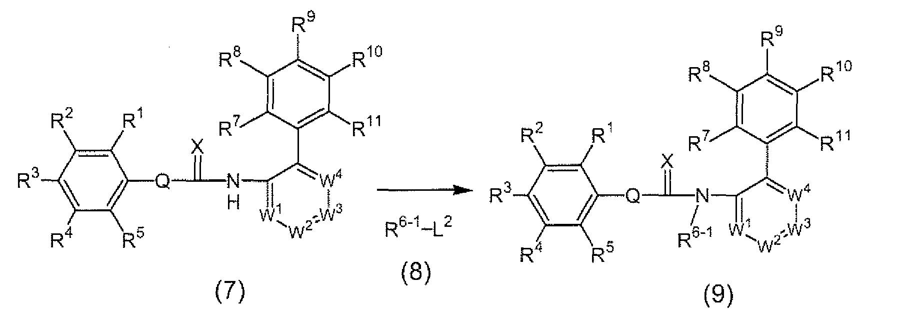 (, wherein, in the formula, L² represents a bromine atom, an iodine atom or a methanesulfonyloxy group, R⁶⁻¹ represents a C1-C3 alkyl group, R¹, R², R³, R⁴, R⁵, R⁷, R⁸, R⁹, R¹⁰, R¹¹, W¹―W²=W³―W⁴ and Q represent same meaning defined in [Invention 1], and X represents same meaning described above.)

Said reaction is carried out in the presence of a base and usually in a solvent.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; and tertiary amines such as triethylamine, diisopropylethylamine, 1,8-diazabicyclo[5.4.0]undec-7-ene, 1,5-diazabicyclo[4.3.0]non-5-ene and the like.

The molar ratio applied to the compound (8) is usually from 1 to 10 moles per 1 mole of the compound (7) and that to the base is usually from 1 to 10 moles.

The reaction time is usually in the range of from 0.5 to 24 hours, and the reaction temperature is in the range of from 0 to 120 °C.

After completion of the reaction, the reaction mixture is subjected to post treatment procedure such as extracting with organic solvent, subsequently drying and concentrating the organic layer, to isolate the compound (9). The isolated compound (9) can be purified by a technique such as chromatography, recrystallization and the like.

### (Production Method 4)

The compound (11) among the compounds of the present invention can be produced by subjecting the compound (10) to reaction with a reducing agent; 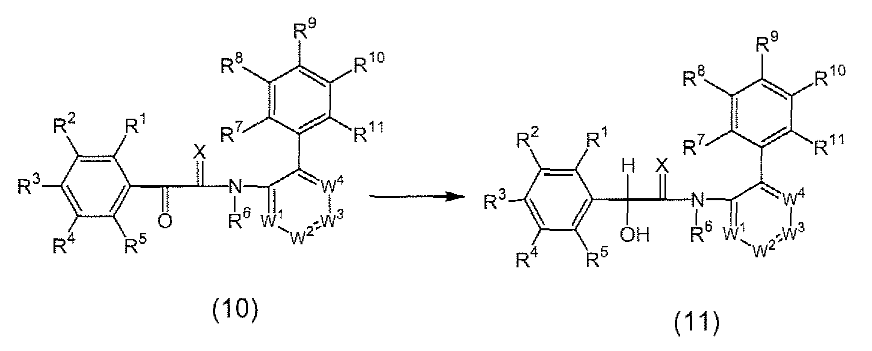 (, wherein, in the formula, R¹, R², R³, R⁴, R⁵, R⁶, R⁷, R⁸, R⁹, R¹⁰, R¹¹ and W¹―W²=W³―W⁴ represent same meaning defined in [Invention 1], and X represents same meaning described above.)

Said reaction is usually carried out in a solvent.

The reducing agent used for the reaction includes sodium borohydride or potassium borohydride.

The solvent used for the reaction includes alcohols such as methanol, ethanol, 2-propanol and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; and water and the mixture thereof.

The molar ratio applied to the reducing agent is usually from 0.25 to 3 moles per 1 mole of the compound (10).

The reaction time is usually in the range of from the instant to 24 hours, and the reaction temperature is in the range of from -20 to 100 °C.

After completion of the reaction, the reaction mixture is subjected to post treatment procedure such as, after being added with an acidic water such as aqueous saturated ammonium chloride, extracting with organic solvent, subsequently drying and concentrating the organic layer obtained, to isolate the compound (11). The isolated compound (11) can be purified by a technique such as chromatography, recrystallization and the like.

### (Production Method 5)

The compound (12) among the compounds of the present invention can be produced by subjecting the compound (10) to reaction with organic metal compounds such as alkylmagnesium halide (Grignard reagent), alkyllithium and the like which correspond to R¹⁴⁻¹; 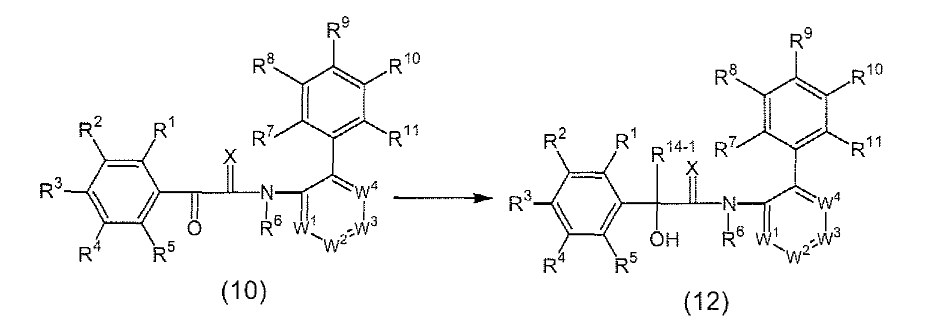 (, wherein, in the formula, R¹⁴⁻¹ represents a C1-C3 alkyl group, R¹, R², R³, R⁴, R⁵, R⁶, R⁷, R⁸, R⁹, R¹⁰, R¹¹ and W¹―W ²=W³―W⁴ represent same meaning defined in [Invention 1], and X represents same meaning described above.)

Said reaction is usually carried out in a solvent.

The organic metal compound corresponding to R¹⁴⁻¹ used in the reaction is defined by an organic metal compound in which R¹⁴⁻¹ is the organic group coupled with a metal element, and is illustrated by alkylmagnesium halide and alkyllithium. Specifically included are methylmagnesiumbromide, methyllithium, ethylmagnesiumchloride and ethyllithium.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; and aromatic hydrocarbons such as toluene, xylene and the like.

The molar range applied to the organic metal compound is usually from 1 to 3 moles per 1 mole of the compound (10).

The reaction time is usually in the range of from the instant to 24 hours, and the reaction temperature is in the range of from -80 to 50 °C.

After completion of the reaction, the reaction mixture is subjected to post treatment procedure such as extracting with organic solvent, subsequently drying and concentrating the organic layer, to isolate the compound (12). The isolated compound (12) can be purified by a technique such as chromatography, recrystallization and the like.

### (Production Method 6)

The compound (14) and the compound (16) among the compounds of the present invention can be produced by the following method. That is, the compound (14) can be produced by subj ecting the compound (13) to reaction with an alkylsulfonyl chloride compound (Step 6-1), and furthermore, the compound (16) can be produced by subjecting the compound (14) to reaction with the compound (15)

### (Step 6-2);

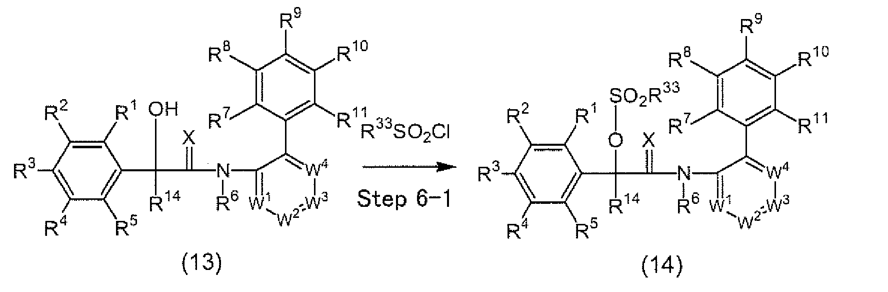

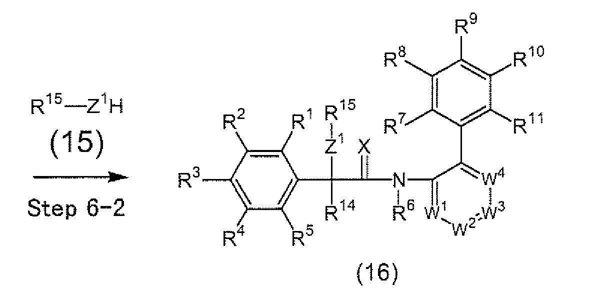

(, wherein, in the formula, R³³ represents a C1-C3 alkyl group, R¹, R², R³, R⁴, R⁵, R⁶, R⁷, R⁸, R⁹, R¹⁰, R¹¹, R¹⁴, R¹⁵ and W¹―W²=W³ -W⁴ represent same meaning defined in [Invention 1], and X and Z¹ represent same meaning described above.)

### (Step 6-1)

Said reaction is carried out in the presence of a base and usually in a solvent.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the alkylsulfonyl chloride compound is usually from 1 to 3 moles per 1 mole of the compound (13) and that to the base is usually from 1 to 10 moles.

The reaction time is usually in the range of from 0.1 to 24 hours, and the reaction temperature is in the range of from -20 to 100 °C.

After completion of the reaction, the reaction mixture is subjected to post treatment procedure such as extracting with organic solvent, if necessary, after the organic layer being washed by acidic water, basic water or the like, subsequently drying and concentrating the organic layer, to isolate the compound (14). The isolated compound (14) can be purified by a technique such as recrystallization and the like.

### (Step 6-2)

Said reaction is carried out with or without the presence of a solvent with or without the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the compound (15) is usually from 1 mole to excessive amount per 1 mole of the compound (14). The reaction time is usually in the range of from 0.5 to 24 hours, and the reaction temperature is in the range of from 0 to 150 °C.

After completion of the reaction, the reaction mixture is subjected to post treatment procedure such as extracting with organic solvent, if necessary, after the organic layer being washed by acidic water, basic water or the like, subsequently drying and concentrating the organic layer, to isolate the compound (16). The isolated compound (16) can be purified by a technique such as recrystallization and the like.

### (Production Method 7)

The compound (19) among the compounds of the present invention can be produced by subjecting the compound (17) to reaction with the compound (18) or the salt thereof; 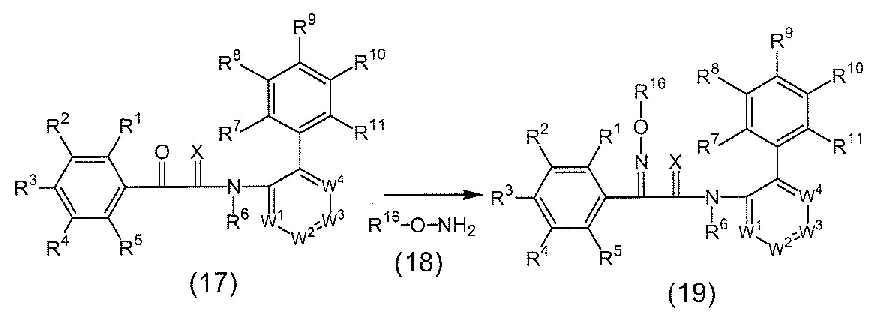 (, wherein, in the formula, R¹, R², R³, R⁴, R⁵, R⁶, R⁷, R⁸, R⁹, R¹⁰, R¹¹, R¹⁶ and W¹―W²=W³―W⁴ represent same meaning defined in [Invention 1], and X represents same meaning described above.)

Said reaction is usually carried out in a solvent, and if necessary, in the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; alcohols such as methanol, ethanol, propanol and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate, sodium bicarbonate and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar range applied to the base is usually from the catalytic amount to 10 moles per 1 mole of the compound (17) and that to the compound (18) or the salt thereof is usually from 1 to 10 moles.

The reaction time is usually in the range of from 1 to 24 hours, and the reaction temperature is in the range of from 0 to 150 °C.

After completion of the reaction, the reaction mixture is subjected to post treatment procedure such as extracting with organic solvent, and if necessary, after the organic layer being washed by acidic water, and subsequently drying and concentrating the organic layer, to isolate the compound (19). The isolated compound (19) can be purified by a technique such as chromatography, recrystallization and the like.

### (Production Method 8)

The compound (22) among the compounds of the present invention can be produced by subjecting the compound (20) to reaction with the compound (21): 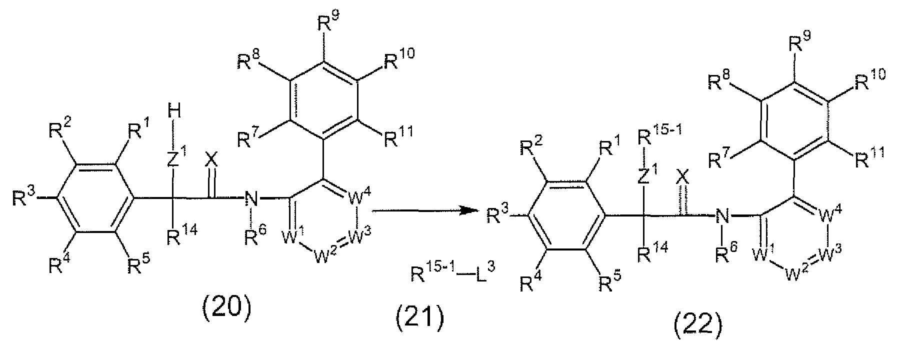 (, wherein, in the formula, L³ represents a chlorine atom, a bromine atom and a methanesulfonyloxy group; R¹⁵⁻¹ represents a C1-C6 alkyl group, a C1-C6 haloalkyl group, a C3-C6 alkenyl group, a C3-C6 haloalkenyl group, a C3-C6 alkynyl group, a C3-C6 haloalkynyl group, a C3-C6 cycloalkyl group, a (C1-C6 alkyl) carbonyl group, a (C1-C6 haloalkyl) carbonyl group, a (C1-C6 alkoxy) carbonyl group, a (C1-C6 haloalkoxy)carbonyl group, a (C3-C6 alkenyloxy)carbonyl group, a (C3-C6 haloalkenyloxy)carbonyl group, a (C3-C6 alkynyloxy) carbonyl group, a (C3-C6 haloalkynyloxy) carbonyl group or a C1-C3 alkylsulfonyl group; R¹, R², R³, R⁴, R⁵, R⁶, R⁷, R⁸, R⁹, R¹⁰, R¹¹, R¹⁴, W¹―W²=W³―W⁴ and X represent same meaning defined in [Invention 1]; and Z¹ represents same meaning described above.)

Said reaction is usually carried out in a solvent with the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and water and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar range applied to the compound (21) is usually from 1 to 3 moles per 1 mole of the compound (20) and that to the base is usually from 1 to 3 moles.

The reaction time is usually in the range of from 1 to 24 hours, and the reaction temperature is in the range of from 0 to 150 °C.

After completion of the reaction, the reaction mixture is subjected to post treatment procedure such as extracting with organic solvent, subsequently drying and concentrating the organic layer, to isolate the compound (22). The isolated compound (22) can be purified by a technique such as chromatography, recrystallization and the like.

### (Production Method 9)

The compound (28) among the compounds of the present invention can be produced by subjecting the compound (26) to reaction with the compound (27); 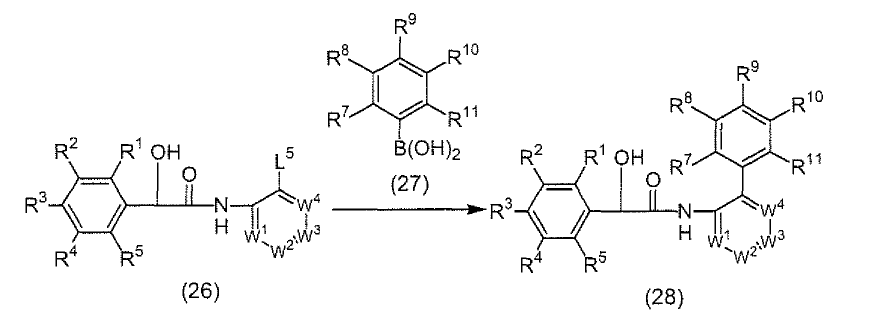 (, wherein, in the formula, L⁵ represents a chlorine atom, a bromine atom or an iodine atom, and R¹, R², R³, R⁴, R⁵, R⁷, R⁸, R⁹, R¹⁰, R¹¹ and W¹―W²=W³―W⁴ represent same meaning defined in [Invention 1].)

Said reaction is carried out in a solvent with the presence of a catalyst.

The solvent used for the reaction includes alcohols such as methanol, ethanol, propanol, butanol, 2-propanoland and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; water and the mixture thereof.

The catalyst used for the reaction includes palladium acetate, tetrakistriphenylphosphine palladium, {1,1'-bis(diphenylphosphino)ferrocene}dichloropalladium methylene chloride complex and bis (triphenylphosphine) palladium dichloride.

The molar range applied to the compound (27) is usually from 1 to 5 moles per 1 mole of the compound (26) and that to the catalyst is usually from 0.001 to 0.1 moles.

Said reaction maybe, if necessary, carried out in the presence of a base (, which includes inorganic base such as sodium acetate, potassium acetate, potassium carbonate, tripotassium phosphate, sodium bicarbonate and the like) and/or a phase-transfer catalyst (, which includes quaternary ammonium salts such as tetrabutylammonium bromide, benzyltriethylammonium bromide and the like).

The reaction time is usually in the range of from 0.5 to 24 hours, and the reaction temperature is in the range of from 50 to 120 °C.

After completion of the reaction, the reaction mixture is subjected to post treatment procedure such as filtrating as itself, subsequently concentrating the filtrate, to isolate the compound (28). The isolated compound (28) can be purified by a technique such as chromatography, recrystallization and the like.

### (Production Method 10)

The compound (42) among the compounds of the present invention can be produced by subjecting the compound (40) to reaction with the compound (41): 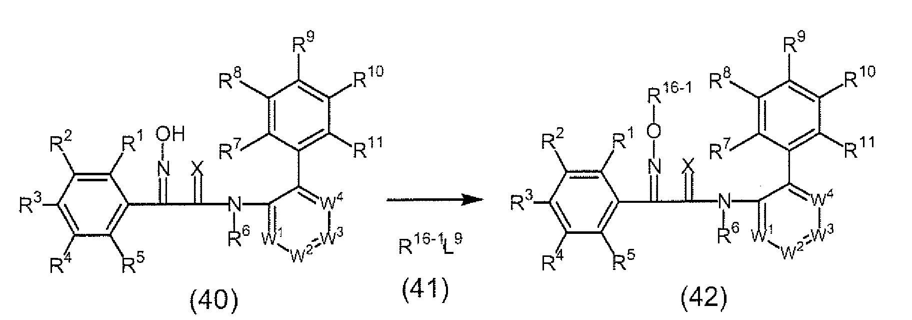 (, wherein, in the formula, L⁹ represents a chlorine atom, a bromine atom and a methanesulfonyloxy group; R¹⁶⁻¹ represents a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group; R¹, R², R³, R⁴, R⁵, R⁶, R⁷, R⁸, R⁹, R¹⁰, R¹¹ and W¹―W²=W³―W⁴ represent same meaning defined in [Invention 1]; and X represents same meaning described above.)

Said reaction is usually carried out in a solvent with the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; and alkali metal hydrides such as sodium hydride and the like.

The molar range applied to the compound (41) is usually from 1 to 3 moles per 1 mole of the compound (40) and that to the base is usually from 1 to 10 moles.

The reaction time is usually in the range of from 1 to 24 hours, and the reaction temperature is in the range of from 0 to 150 °C.

After completion of the reaction, the reaction mixture is subjected to post treatment procedure such as extracting with organic solvent, subsequently drying and concentrating the organic layer, to isolate the compound (42). The isolated compound (42) can be purified by a technique such as chromatography, recrystallization and the like.

### (Production Method 11)

The compound (45) among the compounds of the present invention can be produced by subjecting the compound (43) to reaction with the compound (44) : 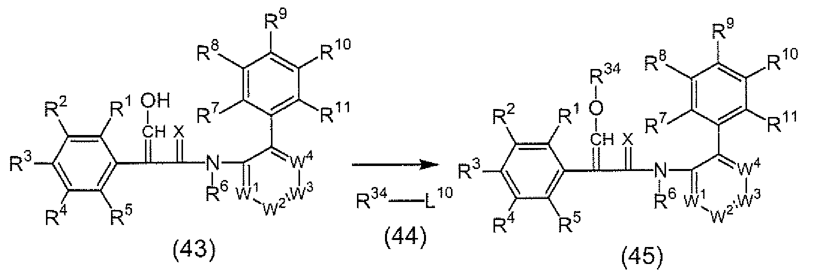 [, wherein, in the formula, L¹⁰ represents leaving groups such as a chlorine atom, a bromine atom, an iodine atom, p-toluenesulfonyloxy group, a methanesulfonyloxy group and the like; R³⁴ represents a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group; R¹, R², R³, R⁴, R⁵, R⁶, R⁷, R⁸, R⁹, R¹⁰, R¹¹ and W¹―W²=W³―W⁴ represent same meaning defined in [Invention 1]; and X represents same meaning described above.)

Said reaction is usually carried out in a solvent with the presence of a base.

The solvent used for the reaction includes ethers such as 1,4-dioxane, tetrahydrofuran, ethyleneglycoldimethylether, tert-butylmethylether and the like;
aliphatic hydrocarbons such as hexane, heptane, octane and the like; aromatic hydrocarbons such as toluene, xylene and the like; halogenated hydrocarbons such as chlorobenzene and the like; esters such as butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar range applied to the compound (44) is usually from 1 to 3 moles per 1 mole of the compound (43) and that to the base is usually from 1 to 5 moles.

The reaction time is usually in the range of from 1 to 24 hours, and the reaction temperature is in the range of from 0 to 150 °C.

After completion of the reaction, the reaction mixture is subjected to post treatment procedure such as extracting with organic solvent, subsequently drying and concentrating the organic layer, to isolate the compound (45). The isolated compound (45) can be purified by a technique such as chromatography, recrystallization and the like.

### (Production Method 12)

The compound (52) among the compounds of the present invention can be produced by subjecting the compound (25) to reaction with the compound (18) or the salt thereof to obtain the compound (51) (Step 12-1), and then subjecting the compound (51) to reaction with the compound (27) (Step 12-2); 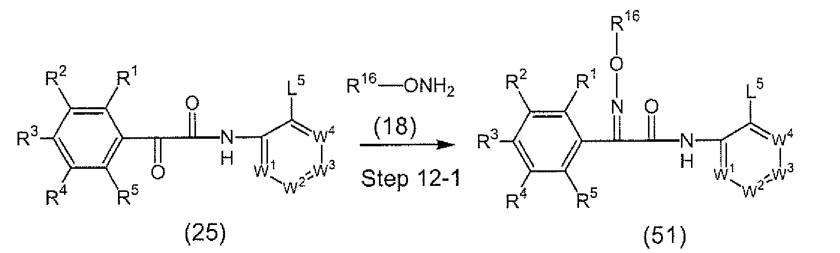 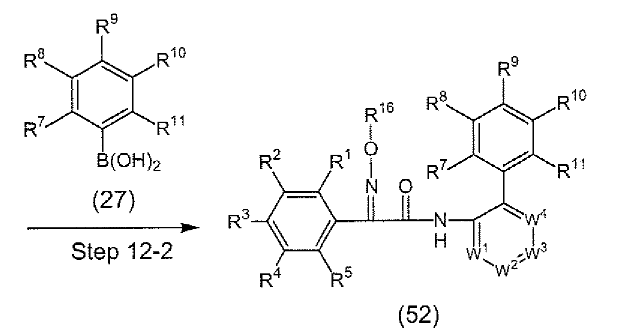 (, wherein, in the formula, R¹, R², R³, R⁴, R⁵, R⁷, R⁸, R⁹, R¹⁰, R¹¹ and W¹―W²=W³―W⁴ represent same meaning defined in [Invention 1], and R¹⁶ and L⁵ represent same meaning described above.)

### (Step 12-1)

Said reaction is usually carried out in a solvent, and if necessary, in the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters butylacetate, ethylacetate and the like; alcohols such as methanol, ethanol, propanol and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate, sodium bicarbonate and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar range applied to the base is usually from the catalytic amount to 10 moles per 1 mole of the compound (25) and that to the compound (18) or the salt thereof is usually from 1 to 10 moles.

The reaction time is usually in the range of from 1 to 24 hours, and the reaction temperature is in the range of from 0 to 150 °C.

After completion of the reaction, the reaction mixture is subjected to post treatment procedure such as extracting with organic solvent, and if necessary, the organic layer being washed by acidic water and the like, subsequently drying and concentrating the organic layer, to isolate the compound (51). The isolated compound (51) can be purified by a technique such as chromatography, recrystallization and the like.

### (Step 12-2)

Said reaction is carried out in a solvent with the presence of a catalyst.

The solvent used for the reaction includes alcohols such as methanol, ethanol, propanol, butanol, 2-propanol and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; water and the mixture thereof.

The catalyst used for the reaction includes palladium acetate, tetrakistriphenylphosphine palladium, {1,1'-bis(diphenylphosphino)ferrocene}dichloropalladium methylene chloride complex and bis (triphenylphosphine) palladium dichloride.

The molar range applied to the compound (27) is usually from 1 to 5 moles per 1 mole of the compound (51) and that to the catalyst is usually from 0.001 to 0.1 moles.

Said reaction may be, if necessary, carried out in the presence of a base (, which includes inorganic base such as sodium acetate, potassium acetate, potassium carbonate, tripotassium phosphate, sodium bicarbonate and the like) and/or a phase-transfer catalyst (, which includes quaternary ammonium salts such as tetrabutylammonium bromide, benzyltriethylammonium bromide and the like).

The reaction time is usually in the range of from 0.5 to 24 hours, and the reaction temperature is in the range of from 50 to 120 °C.

After completion of the reaction, the compound (52) can be isolated by being subjected to post treatment procedure exemplified below.
(1) A method to extract reaction mixture with an organic solvent, followed by the organic layer obtained being dried and concentrated
(2) A method to filtrate reaction mixture, after addition with an organic solvent, if necessary, followed by the filtrate being concentrated

The isolated compound (52) can be purified by technique such as chromatography, recrystallization and the like.

Next, methods for producing intermediate compounds of the compound of the present invention are described as follows.

The compound (3) can be produced, for example, according to the following (Intermediate Compound Production Method 1) or (Intermediate Compound Production Method 2).

### (Intermediate Compound Production Method 1)

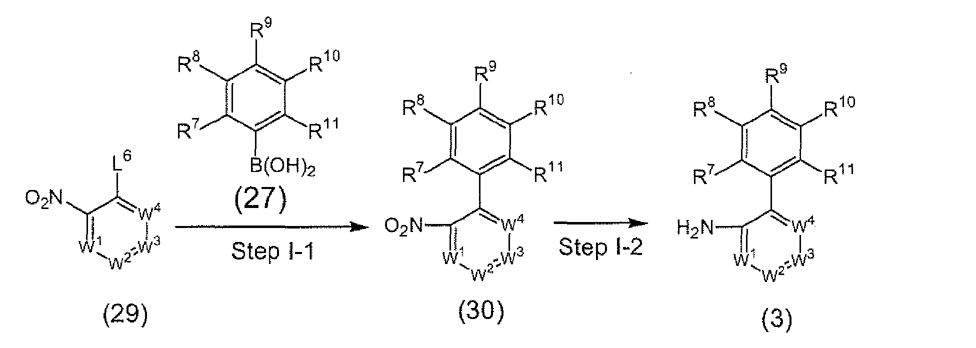

(, wherein, in the formula, L⁶ represents a chlorine atom, a bromine atom and an iodine atom; and R⁷, R⁸, R⁹, R¹⁰, R¹¹ and W¹―W²=W³― W⁴ represent same meaning defined in [Invention 1].)

### (Step I-1)

The compound (30) can be produced by subjecting the compound (29) to reaction with the compound (27).

Said reaction is carried out in a solvent with the presence of a catalyst.

The solvent used for the reaction includes, alcohols such as methanol, ethanol, propanol, butanol, 2-propanoland and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; water and the mixture thereof.

The catalyst used for the reaction includes palladium acetate, tetrakistriphenylphosphine palladium, {1,1' -bis(diphenylphosphino)ferrocene}dichloropalladium methylene chloride complex and bis(triphenylphosphine) palladium dichloride.

The molar ratio applied to the compound (27) is usually from 1 to 5 moles per 1 mole of the compound (29) and that to the catalyst is usually from 0.001 to 0.1 moles.

Saidreactionmaybe, if necessary, carried out in the presence of a base (, which includes inorganic base such as sodium acetate, potassium acetate, potassium carbonate, tripotassium phosphate, sodium bicarbonate and the like) and/or a phase-transfer catalyst (, which includes quaternary ammonium salts such as tetrabutylammonium bromide, benzyltriethylammonium bromide and the like).

The reaction time is usually in the range of from 0.5 to 24 hours, and the reaction temperature is in the range of from 50 to 120 °C.

After completion of the reaction, the compound (30) can be isolated by being subjected to post treatment procedure exemplified below.
(1) A method to extract the reaction mixture with an organic solvent, followed by the organic layer being dried and concentrated
(2) A method to filtrate the reaction mixture, after addition with an organic solvent, if necessary, followed by the filtrate being concentrated

The isolated compound (30) can be purified by a technique chromatography, recrystallization and the like.

### (Step I-2)

The compound (3) can be produced by subjecting the compound (30) to reduction reaction.

Said reduction reaction includes a method to reduce the compound (30) by hydrogen in the presence of hydrogenation catalyst (, said method is referred to as (Step I-2-1) hereinafter), and a method to reduce the compound (30) by iron in the presence of an acidic water (, said method is referred to as (Step I-2-2) hereinafter).

### (Step I-2-1)

The reaction of this step is usually carried out in a solvent.

The solvent used for the reaction of the present step includes alcohols such as methanol, ethanol and the like; and ethers such as 1,4-dioxane, tetrahydrofuran and the like.

The hydrogenation catalyst used for the reaction includes palladium catalysts such as palladium-carbon and platinum catalysts such as platinum-carbon.

The molar ratio applied to hydrogen is usually from 3 to 10 moles per 1 mole of the compound (30) and that to the catalyst is usually from 0.001 to 0.1 moles.

The reaction time is usually in the range of from 1 to 24 hours, and the reaction temperature is in the range of from 20 to 50 °C.

After completion of the reaction, the reaction mixture is subjected to post treatment procedure such as filtrating, subsequently concentrating the filtrate, to isolate the compound (3). The isolated compound (3) can be purified by a technique such as chromatography, recrystallization and the like.

### (Step I-2-2)

The acidic waters used for the reaction in this step include aqueous acetic acid, diluted hydrochloric acid, aqueous sulfuric acid and the like. The reaction may be, if necessary, carried out in the presence of esters such as ethylacetate and the like; and ethers such as tetrahydrofuran and the like. The iron used for the reaction is usually used in the form of powder.

The molar ratio applied to the iron is usually from 6 to 30 moles per 1 mole of the compound (30).

The reaction time is usually in the range of from 0.1 to 10 hours, and the reaction temperature is in the range of from 40 to 100 °C.

After completion of the reaction, the reaction mixture is subjected to filtration, the filtrate obtained being washed by a basic water (such as aqueous saturated sodium bicarbonate and the like) and then being subjected to extraction with organic solvent, subsequently drying and concentrating the organic layer, to isolate the compound (3). The isolated compound (3) can be purified by a technique such as chromatography, recrystallization and the like.

### (Intermediate Compound Production Method 2)

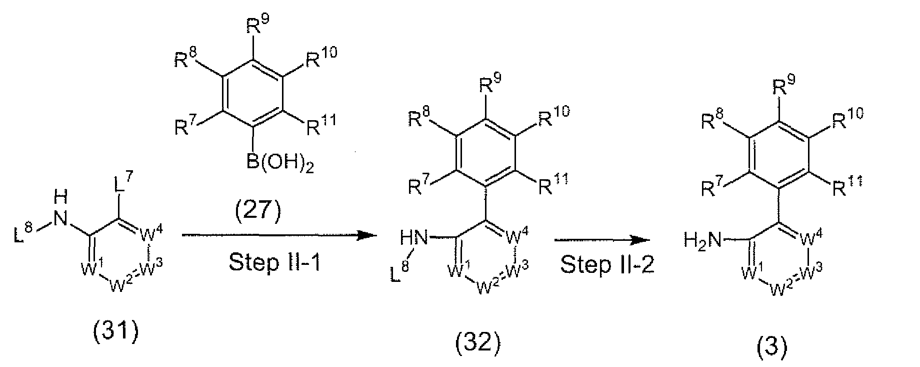

(, wherein, in the formula, L⁷ represents a chlorine atom, a bromine atom and an iodine atom, L⁸ represents a C1-C5 alkylcarbonyl group (an acetyl group, a pivaloyl group and the like) , and R⁷, R⁸, R⁹, R¹⁰, R¹¹ and W¹―W²=W³―W⁴ represent same meaning defined in [Invention 1].)

### (Step II-1)

The compound (32) can be produced by subjecting the compound (31) to reaction with the compound (27).

Said reaction is carried out in a solvent with the presence of a catalyst.

The solvent used for the reaction includes alcohols such as methanol, ethanol, propanol, butanol, 2-propanol and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; and water and the mixture thereof.

The catalyst used for the reaction includes palladium acetate, tetrakistriphenylphosphine palladium, {1,1' -bis(diphenylphosphino)ferrocene}dichloropalladium methylene chloride complex and bis(triphenylphosphine) palladium dichloride.

The molar ratio applied to the compound (27) is usually from 1 to 5 moles per 1 mole of the compound (31) and that to the catalyst is usually from 0.001 to 0.1 moles.

Said reaction maybe, if necessary, carried out in the presence of a base (, which includes inorganic base such as sodium acetate, potassium acetate, potassium carbonate, tripotassium phosphate, sodium bicarbonate and the like) and/or a phase-transfer catalyst (, which includes quaternary ammonium salts such as tetrabutylammonium bromide, benzyltriethylammonium bromide and the like).

The reaction time is usually in the range of from 0.5 to 24 hours, and the reaction temperature is in the range of from 50 to 120 °C.

After completion of the reaction, the compound (32) can be isolated by being subjected to post treatment procedure exemplified below.
(1) A method to extract the reaction mixture with an organic solvent, followed by the organic layer obtained being dried and concentrated
(2) A met hod to filtrate the reaction mixture followed by the filtrate being concentrated
(3) A method to concentrate the reaction mixture as itself The isolated compound (3) can be purified by a technique such as chromatography, recrystallization and the like.

### (Step II-2)

The compound (3) can be produced by subjecting the compound (32) tohydrolysis in the presence of abase (suchassodiumhydroxide, potassium hydroxide and the like).

The reaction is carried out in a solvent.

The solvent used for the reaction includes mixtures with water and alcohols such as methanol, ethanol and the like or ethers such as 1,4-dioxane and the like.

The reaction time is usually in the range of from 1 to 24 hours, and the reaction temperature is in the range of from 20 to 100 °C.

After completion of the reaction, the reaction mixture is subjected to post treatment procedure such as extracting with organic solvent, subsequently drying and concentrating the organic layer, to isolate the compound (3). The isolated compound (3) can be purified by a technique such as chromatography, recrystallization and the like.

The compound (2) can be produced according to the (Intermediate Compound Production Method 3). That is, the compound (2) can be produced by subjecting the compound (34) to reaction with a halogenating agent (such as thionyl chloride, thionyl bromide and the like).

### (Intermediate Compound Production Method 3)

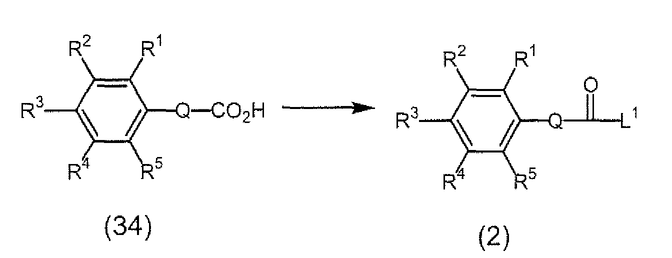

(, wherein, in the formula, L¹ represents a chlorine atom or a bromine atom; and R¹, R², R³, R⁴, R⁵ and Q represent same meaning defined in [Invention 1].)

Said reaction is usually carried out in a solvent.

The solvent used for the reaction includes aromatic hydrocarbons such as toluene, xylene and the like.

The molar ratio applied to the halogenating agent is usually from 1 to 5 moles per 1 mole of the compound (34).

The reaction time is usually in the range of from 0.5 to 24 hours, and the reaction temperature is in the range of from 50 to 120 °C.

After completion of the reaction, the compound (2) can be isolated from the reaction mixture by being subjected to post treatment procedure such as concentration and the like.

Some of the compound (34) can be produced according to the methods described in International Publication WO01/95721, International Publication WO00/41998, International Publication WO96/23763 or International Publication WO96/31464.

Furthermore, the compound (50) among the compound (34) can be produced according to (Intermediate Compound Production Method 4).

### (Intermediate Compound Production Method 4)

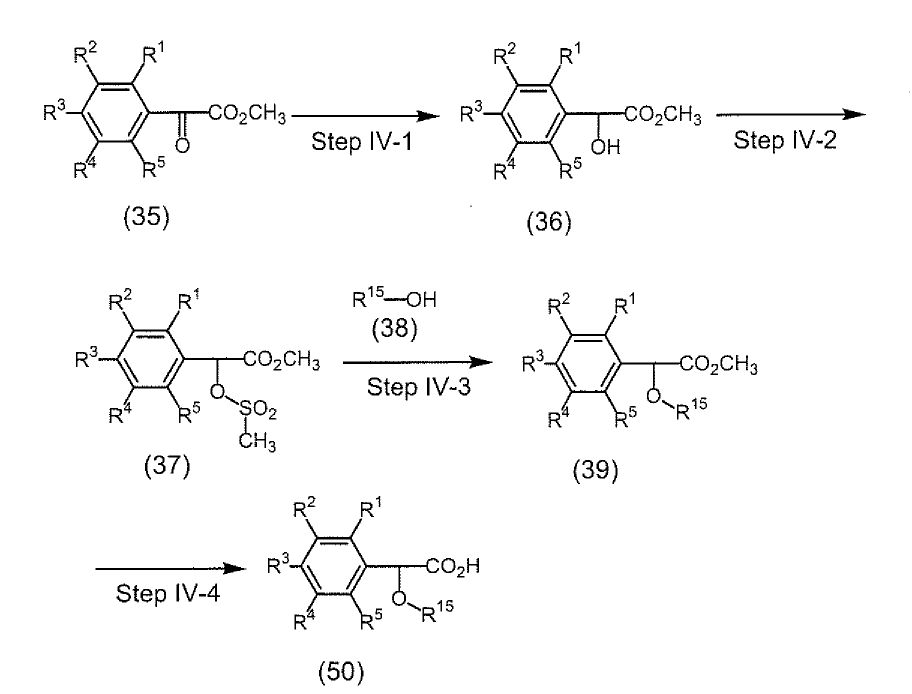

(, wherein, in the formula, R¹, R², R³, R⁴ and R⁵ represent same meaning defined in [Invention 1] and R¹⁵ represents same meaning described above.)

### (Step IV-1)

The compound (36) can be produced by subjecting the compound (35) to react with a reducing agent.

Said reaction is carried out in a solvent.

The reducing agent used for the reaction includes sodium borohydride and potassium borohydride.

The solvent used for the reaction includes alcohols such as methanol, ethanol, 2-propanol and the like; ethers such as 1,4-dioxane, tetrahydrofuran, ethyleneglycoldimethylether, tert-butylmethylether and the like; aliphatic hydrocarbons such as hexane, heptane, octane and the like ; aromatic hydrocarbons such as toluene, xylene and the like ; and water and the mixture thereof.

The molar ratio applied to the reducing agent is usually from 0.25 to 3 moles per 1 mole of the compound (35).

The reaction time is usually in the range of from the instant to 24 hours, and the reaction temperature is in the range of from -20 to 100 °C.

After completion of the reaction, the reaction mixture is subjected to post treatment procedure such as extracting with organic solvent, subsequently drying and concentrating the organic layer, to isolate the compound (36). The isolated compound (36) can be purified by a technique such as chromatography, recrystallization and the like.

### (Step IV-2)

The compound (37) can be produced by subjecting the compound (36) to react with methanesulfonylchloride.

Said reaction is carried out with the presence of a base usually in a solvent.

The solvent used for the reaction includes, ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to methanesulfonyl chloride is usually from 1 to 3 moles per 1 mole of the compound (36) and that of the base is usually from 1 to 10 moles.

The reaction time is usually in the range of from 1 to 24 hours, and the reaction temperature is in the range of from -20 to 100 °C.

After completion of the reaction, the reaction mixture is subjected to post treatment procedure such as concentration, the concentrated residue being added with an organic solvent to be filtrated, subsequently concentrating the filtrate, to isolate the compound (37).

### (Step IV-3)

The compound (39) can be produced by subjecting the compound (37) to react with the compound (38).

Said reaction is usually carried out in a solvent with the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the compound (38) is usually from 1 to excessive amount per 1 mole of the compound (37) and that of the base is usually from the catalytic amount to 5 moles.

The reaction time is usually in the range of from 1 to 24 hours, and the reaction temperature is in the range of from 50 to 150 °C.

After completion of the reaction, the compound (39) can be isolated by being subjected to post treatment procedure exemplified below.
(1) A method to extract the reaction mixture with an organic solvent, followed by the organic layer being dried and concentrated
(2) A method to concentrate the reaction mixture, after addition with an organic solvent such as toluene or the like if necessary The isolated compound (39) can be purified by a technique such as recrystallization and the like.

### (Step IV-4)

The compound (50) can be produced by subjecting the compound (39) to hydrolysis in the presence of a base (such as sodium hydroxide, potassium hydroxide and the like).

The reaction is usually carried out in a solvent such as mixture of water and an alcohol (, for example, methanol, ethanol and the like) and the like.

The molar ratio applied to the base is usually from 1 to 20 moles per 1 mole of the compound (39).

The reaction time is usually in the range of from 0.5 to 24 hours, and the reaction temperature is in the range of from 0 to 120 °C.

After completion of the reaction, the reaction mixture is subjected to post treatment procedure, such as, after being made acidity with an acid such as hydrochloric acid and the like, extracting with organic solvent, subsequently drying and concentrating the organic layer, to isolate the compound (50). The isolated compound (50) can be purified by a technique such as chromatography, recrystallization and the like.

The compound (100) in the compound (50) wherein R¹, R⁴ and R⁵ are hydrogen atoms and R¹⁵ is a 2-propynyl group, is one of the important production intermediates in the present invention. 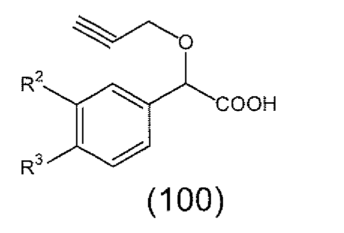 (, wherein, in the formula, R² and R³ independently represent a hydrogen atom, a halogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C1-C6 alkylthio group, a C1-C6 haloalkylthio group, a C3-C6 cycloalkyl group, a C3-C6 cycloalkoxy group or a cyano group; or R² and R³ are combined at the terminals thereof to represent a trimethylene, a tetramethylene or -CH=CH ―CH=CH―.)

The compound (43) can be produced according to, for example, (Intermediate Compound Production Method 5).

### (Intermediate Compound Production Method 5)

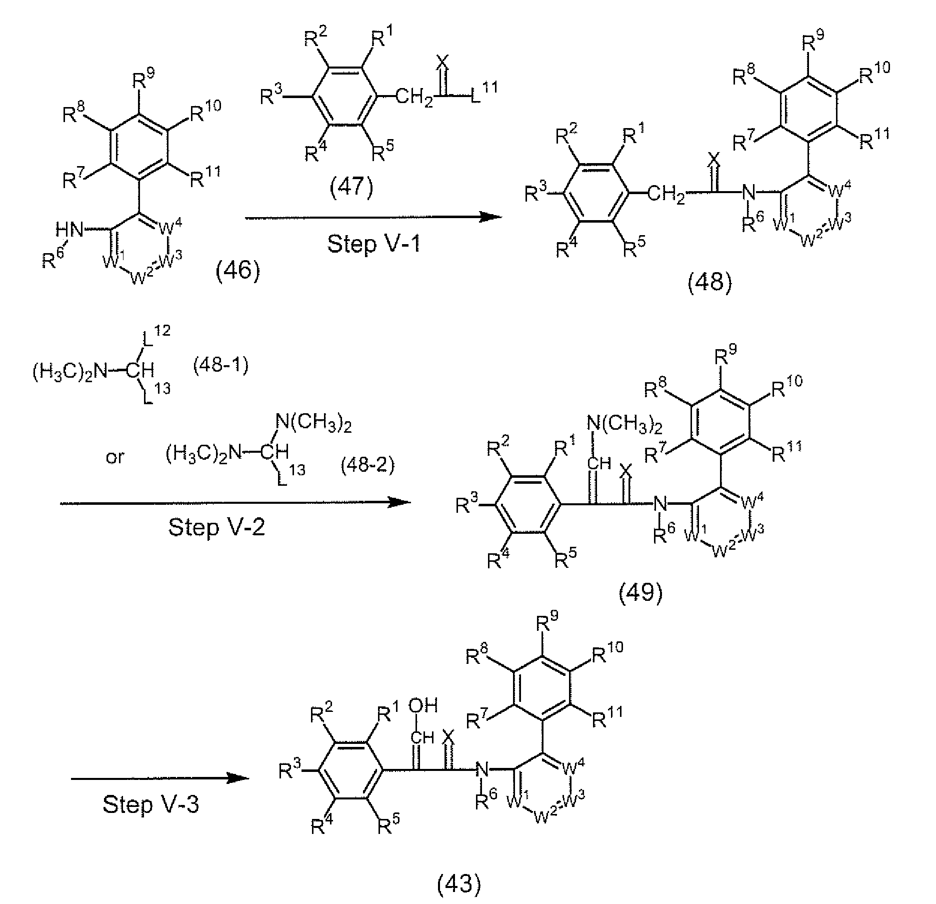

[, wherein, in the formula, L¹¹ represents a chlorine atom or a bromine atom; L¹² and L¹³ are same or different each other and represent an alkoxy group such as a methoxy group and the like; R¹, R², R³, R⁴, R⁵, R⁶, R⁷, R⁸ R⁹, R¹⁰, R¹¹ and W¹―W²=W³―W⁴ represent same meaning defined in [Invention 1]; and X represents same meaning described above.]

### (Step V-1)

The compound (48) can be produced by subjecting the compound (46) to react with the compound (47).

Said reaction is usually carried out in a solvent with the presence of a base.

The solvent used for the reaction includes, ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters butylacetate, ethylacetate and the like; organic nitriles such as acetonitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; alkali metal hydrides such as sodium hydride and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the compound (47) is usually from 0.5 to 2 moles per 1 mole of the compound (46) and that to the base is from 1 to 10 moles.

The reaction time is usually in the range of from 0.1 to 24 hours, and the reaction temperature is in the range of from 0 to 150 °C.

After completion of the reaction, the reaction mixture is subjected to post treatment procedure such as extracting with organic solvent, subsequently drying and concentrating the organic layer, to isolate the compound (48). The isolated compound (48) can be purified by a technique such as chromatography, recrystallization and the like.

### (Step V-2)

The compound (49) can be produced by subjecting the compound (48) to react with the compound (48-1) or the compound (48-2).

Said reaction is usually carried out in a solvent.

The solvent used for the reaction includes ethers such as 1,4-dioxane, tetrahydrofuran, ethyleneglycoldimethylether, t-butylmethylether and the like, aliphatic hydrocarbons such as hexane, heptane and the like, aromatic hydrocarbons such as toluene and the like, halogenated hydrocarbons such as chlorobenzene and the like, organic bases such as pyridine, triethylamine, N,N-dimethylaniline and the like, N,N-dimethylformamide or the mixture thereof.

The molar ratio applied to the compound (48-1) or the compound (48-2) is usually from 1 to 10 moles per 1 mole of the compound (48).

The reaction time is usually in the range of from 1 to 24 hours, and the reaction temperature is in the range of from 50 to 150 °C.

After completion of the reaction, the reaction mixture is subjected to post treatment procedure such as extracting with organic solvent, subsequently drying and concentrating the organic layer, to isolate the compound (49). The isolated compound (49) can be purified by a technique such as chromatography, recrystallization and the like.

### (Step V-3)

The compound (43) can be produced by subjecting the compound (49) to react with water in the presence of an acid.

Said reaction may be carried out in water as the solvent or in the mixture of water and other organic solvents.

The other organic solvents include ethers such as 1, 4-dioxane, tetrahydrofuran, ethyleneglycoldimethylether, t-butylmethylether and the like, aliphatic hydrocarbons such as hexane, heptane and the like, aromatic hydrocarbons such as toluene and the like, halogenated hydrocarbons such as chlorobenzene and the like, organic nitriles such as acetonitrile and the like; N,N-dimethylformamide, dimethylsulfoxide and the mixture thereof.

The acid used for the reaction includes hydrochloric acid, sulfuric acid, p-toluenesulfonic acid and the like.

The molar ratio applied to the acid is usually from 0.1 to 100 moles per 1 mole of the compound (49).

The reaction time is usually in the range of from 1 to 24 hours, and the reaction temperature is in the range of from 20 to 100 °C.

After completion of the reaction, the reaction mixture is subjected to post treatment procedure such as extracting with organic solvent, subsequently drying and concentrating the organic layer, to isolate the compound (43). The isolated compound (43) can be purified by a technique such as chromatography, recrystallization and the like.

The compound (26) can be produced according to (Intermediate Compound Production Method 6).

### (Intermediate Compound Production Method 6)

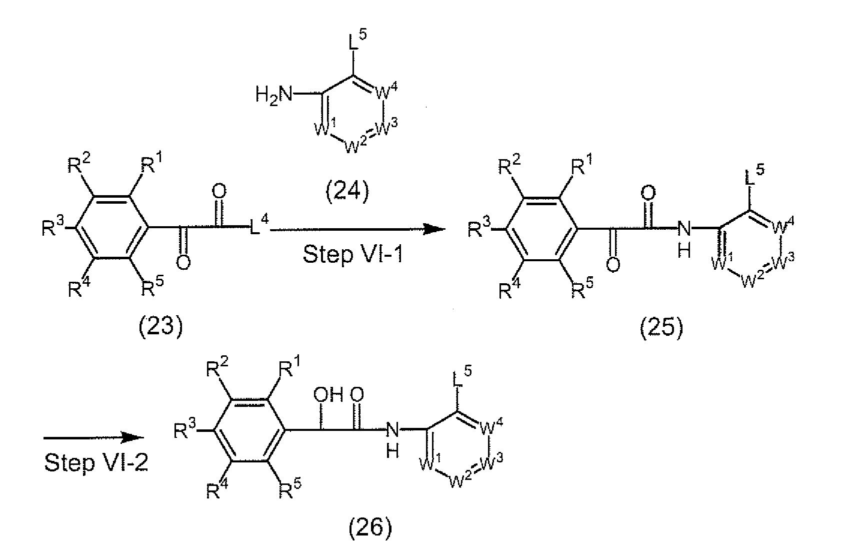

(, wherein, in the formula, L⁴ represents a chlorine atom or a bromine atom, L⁵ represents a chlorine atom, a bromine atom or an iodine atom, and R¹, R², R³, R⁴, R⁵ and W¹―W²=W³―W⁴ represent same meaning defined in [Invention 1].)

### (Step VI-1)

The compound (25) can be produced by subjecting the compound (23) to react with the compound (24).

Said reaction is usually carried out in a solvent with the presence of a base.

The solvent used for the reaction includes ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; halogenated hydrocarbons such as chlorobenzene and the like; esters such as ethylacetate, butylacetate and the like; organic nitriles such as acetonitrile, butyronitrile and the like; acid amides such as N,N-dimethylformamide and the like; sulfoxides such as dimethylsulfoxide and the like; and the mixture thereof.

The base used for the reaction includes carbonates such as sodium carbonate, potassium carbonate and the like; tertiary amines such as triethylamine, diisopropylethylamine, 1,8-diazabicyclo[5.4.0]undec-7-ene, 1,5-diazabicyclo[4.3.0]non-5-ene and the like; and nitrogen-contained aromatic compounds such as pyridine, 4-dimethylaminopyridine and the like.

The molar ratio applied to the compound (23) is usually from 1 to 3 moles per 1 mole of the compound (23) and that of the base is usually from 1 to 10 moles.

The reaction time is usually in the range of from 1 to 24 hours, and the reaction temperature is in the range of from -20 to 100 °C.

After completion of the reaction, the reaction mixture is subjected to post treatment procedure such as extracting with organic solvent, if necessary, the organic layer being washed by acidic water, basic water and the like, subsequently drying and concentrating the organic layer, to isolate the compound (25). The isolated compound (25) can be purified by a technique such as chromatography, recrystallization and the like.

### (Step VI-2)

The compound (26) can be produced by subjecting the compound (25) to react with a reducing agent.

Said reaction is carried out in a solvent.

The reducing agent used for the reaction includes sodium borohydride and potassium borohydride.

The solvent used for the reaction includes alcohols such as methanol, ethanol, 2-propanol and the like; ethers such as 1,4-dioxane, tetrahydrofuran, ethyleneglycoldimethylether, tert-butylmethylether and the like; aliphatic hydrocarbons such as hexane, heptane, octane and the like; aromatic hydrocarbons such as toluene, xylene and the like; water and the mixture thereof.

The molar ratio applied to the reducing agent is usually from 0.25 to 3 moles per 1 mole of the compound (25).

The reaction time is usually in the range of from the instant to 24 hours, and the reaction temperature is in the range of from -20 to 100 °C.

After completion of the reaction, the reaction mixture is subjected to post treatment procedure such as extracting with organic solvent after being added to an acidic water such as aqueous saturated ammonium chloride and the like, the organic layer obtained being washed by basic water, subsequently drying and concentrating the organic layer, to isolate the compound (26). The isolated compound (26) can be purified by a technique such as chromatography, recrystallization and the like.

The specific examples of the compound of the present invention are illustrated below:
A compound represented by the formula (I) 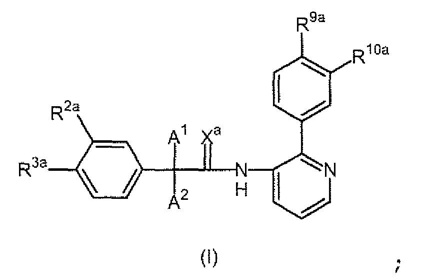 a compound represented by the formula (II) 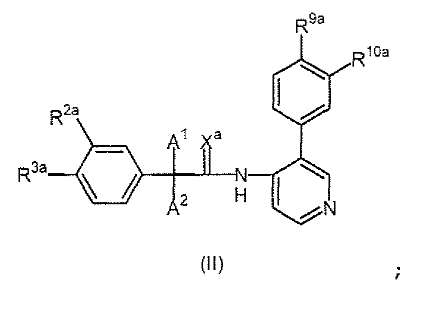 a compound represented by the formula (III) 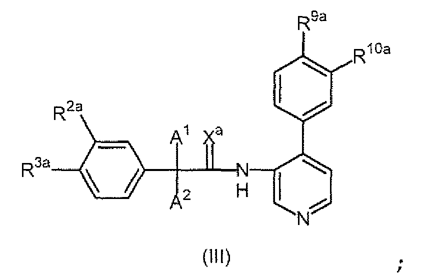 a compound represented by the formula (IV) 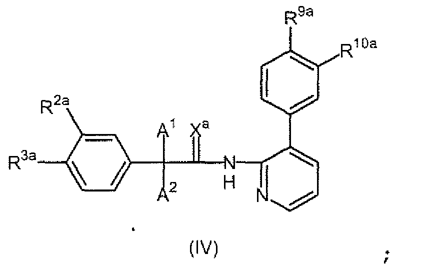 , wherein, in the formula (I) to (IV), R^{2a}, R^{3a}, A¹, A², R^{9a}, R^{10a} and X^{a} represent the combination of substituted groups exhibited in (Table 1).

**Table 1**

| R^{2a} | R^{3a} | A¹ | A² | R^{9a} | R^{10a} | X^{a} |
|---|---|---|---|---|---|---|
| H | H | H | OCH₂C≡CH | OCH₃ | OCH₃ | O |
| H | F | H | OCH₂C≡CH | OCH₃ | OCH₃ | O |
| H | Cl | H | OCH₂C≡CH | OCH₃ | OCH₃ | O |
| H | Br | H | OCH₂C≡CH | OCH₃ | OCH₃ | O |
| H | CH₃ | H | OCH₂C≡CH | OCH₃ | OCH₃ | O |
| H | CH₂CH₃ | H | OCH₂C≡CH | OCH₃ | OCH₃ | O |
| H | CH₂CH₂CH₃ | H | OCH₂C≡CH | OCH₃ | OCH₃ | O |
| H | CH(CH₃)₂ | H | OCH₂C≡CH | OCH₃ | OCH₃ | O |
| H | CH=CH₂ | H | OCH₂C≡CH | OCH₃ | OCH₃ | O |
| H | C≡CH | H | OCH₂C≡CH | OCH₃ | OCH₃ | O |
| H | C≡CCH₃ | H | OCH₂C≡CH | OCH₃ | OCH₃ | O |
| H | OCH₃ | H | OCH₂C≡CH | OCH₃ | OCH₃ | O |
| H | OCH₂CH₃ | H | OCH₂C≡CH | OCH₃ | OCH₃ | O |
| -CH₂CH₂CH₂- | | H | OCH₂C≡CH | OCH₃ | OCH₃ | O |
| -CH₂CH₂CH₂CH₂- | | H | OCH₂C≡CH | OCH₃ | OCH₃ | O |
| -CH=CH-CH=CH- | | H | OCH₂C≡CH | OCH₃ | OCH₃ | O |
| CH₃ | H | H | OCH₂C≡CH | OCH₃ | OCH₃ | O |
| Cl | H | H | OCH₂C≡CH | OCH₃ | OCH₃ | O |
| CH₃ | CH₃ | H | OCH₂C≡CH | OCH₃ | OCH₃ | O |
| Cl | Cl | H | OCH₂C≡CH | OCH₃ | OCH₃ | O |
| H | H | H | OCH₂C≡CH | OCH₃ | OCH₃ | S |
| H | Cl | H | OCH₂C≡CH | OCH₃ | OCH₃ | S |
| H | Br | H | OCH₂C≡CH | OCH₃ | OCH₃ | S |
| H | CH₃ | H | OCH₂C≡CH | OCH₃ | OCH₃ | S |
| -CH₂CH₂CH₂CH₂- | | H | OCH₂C≡CH | OCH₃ | OCH₃ | S |
| H | H | H | OH | OCH₃ | OCH₃ | O |
| H | F | H | OH | OCH₃ | OCH₃ | O |
| H | Cl | H | OH | OCH₃ | OCH₃ | O |
| H | Br | H | OH | OCH₃ | OCH₃ | O |
| H | CH₃ | H | OH | OCH₃ | OCH₃ | O |
| H | CH₂CH₃ | H | OH | OCH₃ | OCH₃ | O |
| H | CH₂CH₂CH₃ | H | OH | OCH₃ | OCH₃ | O |
| H | CH(CH₃)₂ | H | OH | OCH₃ | OCH₃ | O |
| H | CH=CH₂ | H | OH | OCH₃ | OCH₃ | O |
| H | C≡CH | H | OH | OCH₃ | OCH₃ | O |
| H | C≡CCH₃ | H | OH | OCH₃ | OCH₃ | O |
| H | OCH₃ | H | OH | OCH₃ | OCH₃ | O |
| H | OCH₂CH₃ | H | OH | OCH₃ | OCH₃ | O |
| -CH₂CH₂CH₂- | | H | OH | OCH₃ | OCH₃ | O |
| -CH₂CH₂CH₂CH₂- | | H | OH | OCH₃ | OCH₃ | O |
| -CH=CH-CH=CH- | | H | OH | OCH₃ | OCH₃ | O |
| CH₃ | H | H | OH | OCH₃ | OCH₃ | O |
| Cl | H | H | OH | OCH₃ | OCH₃ | O |
| CH₃ | CH₃ | H | OH | OCH₃ | OCH₃ | O |
| Cl | Cl | H | OH | OCH₃ | OCH₃ | O |
| H | H | H | OH | OCH₃ | OCH₃ | S |
| H | Cl | H | OH | OCH₃ | OCH₃ | S |
| H | Br | H | OH | OCH₃ | OCH₃ | S |
| H | CH₃ | H | OH | OCH₃ | OCH₃ | S |
| -CH₂CH₂CH₂CH₂- | | H | OH | OCH₃ | OCH₃ | S |
| H | H | H | OCH₃ | OCH₃ | OCH₃ | O |
| H | F | H | OCH₃ | OCH₃ | OCH₃ | O |
| H | Cl | H | OCH₃ | OCH₃ | OCH₃ | O |
| H | Br | H | OCH₃ | OCH₃ | OCH₃ | O |
| H | CH₃ | H | OCH₃ | OCH₃ | OCH₃ | O |
| H | CH₂CH₃ | H | OCH₃ | OCH₃ | OCH₃ | O |
| H | CH₂CH₂CH₃ | H | OCH₃ | OCH₃ | OCH₃ | O |
| H | CH(CH₃)₂ | H | OCH₃ | OCH₃ | OCH₃ | O |
| H | CH=CH₂ | H | OCH₃ | OCH₃ | OCH₃ | O |
| H | C≡CH | H | OCH₃ | OCH₃ | OCH₃ | O |
| H | C≡CCH₃ | H | OCH₃ | OCH₃ | OCH₃ | O |
| H | OCH₃ | H | OCH₃ | OCH₃ | OCH₃ | O |
| H | OCH₂CH₃ | H | OCH₃ | OCH₃ | OCH₃ | O |
| -CH₂CH₂CH₂- | | H | OCH₃ | OCH₃ | OCH₃ | O |
| -CH₂CH₂CH₂CH₂- | | H | OCH₃ | OCH₃ | OCH₃ | O |
| -CH=CH-CH=CH- | | H | OCH₃ | OCH₃ | OCH₃ | O |
| CH₃ | H | H | OCH₃ | OCH₃ | OCH₃ | O |
| Cl | H | H | OCH₃ | OCH₃ | OCH₃ | O |
| CH₃ | CH₃ | H | OCH₃ | OCH₃ | OCH₃ | O |
| Cl | Cl | H | OCH₃ | OCH₃ | OCH₃ | O |
| H | H | H | OCH₃ | OCH₃ | OCH₃ | S |
| H | Cl | H | OCH₃ | OCH₃ | OCH₃ | S |
| H | Br | H | OCH₃ | OCH₃ | OCH₃ | S |
| H | CH₃ | H | OCH₃ | OCH₃ | OCH₃ | S |
| -CH₂CH₂CH₂CH₂- | | H | OCH₃ | OCH₃ | OCH₃ | S |
| H | H | =NOCH₃ | | OCH₃ | OCH₃ | O |
| H | F | =NOCH₃ | | OCH₃ | OCH₃ | O |
| H | Cl | =NOCH₃ | | OCH₃ | OCH₃ | O |
| H | Br | =NOCH₃ | | OCH₃ | OCH₃ | O |
| H | CH₃ | =NOCH₃ | | OCH₃ | OCH₃ | O |
| H | CH₂CH₃ | =NOCH₃ | | OCH₃ | OCH₃ | O |
| H | CH₂CH₂CH₃ | =NOCH₃ | | OCH₃ | OCH₃ | O |
| H | CH(CH₃)₂ | =NOCH₃ | | OCH₃ | OCH₃ | O |
| H | CH=CH₂ | =NOCH₃ | | OCH₃ | OCH₃ | O |
| H | C≡CH | =NOCH₃ | | OCH₃ | OCH₃ | O |
| H | C≡CCH₃ | =NOCH₃ | | OCH₃ | OCH₃ | O |
| H | OCH₃ | =NOCH₃ | | OCH₃ | OCH₃ | O |
| H | OCH₂CH₃ | =NOCH₃ | | OCH₃ | OCH₃ | O |
| -CH₂CH₂CH₂- | | =NOCH₃ | | OCH₃ | OCH₃ | O |
| -CH₂CH₂CH₂CH₂- | | =NOCH₃ | | OCH₃ | OCH₃ | O |
| -CH=CH-CH=CH- | | =NOCH₃ | | OCH₃ | OCH₃ | O |
| CH₃ | H | =NOCH₃ | | OCH₃ | OCH₃ | O |
| Cl | H | =NOCH₃ | | OCH₃ | OCH₃ | O |
| CH₃ | CH₃ | =NOCH₃ | | OCH₃ | OCH₃ | O |
| Cl | Cl | =NOCH₃ | | OCH₃ | OCH₃ | O |
| H | H | =NOCH₃ | | OCH₃ | OCH₃ | S |
| H | Cl | =NOCH₃ | | OCH₃ | OCH₃ | S |
| H | Br | =NOCH₃ | | OCH₃ | OCH₃ | S |
| H | CH₃ | =NOCH₃ | | OCH₃ | OCH₃ | S |
| -CH₂CH₂CH₂CH₂- | | =NOCH₃ | | OCH₃ | OCH₃ | S |
| H | H | H | OCH₂CH₃ | OCH₃ | OCH₃ | O |
| H | F | H | OCH₂CH₃ | OCH₃ | OCH₃ | O |
| H | Cl | H | OCH₂CH₃ | OCH₃ | OCH₃ | O |
| H | Br | H | OCH₂CH₃ | OCH₃ | OCH₃ | O |
| H | CH₃ | H | OCH₂CH₃ | OCH₃ | OCH₃ | O |
| -CH₂CH₂CH₂CH₂- | | H | OCH₂CH₃ | OCH₃ | OCH₃ | O |
| H | H | H | OCH₂CF₃ | OCH₃ | OCH₃ | O |
| H | F | H | OCH₂CF₃ | OCH₃ | OCH₃ | O |
| H | Cl | H | OCH₂CF₃ | OCH₃ | OCH₃ | O |
| H | Br | H | OCH₂CF₃ | OCH₃ | OCH₃ | O |
| H | CH₃ | H | OCH₂CF₃ | OCH₃ | OCH₃ | O |
| -CH₂CH₂CH₂CH₂- | | H | OCH₂CF₃ | OCH₃ | OCH₃ | O |
| H | H | H | OCH₂CH₂CH₃ | OCH₃ | OCH₃ | O |
| H | F | H | OCH₂CH₂CH₃ | OCH₃ | OCH₃ | O |
| H | Cl | H | OCH₂CH₂CH₃ | OCH₃ | OCH₃ | O |
| H | Br | H | OCH₂CH₂CH₃ | OCH₃ | OCH₃ | O |
| H | CH₃ | H | OCH₂CH₂CH₃ | OCH₃ | OCH₃ | O |
| -CH₂CH₂CH₂CH₂- | | H | OCH₂CH₂CH₃ | OCH₃ | OCH₃ | O |
| H | H | H | OCH₂CH=CH₂ | OCH₃ | OCH₃ | O |
| H | F | H | OCH₂CH=CH₂ | OCH₃ | OCH₃ | O |
| H | Cl | H | OCH₂CH=CH₂ | OCH₃ | OCH₃ | O |
| H | Br | H | OCH₂CH=CH₂ | OCH₃ | OCH₃ | O |
| H | CH₃ | H | OCH₂CH=CH₂ | OCH₃ | OCH₃ | O |
| -CH₂CH₂CH₂CH₂- | | H | OCH₂CH=CH₂ | OCH₃ | OCH₃ | O |
| CH₃ | H | H | OCH₂CH₃ | OCH₃ | OCH₃ | O |
| H | H | =NOCH₂CH₃ | | OCH₃ | OCH₃ | O |
| H | F | =NOCH₂CH₃ | | OCH₃ | OCH₃ | O |
| H | Cl | =NOCH₂CH₃ | | OCH₃ | OCH₃ | O |
| H | Br | =NOCH₂CH₃ | | OCH₃ | OCH₃ | O |
| H | CH₃ | =NOCH₂CH₃ | | OCH₃ | OCH₃ | O |
| H | H | =NOCH₂F | | OCH₃ | OCH₃ | O |
| H | F | =NOCH₂F | | OCH₃ | OCH₃ | O |
| H | Cl | =NOCH₂F | | OCH₃ | OCH₃ | O |
| H | Br | =NOCH₂F | | OCH₃ | OCH₃ | O |
| H | CH₃ | =NOCH₂F | | OCH₃ | OCH₃ | O |
| H | H | =CCl₂ | | OCH₃ | OCH₃ | O |
| H | F | =CCl₂ | | OCH₃ | OCH₃ | O |
| H | Cl | =CCl₂ | | OCH₃ | OCH₃ | O |
| H | Br | =CCl₂ | | OCH₃ | OCH₃ | O |
| H | CH₃ | =CCl₂ | | OCH₃ | OCH₃ | O |
| H | H | =CHOCH₂F | | OCH₃ | OCH₃ | O |
| H | F | =CHOCH₂F | | OCH₃ | OCH₃ | O |
| H | Cl | =CHOCH₂F | | OCH₃ | OCH₃ | O |
| H | Br | =CHOCH₂F | | OCH₃ | OCH₃ | O |
| H | CH₃ | =CHOCH₂F | | OCH₃ | OCH₃ | O |
| -CH₂CH₂CH₂CH₂- | | =NOCH₂CH₃ | | OCH₃ | OCH₃ | O |
| -CH₂CH₂CH₂CH₂- | | =NOCH₂F | | OCH₃ | OCH₃ | O |
| -CH₂CH₂CH₂CH₂- | | =CCl₂ | | OCH₃ | OCH₃ | O |
| -CH₂CH₂CH₂CH₂- | | =CHOCH₂F | | OCH₃ | OCH₃ | O |
| -CH₂CH₂CH₂CH₂- | | =CCl₂ | | OCH₃ | OCH₃ | S |
| H | H | H | OCO₂CH₃ | OCH₃ | OCH₃ | O |
| H | F | H | OCO₂CH₃ | OCH₃ | OCH₃ | O |
| H | Cl | H | OCO₂CH₃ | OCH₃ | OCH₃ | O |
| H | Br | H | OCO₂CH₃ | OCH₃ | OCH₃ | O |
| H | CH₃ | H | OCO₂CH₃ | OCH₃ | OCH₃ | O |
| -CH₂CH₂CH₂CH₂- | | H | OCO₂CH₃ | OCH₃ | OCH₃ | O |
| H | H | H | OCO₂CH₂CH₃ | OCH₃ | OCH₃ | O |
| H | F | H | OCO₂CH₂CH₃ | OCH₃ | OCH₃ | O |
| H | Cl | H | OCO₂CH₂CH₃ | OCH₃ | OCH₃ | O |
| H | Br | H | OCO₂CH₂CH₃ | OCH₃ | OCH₃ | O |
| H | CH₃ | H | OCO₂CH₂CH₃ | OCH₃ | OCH₃ | O |
| -CH₂CH₂CH₂CH₂- | | H | OCO₂CH₂CH₃ | OCH₃ | OCH₃ | O |
| H | H | H | OCH₂C≡CCH₃ | OCH₃ | OCH₃ | O |
| H | F | H | OCH₂C≡CCH₃ | OCH₃ | OCH₃ | O |
| H | Cl | H | OCH₂C≡CCH₃ | OCH₃ | OCH₃ | O |
| H | Br | H | OCH₂C≡CCH₃ | OCH₃ | OCH₃ | O |
| H | CH₃ | H | OCH₂C≡CCH₃ | OCH₃ | OCH₃ | O |
| -CH₂CH₂CH₂CH₂- | | H | OCH(CH₃)C≡CCH₃ | OCH₃ | OCH₃ | O |
| H | H | H | OCH (CH₃)C ≡CCH₃ | OCH₃ | OCH₃ | O |
| H | F | H | OCH (CH₃)C≡CCH₃ | OCH₃ | OCH₃ | O |
| H | Cl | H | OCH(CH₃)C ≡CCH₃ | OCH₃ | OCH₃ | O |
| H | Br | H | OCH (CH₃)C≡CCH₃ | OCH₃ | OCH₃ | O |
| H | CH₃ | H | OCH(CH₃)C ≡CCH₃ | OCH₃ | OCH₃ | O |
| -CH₂CH₂CH₂CH₂- | | H | OCH (CH₃)C≡CCH₃ | OCH₃ | OCH₃ | O |
| -CH₂CH₂CH₂- | | H | OCH₂C≡CCH₃ | OCH₃ | OCH₃ | O |
| H | H | H | OCH₂C≡CH | OCH₂CH₃ | OCH₂CH₃ | O |
| H | F | H | OCH₂C≡CH | OCH₂CH₃ | OCH₂CH₃ | O |
| H | Cl | H | OCH₂C≡CH | OCH₂CH₃ | OCH₂CH₃ | O |
| H | Br | H | OCH₂C≡CH | OCH₂CH₃ | OCH₂CH₃ | O |
| H | CH₃ | H | OCH₂C≡CH | OCH₂CH₃ | OCH₂CH₃ | O |
| -CH₂CH₂CH₂CH₂- | | H | OCH₂C≡CH | OCH₂CH₃ | OCH₂CH₃ | O |
| H | H | H | OCH₂C≡CH | OCH₂CH₃ | OCH₃ | O |
| H | F | H | OCH₂C≡CH | OCH₂CH₃ | OCH₃ | O |
| H | Cl | H | OCH₂C≡CH | OCH₂CH₃ | OCH₃ | O |
| H | Br | H | OCH₂C≡CH | OCH₂CH₃ | OCH₃ | O |
| H | CH₃ | H | OCH₂C≡CH | OCH₂CH₃ | OCH₃ | O |
| -CH₂CH₂CH₂CH₂- | | H | OCH₂C≡CH | OCH₂CH₃ | OCH₃ | O |
| H | H | H | OCH₂C≡CH | OCH₂C≡CH | OCH₃ | O |
| H | F | H | OCH₂C≡CH | OCH₂C≡CH | OCH₃ | O |
| H | Cl | H | OCH₂C≡CH | OCH₂C≡CH | OCH₃ | O |
| H | Br | H | OCH₂C≡CH | OCH₂C≡CH | OCH₃ | O |
| H | CH₃ | H | OCH₂C≡CH | OCH₂C≡CH | OCH₃ | O |
| -CH₂CH₂CH₂CH₂- | | H | OCH₂C≡CH | OCH₂C≡CH | OCH₃ | O |
| H | H | H | OCH₂C≡CH | OCH₃ | OCH₂CH₃ | O |
| H | F | H | OCH₂C≡CH | OCH₃ | OCH₂CH₃ | O |
| H | Cl | H | OCH₂C≡CH | OCH₃ | OCH₂CH₃ | O |
| H | Br | H | OCH₂C≡CH | OCH₃ | OCH₂CH₃ | O |
| H | CH₃ | H | OCH₂C≡CH | OCH₃ | OCH₂CH₃ | O |
| -CH₂CH₂CH₂CH₂- | | H | OCH₂CH=CH₂ | OCH₃ | OCH₂CH₃ | O |
| H | CH₃ | H | OCH₂C≡CH | OCH₂CH₃ | OCH₂CH₃ | S |
| H | H | H | OCH₂C≡CH | OCH₃ | OCH₂C≡CH | O |
| H | F | H | OCH₂C≡CH | OCH₃ | OCH₂C≡CH | O |
| H | Cl | H | OCH₂C≡CH | OCH₃ | OCH₂C≡CH | O |
| H | Br | H | OCH₂C≡CH | OCH₃ | OCH₂C≡CH | O |
| H | CH₃ | H | OCH₂C≡CH | OCH₃ | OCH₂C≡CH | O |
| -CH₂CH₂CH₂CH₂- | | H | OCH₂C≡CH | OCH₃ | OCH₂C≡CH | O |

Following is the illustration exemplifying the compound (100) which is the important intermediate in the present invention. A compound represented by the formula (V) 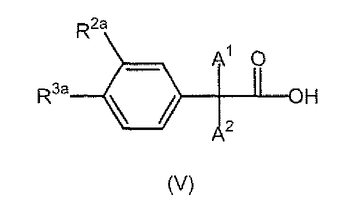 , wherein, in the formula(V), R^{2a}, R^{3a}, A¹, and A² represent the combination of substituted groups exhibited in (Table 2).

**Table 2**

| R^{2a} | R^{3a} | A¹ | A² |
|---|---|---|---|
| H | H | H | OCH₂C≡CH |
| H | F | H | OCH₂C≡CH |
| H | Cl | H | OCH₂C≡CH |
| H | Br | H | OCH₂C≡CH |
| H | CH₃ | H | OCH₂C≡CH |
| H | CH₂CH₃ | H | OCH₂C≡CH |
| H | CH₂CH₂CH₃ | H | OCH₂C≡CH |
| H | CH(CH₃)₂ | H | OCH₂C≡CH |
| H | CH=CH₂ | H | OCH₂C≡CH |
| H | C≡CH | H | OCH₂C≡CH |
| H | C≡CCH₃ | H | OCH₂C≡CH |
| H | OCH₃ | H | OCH₂C≡CH |
| H | OC₂H₅ | H | OCH₂C≡CH |
| -CH₂CH₂CH₂- | | H | OCH₂C≡CH |
| -CH₂CH₂CH₂CH₂- | | H | OCH₂C≡CH |
| -CH=CH-CH=CH- | | H | OCH₂C≡CH |
| CH₃ | H | H | OCH₂C≡CH |
| Cl | H | H | OCH₂C≡CH |
| CH₃ | CH₃ | H | OCH₂C≡CH |
| Cl | Cl | H | OCH₂C≡CH |

Plant diseases against which the compound of the present invention has controlling activity include, for example, a disease due to Phycomycetes, specifically following diseases are illustrated:
*Peronospora brassicae* of vegetables and radishes; *Peronospora spinaciae* of spinach; *Peronospora tabacina* of tobacco;
*Pseudoperonospora cubensis* of gourds; *Plasmopara viticola* of grapes; *Phytophthora cactorum* of apple, strawberry and ginseng;
*Phytophora capsici* of tomato and cucumber; *Phytophthora cinnamomi* of pineapple; *Phytophthora infestans* of potato and tomato;
*Phytophthora nicotianae var. nicotianae* of tobacco, broad bean and leek; *Pythium sp.* of spinach; *Pythium aphanidermatum* of cucumber; *Pythium sp.* of wheat; *Pythium debaryanum* of tobaco; and
*Pythium rot* of soybean (*Pythium aphanidermatum, P. debaryanum, P. irregulare, P. myriotylum, P. ultimum*).

The fungicidal composition of the present invention comprises the compound of the present invention and a carrier. The carrier is inert to the compound of the present invention and appropriately chosen depending on types applying the fungicidal composition of the present invention and the like. The fungicidal composition of the present invention may further comprise adjuvant for formulation such as surfactant and the like depending on the necessity. The fungicidal composition of the present invention includes formulation forms such as emulsifiable concentrate, wettable powder, dry flowable, flowable, dust, granule and the like. The fungicidal composition of the present invention usually contains 0.1 to 90 % by weight of the compound of the present invention.

Solid carriers used for the fungicidal composition of the present invention include, for example, fine powders or granules of minerals such as kaolin clay, attapulgite clay, bentonite, montmorillonite, terra alba, pyrophyllite, talc, diatomaceous earth, calcite and the like; natural organic substances such as corncob powder, walnut shell powder and the like; synthetic organic substances such as urea and the like; salts such as calcium carbonate, ammonium sulfate and the like; synthetic inorganic substances such as synthetic hydrous silicon oxide and the like. Liquid carriers include, for example, aromatic hydrocarbons such as xylene, alkylbenzene, methylnaphthalene and the like; alcohols such as 2-propanol, ethylene glycol, propylene glycol, cellosolve and the like; ketones such as acetone, cyclohexanone, isophorone and the like; vegetable oils such as soybean oil, cottonseed oil and the like; petroleum aliphatic hydrocarbons, esters, dimethylsulfoxide, acetonitrile and water.

Surfactants include, for example, anionic surfactants such as alkylsulfuric acid ester salt, alkylarylsulfonic acid salt, dialkylsulfosuccinic acid salt, polyoxyethylenealkylaryletherphosphoric acid ester salt, lignin sulfonic acid salt, polycondensed naphthalenesulfonateformaldehyde and the like; and nonionic surfactants such as polyoxyethylenealkylarylether, polyoxyethylenealkylpolyoxypropylene block copolymer, sorbitan fatty acid ester and the like.

Another adjuvant for formulation includes, for example, water-soluble polymers such as polyvinylalcohol, polyvinylpyrrolidone and the like; Arabian gum; alginic acid and its salt thereof; polysaccharides such as CMC(carboxymethylcellulose), xanthan gum and the like; inorganic substances such as alminum magnesium silicate, alumina sol and the like; and antiseptics, colorants, PAP(isopropyl acidic phosphate), stabilizers such as BHT and the like.

By applying the fungicidal composition of the present invention to treatment for plants, said plants can be protected from plant diseases; and by applying the fungicidal composition of the present invention to treatment for soils, said plants grown on said soils can be protected from plant diseases.

When the fungicidal composition of the present invention is applied to foliage treatment for plants or soil treatment, the application amount thereof, which may be varied with a kind of control-object plants, a kind and an infestation level of control-object diseases, formulation types, application timings, weather conditions and the like, is usually 1 to 5000 g of the compound of the present invention per 10000 m², preferably 5 to 1000 g.

Emulsifiable concentrate, wettable powder, flowable and the like are usually sprayed after diluted with water. In this case, the concentration of the compound of the present invention is usually in the range of from 0.0001 to 3 % by weight, preferably from 0.0005 to 1 % by weight. Dust, granule and the like are usually directly used without dilution.

The fungicidal composition of the present invention can be also applied in treatment methods such as seed disinfection and the like. The methods include, for example, a method to soak seeds of a plant in the liquid fungicidal composition of the present invention which prepared in 1 to 1000 ppm in terms of concentration of the compound of the present invention, a method to spray or coat seeds of a plant with the liquid fungicidal composition of the present invention which prepared in 1 to 1000 ppm in terms of concentration of the compound of the present invention, and a method to coat seeds of a plant with the powder of the composition controlling plant diseases of the present invention.

The method for controlling plant diseases of the present invention is performed by applying effective amount of the compound of the present invention to a plant or a soil growing the plant in which infection is predictable and/or to a plant or a soil growing the plant in which infection is confirmed.

The fungicidal composition of the present invention is usually used as an agent controlling plant diseases for agriculture or gardening, that is, as an agent controlling plant diseases to control plant diseases on plowed fields, paddy fields, orchards, tea fields, pastures, lawn and the like.

The fungicidal composition of the present invention may be used together with other plant disease controlling compositions, pesticides, acaricides, nematicides, herbicides, plant growth regulators and/or fertilizers.

Examples of the active ingredient of such other plant diseases controlling composition include:
chlorothalonil; fluazinam; dichlofluanid; fosetyl-Al; cyclic imide derivatives such as captan, captafol, folpet and the like;
dithiocarbamate derivatives such as maneb, mancozeb, thiuram, ziram, zineb, propineb and the like; inorganic or organic copper derivatives such as basic copper sulfate, basic copper chloride, copper hydroxide, copper-oxinate and the like; acylalanine derivatives such as metalaxyl, furalaxyl, ofurace, cyprofuram, benalaxyl, oxadixyl and the like; strobilurine like compound such as kresoxim-methyl, azoxystrobin, trifloxystrobin, picoxystrobin, pyraclostrobin, dimoxystrobin and the like; anilinopyrimidine derivatives such as cyprodinil, pyrimethanil, mepanipyrim and the like; phenyl pyrrole derivatives such as fenpiclonil, fludioxonil and the like; imide derivatives such as procymidone, iprodione, vinclozolin and the like; benzimidazole derivatives such as carbendazim, benomyl, thiabendazole, thiophanate methyl and the like; amine derivatives such as fenpropimorph, tridemorph, fenpropidin, spiroxamine and the like; azole derivatives such as propiconazole, triadimenol, prochloraz, penconazole, tebuconazole, flusilazole, diniconazole, bromuconazole, epoxiconazole, difenoconazole, cyproconazole, metconazole, triflumizole, tetraconazole, myclobutanil, fenbuconazole, hexaconazole, fluquinconazole, triticonazole, bitertanol, imazalil, flutriafol and the like; cymoxanil; dimethomorph; famoxyadone, fenamidone; iprovalicarb; benthiavalicarb; cyazofamid, zoxamide, ethaboxam; nicobifen; fenhexamid; quinoxyfen; diethofencarb and acibenzolar S-methyl.

The present invention is further illustrated in detail with production examples, formulation examples, test examples and other manner, but should not be construed to be limited thereto.

At first, the production examples are illustrated as follows.

### Production Example 1

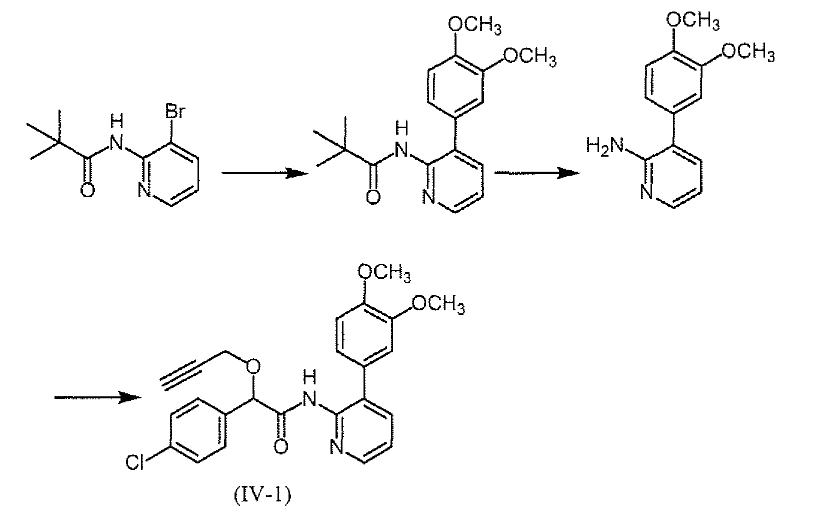

2g of N-(3-bromopyridine-2-yl)-2,2-dimethylpropionamide, 1.4 g of 3,4-dimethoxyphenylboronic acid, 5 g of tripotassium phosphate hydrate, 190 mg of {1,1'-bis(diphenylphosphino)ferrocene}dichloropalladium(II) methylenechloride complex and 50 ml of ethyleneglycoldimethylether were mixed, followed by stirring under a nitrogen atmosphere at 80 °C for 2 hours. Then, the reaction mixture was added to water to be extracted with ethyl acetate. The organic layer was concentrated under reduced pressure and then the concentrated residue obtained was subjected to a silica gel column chromatography (eluent; ethylacetate) to obtain 2.1 g of N-(3-(3,4-dimethoxyphenyl)pyridine-2-yl)-2,2-dimethylpropionamide.
¹H-NMR(CDCl₃,TMS)δ(ppm): 1.15(9H,s), 3.89(3H,s), 3.94(3H,s), 6.87-6.98(3H,m), 7.17(1H,dd,J=4.8Hz,7.6Hz), 7.61(1H,dd,J=2.0Hz,7.6Hz), 7.69(1H,s), 8.47(1H,dd,J=2.0Hz,4.8Hz)

The mixture of 2.1 g of N-(3-(3,4-dimethoxyphenyl)pyridine-2-yl)-2,2-dimethylpropionamide, 20 ml of methanol and 15 ml of aqueous 3M potassium hydroxide was heated under reflux for 4 hours. After being cooled down to room temperature, the reaction mixture was added with water to be extracted with ethylacetate. The organic layer was concentrated under reduced pressure and then the concentrated residue was subjected to a silica gel column chromatography (eluent; ethylacetate) to obtain 1.2 g of 2-amino-3-(3,4-dimethoxyphenyl)pyridine.
¹H-NMR(CDCl₃,TMS)δ(ppm): 3.90(3H,s), 3.93(3H,s), 4.62(2H,s), 6.74(1H,dd,J=4.9Hz,7.3Hz), 6.94-7.01(3H,m), 7.36(1H,dd,J=1.7Hz,7.3Hz), 8.06(1H,dd,J=1.7Hz,4.9Hz)

0.5 g of 2-(2-propynyloxy)-2-(4-chlorophenyl)acetyl chloride (, which was prepared by the method of Referencial Production Example 1 described hereinafter) was added dropwise to the mixture of 0.44 g of 2-amino-3-(3,4-dimethoxyphenyl)pyridine, 0.20 g of triethylamine, 0.05 g of 4-dimethylaminopyridine and 15 ml of tetrahydrofuran, followed by stirring at room temperature for 3 hours. Thereafter, the reaction mixture was added with ethylacetate and then filtrated to remove insolubles. The filtrate was concentrated under reduced pressure and then the residue was subjected to a silica gel column chromatography (eluent; hexane/ethylacetate =1/1) to obtain 0.31 g of N-(3-(3,4-dimethoxyphenyl)pyridine-2-yl)-2-(2-propynyloxy)-2-(4-chlorophenyl) acetamide (, which is referred to as the compound of the present invention (IV-1) hereinafter).
¹H-NMR(CDCl₃,TMS)δ(ppm): 2.47 (1H, t,J=2.4Hz), 3.85(3H,s), 3.94(3H,s), 4.01(1H,dd,J=2.4Hz,15.7Hz), 4.18(1H,dd,J=2.4Hz,15.7Hz), 5.05(1H,s), 6.84-6.88(3H,m), 7.18(1H,dd,J=4.8Hz,7.7Hz), 7.28-7.33(4H,m), 7.63(1H,dd,J=1.7Hz,7.7Hz), 8.47(1H,dd,J=1.7Hz,4.8Hz), 8.79(1H,s)

### Production Example 2

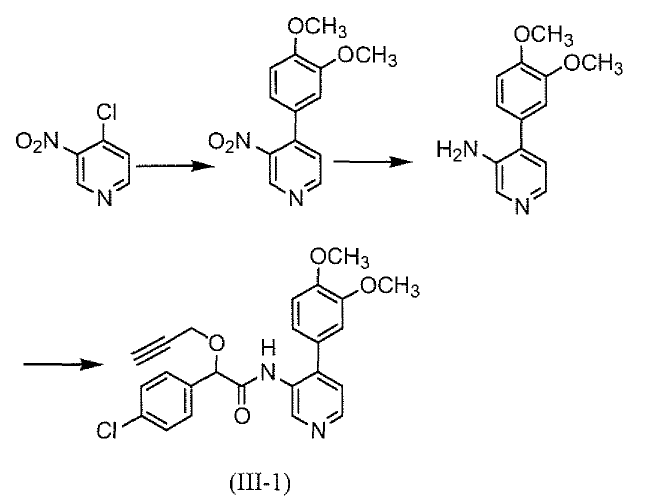

12 g of 4-chloro-3-nitropyridine, 11 g of 3,4-dimethoxyphenylboronic acid, 39 g of tripotassium phosphate hydrate, 988 mg of {1,1'-bis(diphenylphosphino)ferrocene}dichloropalladium(II) methylenechloride complex and 100 ml of ethyleneglycoldimethylether were mixed, followed by stirring under a nitrogen atmosphere at 80 °C for 2 hours. Then, the reaction mixture was added with water to be extracted with ethylacetate. The organic layer was dried by anhydrous magnesium sulfate and then concentrated under reduced pressure, followed by the residue being subjected to a silica gel column chromatography (eluent; hexane/ethylacetate =1/1) to obtain 13 g of 4-(3,4-dimethoxyphenyl)-3-nitropyridine.
¹H-NMR(CDCl₃,TMS)δ(ppm): 3.89(3H,s), 3.94(3H,s), 6.83-6.96(3H,m), 7.42(1H,d,J=4.8Hz), 8.76(1H,d,J=4.8Hz), 9.00(1H,s)

5.0 g of 4-(3,4-dimethoxyphenyl) -3-nitropyridine was added to the mixture of 25 ml of acetic acid, 25 ml of water and 4.3 g of iron powder at 70 °C, followed by stirring for 2 hours. Then, the reaction mixture was added with aqueous sodium bicarbonate to be extracted with ethylacetate. The organic layer was concentrated under reduced pressure and then the residue was subjected to a silica gel column chromatography (eluent; hexane/ethylacetate =1/1) to obtain 3.2 g of 3-amino-4-(3,4-dimethoxyphenyl)pyridine.
¹H-NMR(CDCl₃,TMS)δ(ppm): 3.83 (2H,s), 3.91(3H,s), 3.93(3H,s), 6.95-7.05(4H,m), 8.05(1H,d,J=4.8Hz), 8.14(1H,s)

0.4 g of 2-(2-propynyloxy)-2-(4-chlorophenyl)acetyl chloride was added dropwise to the mixture of 0.40 g of 3-amino-4-(3, 4-dimethoxyphenyl) pyridine, 0.21 g of triethylamine and 10 ml of tetrahydrofuran, followed by stirring at room temperature for 2 hours. Then, the reaction mixture was added with ethyl acetate and then filtrated to remove insolubles. The filtrate was concentrated under reduced pressure and then the residue was subjected to a silica gel column chromatography (eluent; hexane/ethylacetate =1/1) to obtain 0.50 g of N-(4-(3,4-dimethoxyphenyl)pyridine-3-yl)-2-(2-propynyloxy)-2-(4-chlorophenyl) acetamide (, which is referred to as the compound of the present invention (III-1) hereinafter).
¹H-NMR(CDCl₃,TMS)δ(ppm): 2.49(1H,t,J=2.4Hz), 3.90-3.99(7H,m), 4.16(1H,dd,J=2.4Hz,16.0Hz), 5.08(1H,s), 6.88(1H,d,J=2.0Hz), 6.95(1H,dd,J=2.0Hz,8.3Hz), 7.01(1H,d,J=8.3Hz), 7.21(1H,d,J=4.8Hz), 7.29(2H,d,J=8.3Hz), 7.34(2H,d,J=8.3Hz), 8.42(1H,d,J=4.8Hz), 8.70(1H,s), 9.51(1H,s)

### Production Example 3

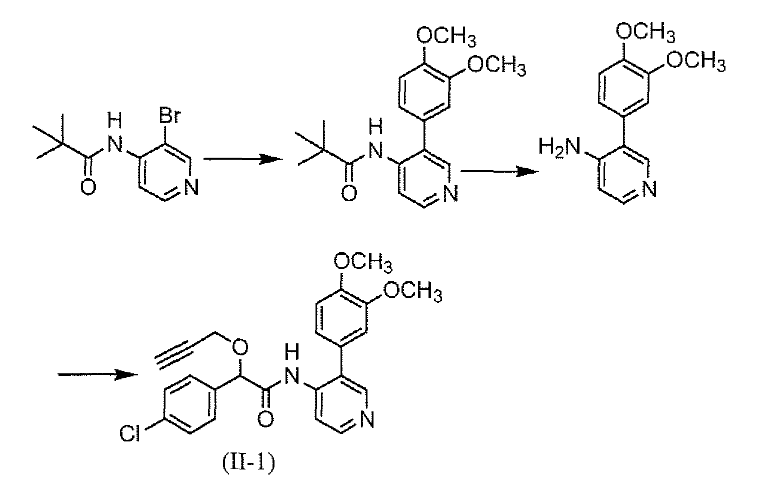

8.5 g of N-(3-bromopyridine-4-yl)-2,2-dimethylpropionamide, 5.0 g of 3,4-dimethoxyphenylboronic acid, 17.5 g of tripotassium phosphate hydrate, 670 mg of {1,1' -bis(diphenylphosphino)ferrocene}dichloropalladium(II) methylenechloride complex and 100 ml of ethyleneglycoldimethylether were mixed, followed by stirring under a nitrogen atmosphere at 80 °C for 2 hours. Then, the reaction mixture was added with water to be extracted with ethylacetate. The organic layer was concentrated under reduced pressure, followed by the residue being subj ected to a silica gel column chromatography (eluent; ethylacetate) to obtain 8 g of N-3-(3,4-dimethoxyphenyl)pyridine-4-yl)-2,2-dimethylpropionam ide.
¹H-NMR(CDCl₃,TMS)δ(ppm): 1.14(9H,s), 3.91(3H,s), 3.97(3H,s), 6.86(1H,d,J=2.0Hz), 6.93(1H,dd,J=2.0Hz,8.0Hz), 7.03(1H,d,J=8.0Hz), 7.81(1H,s), 8.41(1H,s), 8.42(1H,d,J=5.5Hz), 8.50(1H,d,J=5.5Hz)
4.0 g of N-(3-(3,4-dimethoxyphenyl)pyridine-4-yl)-2,2-dimethylpropiona mide, 20 ml of methanol and 15 ml of aqueous 3M potassium hydroxide were mixed, followed by for 4 hours heating with reflux. Then, the reaction mixture was cooled down to room temperature, and then the reaction mixture was added with water to be extracted by ethylacetate. The organic layer was concentrated under reduced pressure and then the residue was subjected to a silica gel column chromatography (eluent; hexane/ethylacetate =1/1) to obtain 2.3 g of 4-amino-3-(3,4-dimethoxyphenyl)pyridine.
¹H-NMR(CDCl₃,TMS)δ(ppm): 3.86(2H,s), 3.93(6H,s), 6.95(1H,d,J=8.3Hz), 7.03-7.05(2H,m), 7.22-7.27(2H,m), 8.11(1H,d,J=2.8Hz)
0.4 g of 2-(2-propynyloxy)-2-(4-chlorophenyl)acetyl chloride was added dropwise to the mixture of 0.40 g of 4-amino-3-(3,4-dimethoxyphenyl)pyridine, 0.25 g of triethylamine and 10 ml of tetrahydrofuran, followed by stirring at room temperature for 3 hours. Thereafter, the reaction mixture was added with ethylacetate and then fittrated to remove insolubles. The filtrate was concentrated under reduced pressure and then the residue was subjected to a silica gel column chromatography (eluent; hexane/ethylacetate =1/1) to obtain 0.72 g of N-(3-(3,4-dimethoxyphenyl)pyridine-4-yl)-2-(2-propynyloxy)-2-(4-chlorophenyl)acetamide (, which is referred to as the compound of the present invention (II-1) hereinafter).

¹H-NMR(CDCl₃,TMS)δ(ppm): 8.99(1H,s), 8.68-8.71-(1H,m), 8.43(1H,dd,J=4.8Hz,1.6Hz), 7.00-7.37(8H,m), 5.09(1H,s), 3.95-4.20(8H,m), 2.47(1H,t,J=2.4Hz)

### Production Example 4

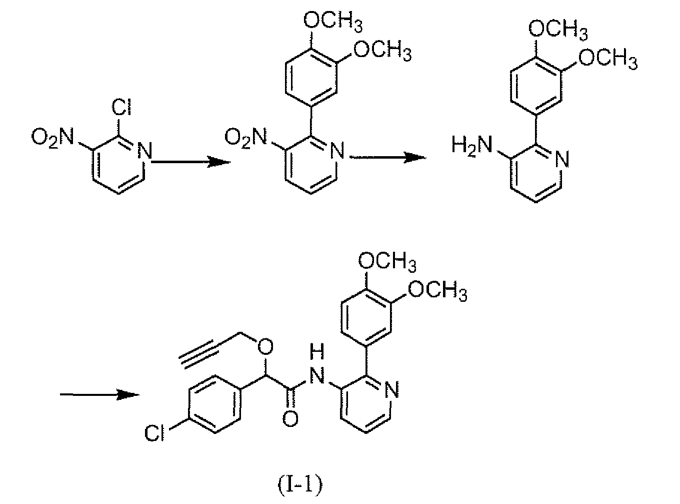

To 50 ml of ethyleneglycoldimethylether, 4.76 g of 2-chloro-3-nitropyridine, 6.00 g of 3,4-dimethoxyphenylboronic acid, 19.1 g of tripotassium phosphate hydrate and 490 mg of {1,1' -bis(diphenylphosphino)ferrocene}dichloropalladium(II) methylenechloride complex were added, followed by stirring under a nitrogen atmosphere at 80°C for 4 hours. Thereafter, the reaction mixture was cooled down to room temperature followed by filtration, and then the filtrate was concentrated under reduced pressure. The residue was subjected to a silica gel column chromatography to obtain 5.35 g of 2-(3,4-dimethoxyphenyl)-3-nitropyridine.
¹H-NMR(CDCl₃,TMS)δ(ppm): 8.83(1H,dd,J=1.2Hz,4.6Hz), 8.07(1H,dd,J=1.1Hz,8.3Hz), 7.39(1H,dd,J=4.6Hz,8.3Hz), 7.19(1H,d,J=2.0Hz), 7.12(1H,dd,J=2.0Hz,8.3Hz), 6.93(1H,d,J=8.3Hz), 3.93(6H,s)
2.8 g of 4-(3,4-dimethoxyphenyl)-3-nitropyridine was dissolved to 100 ml of ethanol at room temperature, followed by addition of 150 mg of 5 % platinum-carbon and then stirring under a hydrogen atmosphere for 4 hours. Thereafter, the reaction mixture was filtrated through celite, followed by the filtrate being concentrated under reduced pressure. The residue was subjected to a silica gel column chromatography (eluent; hexane/ethylacetate =1/1) to obtain 2.1 g of 3-amino-2-(3,4-dimethoxyphenyl)pyridine.
Melting point: 213.5 °C
0.85 g of 2-(2-propynyloxy)-2-(4-chlorophenyl)acetyl chloride was added dropwis to the mixture of 0.90 g of 3-amino-2-(3,4-dimethoxyphenyl) pyridine, 0.40 g of triethylamine and 15 ml of tetrahydrofuran, followed by stirring at room temperature for 2 hours. Thereafter, the reaction mixture was added with ethylacetate and then filtrated to remove insolubles. The filtrate was concentrated under reduced pressure and then the residue was subjected to a silica gel column chromatography (eluent; hexane/ethylacetate =1/1) to obtain 1 g of N-(2-(3,4-dimethoxyphenyl)pyridine-3-yl)-2-(2-propynyloxy)-2-(4-chlorophenyl) acetamide (, which is referred to as the compound of the present invention (I-1) hereinafter).
Melting point: 125.2 °C

### Production Example 5

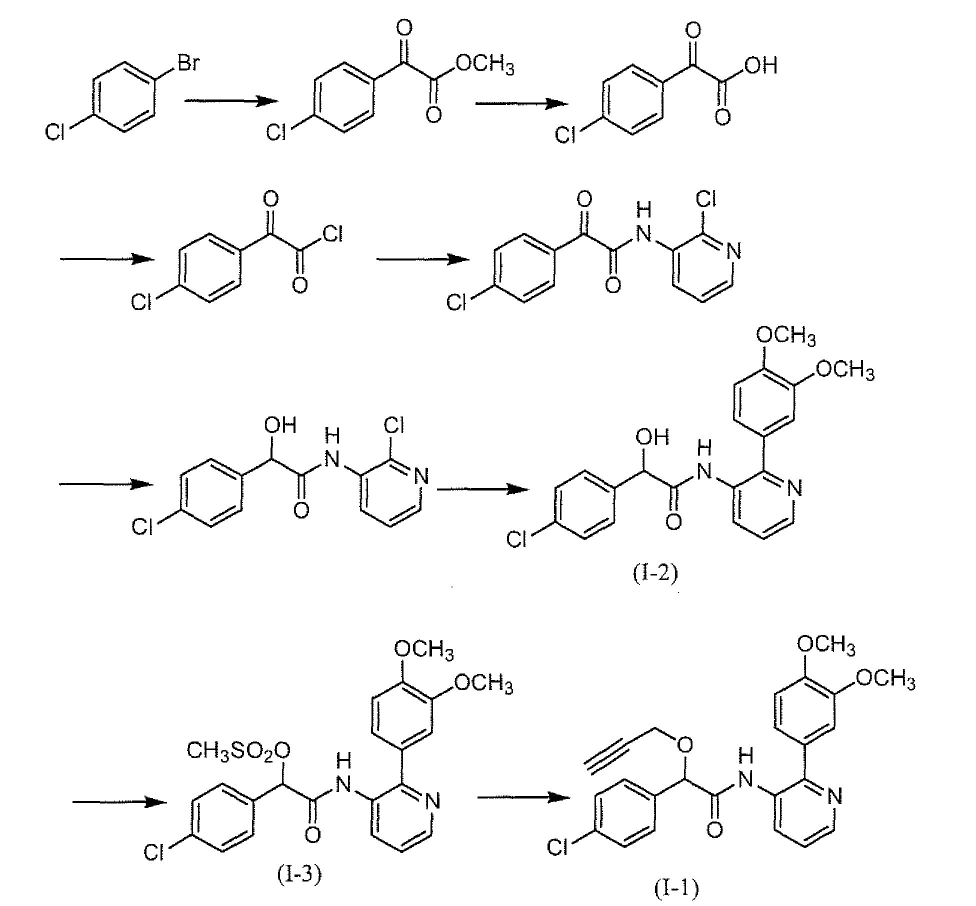

40.0 g of 4-bromochlorobenzene was dissolved to 140 ml of tetrahydrofuran under a nitrogen atmosphere and then added with 5.33 g of magnesium, followed by stirring to prepare Grignard reagent. 750 ml of tetrahydrofuran was dissolved with 49.3 g of dimethyl oxalate, followed by dropping at -70 °C with Grignard reagent aforementioned for 30 minutes. Thereafter, the reaction mixture was warmed to room temperature in 2 hours, followed by stirring at room temperature for 2 hours. Then, the reaction mixture was added with ice water and aqueous saturated ammonium chloride, followed by concentration under reduced pressure. The residue was filtrated, followed by the filtrate being extracted with ethylacetate. The organic layer was washed by aqueous saturated sodium chloride and then dried by magnesium sulfate, followed by concentration under reduced pressure. The residue was subjected to a silica gel column chromatography to obtain 22.1 g of 4-chlorophenylglyoxylic acid methyl ester.
¹H-NMR(CDCl₃,TMS)δ(ppm): 7.98-8.01(2H,m), 7.48-7.51(2H,m), 3.98(3H,s)

To 300 ml of ethanol, 12.2 g of 4-chlorophenylglyoxylic acid methyl ester and 12.6 g of aqueous 20 % sodium hydroxide were added, followed by stirring at room temperature for 3 hours. Thereafter, the reaction mixture was acidified by addition of 36 % hydrochloric acid, followed by extraction in three times with chloroform. The organic layer was dried by anhydrous magnesium sulfate and then concentrated under reduced pressure. The residue was washed by hexane and then dried to obtain 5.2 g of 4-chlorophenylglyoxylic acid.
¹H-NMR(CDCl₃,TMS)δ(ppm): 8.36-8.39(1H,m), 8.0(1H,br), 7.51-7.54(2H,m)

To 20 ml of toluene, 1.5 g of 4-chlorophenylglyoxylic acid, 0.95 ml of thionyl chloride and 20 mg of N,N-dimethylformamide were added, followed by stirring at 80 °C for 1 hour. Thereafter, the reaction mixture was cooled down to room temperature and then concentrated under reduced pressure to obtain crude 4-chlorophenylglyoxyl chloride. To the solution prepared by mixing 1.0 g of 3-amino-2-chloropyridine, 1.7 ml of triethylamine and 20 ml of tetrahydrofuran; 4-methylphenylglyoxyl chloride described above was added at the temperature range of from 0 to 5 °C, followed by stirring at room temperature for 2 hours. Thereafter, the reaction mixture was added with water to be extracted with ethylacetate. The organic layer obtained was successively washed by 5 % hydrochloric acid, aqueous saturated sodium bicarbonate and aqueous saturated sodium chloride and then dried by magnesium sulfate, followed by concentration under reduced pressure. The residue was washed by hexane to obtain 1.5 g of N-(2-chloropyridine-3-yl)-2-oxo-2-(4-chlorophenyl)acetamide.
¹H-NMR(CDCl₃,TMS)δ(ppm): 9.59(1H,s), 8.84(1H,dd,J=8.0Hz,1.6Hz), 8.42(2H,d,J=8.8Hz), 8.22(dd,1H,J=4.8Hz,1.6Hz), 7.52(2H,d,J=8.8Hz), 7.35(dd,1H,J=8.4Hz,4.7Hz)

To 10 ml of ethanol, 1.0 g of N-(2-chloropyridine-3-yl)-2-oxo-2-(4-chlorophenyl)acetamide was dissolved and was added with 38 mg of sodium borohydride at 0 °C, followed by further stirring at room temperature for 1 hour. Thereafter, the reaction mixture was added with aqueous saturated ammonium chloride to be extracted with ethylacetate. The organic layer was successively washed by aqueous saturated sodium bicarbonate and aqueous saturated sodium chloride and then dried by magnesium sulfate, followed by concentration under reduced pressure to obtain 1.0 g of N-(2-chloropyridine-3-yl)-2-hydroxy-2-(4-chlorophenyl)acetami de.
¹H-NMR(CDCl₃,TMS)δ(ppm): 9.00(1H,s), 8.71(1H,dd,J=8.0Hz,1.6Hz), 8.12(dd,1H,J=4.8Hz,2.0Hz), 7.39-7.49(4H,m), 7.24-7.27(1H,m), 5.29(1H,d,J=3.6Hz), 3.30(1H,d, J=3.6Hz)

To 10 ml of ethyleneglycoldimethylether, 0.72 g of N-(2-chloro-pyridine-3-yl)-2-hydroxy-2-(4-chlorophenyl)acetam ide, 1.57 g of 3,4-dimethoxyphenylboronic acid, 1.7 g of tripotassium phosphate hydrate and 63 mg of {1,1' -bis(diphenylphosphino)ferrocene}dichloropalladium(II) methylenechloride complex were mixed, followed by stirring under a nitrogen atmosphere at 80 °C for 3 hours. Thereafter, the reaction mixture was cooled down to room temperature and then filtrated, followed by concentrating the filtrate under reduced pressure. The residue was subjected to a silica gel column chromatography to obtain 0.54 g of N-{2-(3,4-dimethoxyphenyl)pyridine-3-yl}-2-hydroxy-2-(4-chlor ophenyl)acetamide (, which is referred to as the compound of the present invention (I-2) hereinafter).
¹H-NMR(CDCl₃,TMS)δ(ppm): 8.69(1H,dd,J=8.4Hz,1.2Hz), 8.45(1H,s), 8.36(1H,dd,J=4.4Hz,1.6Hz), 7.24-7.33(5H,m), 7.01(1H,s), 6.82-6.83(2H,m), 5.03(1H,d,J=2.4Hz), 4.04(1H,s), 3.95(3H,s), 3.87(3H,s)

To 10 ml of tetrahydrofuran, 0.34 g of N-{2-(3,4-dimethoxyphenyl)pyridine-3-yl}-2-hydroxy-2-(4-chlor ophenyl) acetamide and 0.24 ml of triethylamine were mixed and then further mixed with 0.10 ml of methanesulfonyl chloride at about 0 °C, followed by stirring at room temperature for 2 hours. Thereafter, the reaction mixture was added with water to be extracted with ethylacetate. The organic layer was washed by 1 % hydrochloric acid and water, and then dried by magnesium sulfate, followed by concentration under reduced pressure to obtain N-{2-(3,4-dimethoxyphenyl)pyridine-3-yl}-2-methanesulfonyloxy -2-(4-chlorophenyl)acetamide (, which is referred to as the compound of the present invention (1-3) hereinafter). This N-{2-(3,4-dimethoxyphenyl)pyridine-3-yl}-2-methanesulfonyloxy -2-(4-chlorophenyl) acetamide and 1.5 ml of 2-prapyn-1-ol were mixed, followed by stirring at about 80 °C for 3 hours. Thereafter, the reaction mixture was cooled down to room temperature and then added with water, followed by extraction with ethylacetate. The organic layer was successively washed by 3 % hydrochloric acid, aqueous saturated sodium bicarbonate and aqueous saturated sodium chloride, and then dried by magnesium sulfate, followed by concentration under reduced pressure. The residue was subjected to a silica gel column chromatography to obtain 123 mg of N-{2-(3,4-dimethoxyphenyl)-pyridine-3-yl}-2-(2-propynyloxy)-2-(4-chlorophenyl)acetamide (the compound of the present invention (I-1)}.

### Production Example 6

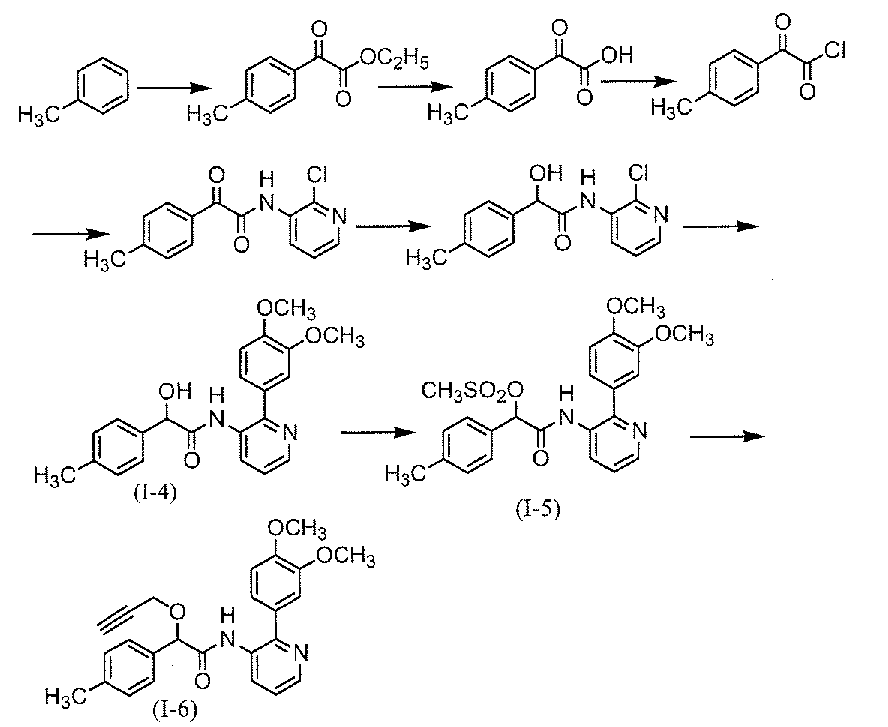

To the mixture of 8 g of aluminum chloride and 80 ml of dichloromethane, 7.85 g of ethyloxalyl chloride was added dropwise at 0 °C, and further 4.60 g of toluene being added dropwise at the same temperature, followed by stirring at room temperature for 1 hour. Thereafter, the reaction mixture was added to ice water to be extracted by chloroform. The organic layer was washed twice by aqueous saturated sodium chloride and then dried by magnesium sulfate followed by concentration under reduced pressure to obtain 9.3 g of 4-methylphenylglyoxylic acid ethyl ester as a crude product.
¹H-NMR(CDCl₃,TMS)δ(ppm): 7.91(2H,d,J=8.2Hz), 7.31(2H,d,J=8.1Hz), 4.44(2H,q,J=7.1Hz), 2.44(3H,s), 1.42(2H,t,J=7.0Hz)
9.3 g of 4-methylphenylglyoxylic acid ethyl ester, 13 ml of aqueous 30 % sodium hydroxide and 15 ml of ethanol were mixed, followed by heating with reflux for 2 hours. Thereafter, the reaction mixture was cooled down to room temperature, and then the reaction mixture was acidified by addition of 5 % hydrochloric acid to be extracted by ethylacetate. The organic layer was washed twice by aqueous saturated sodium chloride and then dried by magnesium sulfate, followed by concentration under reduced pressure. The residue was washed by hexane, followed by drying to obtain 5.2 g of 4-methylphenylglyoxylic acid.
¹H-NMR(CDCl₃, TMS)δ(ppm): 8.25(2H,d,J=8.3Hz), 7.32(2H,d,J=8.0Hz), 4.28(1H,br), 2.45(3H,s)

To 150 ml of toluene, 10.0 g of 4-methylphenylglyoxylic acid, 7.2 ml of thionyl chloride and 0.2 g of N,N-dimethylformamide were mixed, followed by stirring at 80 °C for 1 hour. Thereafter, the reaction mixture was cooled down to room temperature, and then said reaction mixture was concentrated under reduced pressure to obtain 12.1 g of 4-methylphenylglyoxyl chloride. To the solution prepared by mixing 2.1 g of 3-amino-2-chloropyridine and 2.5 ml of triethylamine to 50 ml of tetrahydrofuran, 3 g of 4-methylphenylglyoxyl chloride was mixed at the temperature range of from 0 to 5 °C, followed by stirring at room temperature for 4 hours. Then the reaction mixture was added with water to be extracted with ethylacetate. The organic layer was successively washed by 5 % hydrochloric acid, aqueous saturated sodium bicarbonate and aqueous saturated sodium chloride and then dried by magnesium sulfate, followed by concentration under reduced pressure. The residue was washed by hexane to obtain 4.0 g of N-(2-chloropyridine-3-yl)-2-oxo-2-(4-methylphenyl)acetamide.
¹H-NMR(CDCl₃,TMS) δ(ppm): 9.60(1H,s), 8.86(1H,dd,J=8.4, 1.6Hz), 8.35(2H,d,J=8.0Hz), 8.20(dd,1H,J=4.4,1.2Hz), 7.32-7.35(3H,m), 2.46(3H,s)

To 10 ml of ethanol, 1.5 g of N-(2-chloropyridine-3-yl)-2-oxo-2-(4-methylphenyl)acetamide was dissolved and then added with 62 mg of sodium borohydride at 0 °C, followed by stirring at room temperature for 3 hours. Thereafter, the reaction mixture was added with aqueous saturated ammonium chloride to be extracted by ethylacetate. The organic layer was successively washed by aqueous saturated sodium bicarbonate and aqueous saturated sodium chloride, and then dried by magnesium sulfate, followed by concentration under reduced pressure to obtain 285 mg of N-(2-chloropyridine-3-yl)-2-hydroxy-2-(4-methylphenyl)acetami de.
¹H-NMR(CDCl₃,TMS)δ(ppm): 8.93(1H,s), 8.73(1H,dd,J=8.4,1.6Hz), 8.11(dd,1H,J=4.4,1.6Hz), 7.39(2H,d,J=8.0Hz), 7.22-7.26(3H,m), 5.25(1H,d,J=2.0Hz), 3.18(1H,d, J=2.0Hz), 2.36(3H,s)

To 20 ml of ethyleneglycoldimethylether, 1.3 g of N-(2-chloropyridine-3-yl)-2-hydroxy-2-(4-methylphenyl)acetami de, 1.0 g of 3, 4-dimethoxyphenylboronic acid, 3.0 g of tripotassium phosphate hydrate and 0.12 g of {1,1' -bis(diphenylphosphino)ferrocene}dichloropalladium(II) methylenechloride complex were mixed, followed by stirring under a nitrogen atmosphere at 80 °C for 3 hours. Thereafter, the reaction mixture was cooled down to room temperature to be filtrated, followed by concentrating the filtrate under reduced pressure. The residue was subjected to a silica gel column chromatography to obtain 2.4 g of N-{2-(3,4-dimethoxyphenyl)pyridine-3-yl}-2-hydroxy-2-(4-methy lphenyl)acetamide (, which is referred to as the compound of the present invention (I-4) hereinafter).
¹H-NMR(CDCl₃,TMS)δ(ppm): 8.73(1H,d,J=8.0Hz), 8.40-8.41(1H,m), 8.37(1H,s), 7.15-8.24(4H,m), 7.01(1H,d,J=1.2Hz), 6.88-6.90(1H,m), 6.84(1H,d,J=8.4Hz), 5.08(1H,d,J=2.8Hz), 3.96(3H,s), 3.88(3H,s), 3.32(1H,d,J=2.8Hz), 2.37(3H,s)

To 10 ml of tetrahydrofuran, 0.63 g of N-{2-(3,4-dimethoxyphenyl)pyridine-3-yl}-2-hydroxy-2-(4-methy lphenyl) acetamide and 0.3 ml of triethylamine were dissolved and then added with 0.14 ml of methanesulfonyl chloride at about 0 °C, followed by stirring at room temperature for 30 minutes. Thereafter, the reaction mixture was added with water to be extracted by ethylacetate. The organic layer was washed by water and then dried by anhydrous magnesium sulfate, followed by concentration under reduced pressure to obtain N-{2-(3,4-dimethoxyphenyl)pyridine-3-yl}-2-methanesulfonyloxy -2-(4-methylphenyl)acetamide (, which is referred to as the compound of the present invention (1-5) hereinafter). This N-{2-(3,4-dimethoxyphenyl)pyridine-3-yl}-2-methanesulfonyloxy -2-(4-methylphenyl)acetamide and 2.5 ml of 2-propyn-1-ol were mixed, followed by stirring at around 80 °C for 1 hour. Thereafter, the reaction mixture was cooled down to room temperature and then added with water to be extracted by ethylacetate. The organic layer was successively washed by water, aqueous saturated ammonium chloride and aqueous saturated sodium chloride, and then dried by magnesium sulfate, followed by concentration under reduced pressure. The residue was subjected to a silica gel column chromatography to obtain 397 mg of N-{2-(3,4-dimethoxyphenyl)pyridine-3-yl}-2-propynyloxy-2-(4-m ethylphenyl)acetamide (, which is referred to as the compound of the present invention (I-6) hereinafter).
¹H-NMR(CDCl₃,TMS)δ(ppm): 9.00(1H,s), 8.73(1H,dd,J=8.0Hz,1.6Hz), 8.42(1H,dd,J=4.4Hz,1.6Hz), 7.00-7.31(8H,m),5.07(1H,s),3.91-4.17(8H,m),2.47(1H,t,J=2.0Hz), 2.34(3H,s)

### Production Example 7

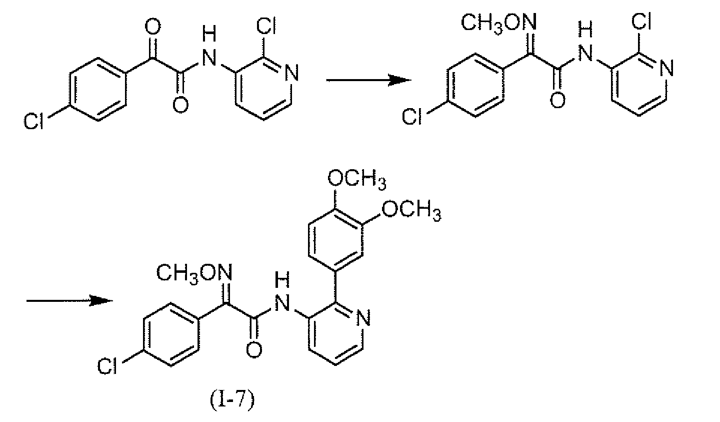

The mixture of 2.00 g of N-(2-chloropyridine-3-yl)-2-oxo-2-(4-chlorophenyl)acetamide, 20 ml of ethanol, 800 mg of pyridine and 1.13 g of methoxyamine hydrochloric acid salt were stirred at room temperature for 2 hours. Thereafter, the reaction mixture was concentrated under reduced pressure. The residue obtained was added with water to be extracted by ethylacetate; the extract was washed twice by 5 % hydrochloric acid and aqueous saturated sodium chloride followed by drying with magnesium sulfate and then the solvent being distilled off under reduced pressure; and the residue was washed by n-hexane followed by filtration and drying to obtain 1.95 g of N-(2-chloropyridine-3-yl)-2-methoxyimino-2-(4-chlorophenyl)ac etamide.
¹H-NMR(CDCl₃,TMS)δ(ppm): 8.8-8.9(2H,m), 8.19(1H,dd,J=1.7Hz,4.6Hz), 7.60-7.70(2H,m), 7.30-7.40(2H,m), 4.14(3H,s)

To 10 ml of ethyleneglycoldimethylether, 0.5 g of N-(2-chloropyridine-3-yl)-2-methoxyimino-2-(4-chlorophenyl)acetamide, 0.31 g of 3,4-dimethoxyphenylboronic acid, 0.65 g of tripotassium phosphate hydrate and 25 mg of {1,1' -bis(diphenylphosphino)ferrocene}dichloropalladium(II) methylenechloride complex were mixed, followed by stirring under a nitrogen atmosphere at 80 °C for 2 hours. Thereafter, the reaction mixture was cooled down to room temperature to be filtrated, followed by concentration of the filtrate under reduced pressure. The concentrated residue was subjected to a silica gel column chromatography to obtain 0.55 g of N-{2-(3,4-dimethoxyphenyl)pyridine-3-yl}-2-methoxyimino-2-(4-chlorophenyl)acetamide (, which is referred to as the compound of the present invention (I-7) hereinafter).
¹H-NMR(CDCl₃,TMS)δ(ppm): 8.81(1H,dd,J=8.7Hz,2.0Hz), 8.47(1H,dd,J=4.9Hz,1.6Hz), 8.34(1H,s), 6.92-7.60(m,8H), 3.93(6H,s), 3.87(3H,s)

### Production Example 8

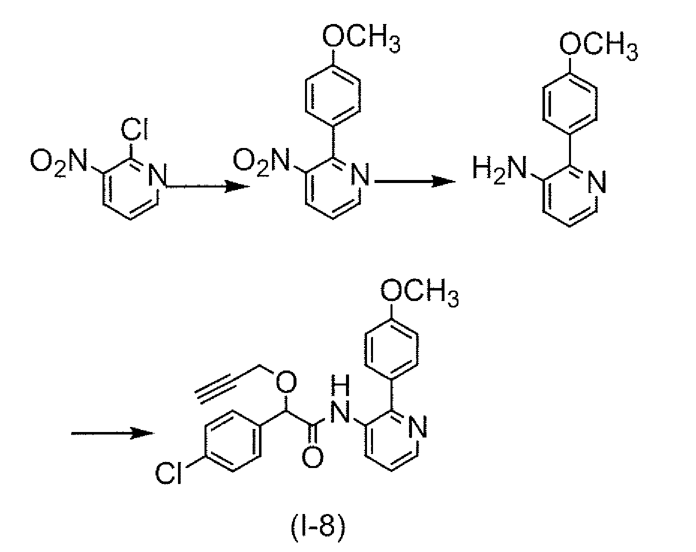

To 50 ml of ethyleneglycoldimethylether, 0.92 g of 2-chloro-3-nitropyridine, 0.80 g of 4-methoxyphenylboronic acid, 3.4 g of tripotassium phosphate hydrate, 130 mg of {1,1' -bis(diphenylphosphino)ferrocene}dichloropalladium(II) methylenechloride complex and 1.5 g of celite were added, followed by stirring under a nitrogen atmosphere at 80 °C for 1.5 hours. Thereafter, the reaction mixture was cooled down to room temperature to be filtrated, followed by concentration of the filtrate under reduced pressure. The residue was subj ected to a silica gel column chromatography to obtain 0.76 g of 2-(4-methoxyphenyl)-3-nitropyridine.
¹H-NMR(CDCl₃,TMS)δ(ppm): 3.86(3H,s), 6.98(2H,d,J=8.7Hz), 7.37(1H,dd,J=4.7Hz,8.0Hz), 7.53(2H,d,J=8.7Hz), 8.08(1H,dd,J=1.4Hz,8.0Hz), 8.81(1H,dd,J=1.4Hz,4.7Hz) 0.76 g of 4-(4-methoxyphenyl)-3-nitropyridine was dissolved to 100 ml of ethanol at room temperature and then added with 50 mg of 5 % platinum-carbon, followed by stirring under a hydrogen atmosphere for 4 hours. Thereafter, the reaction mixture was filtrated thruogh celite, followed by concentration of the filtrate under reduced pressure. The residue was subjected to a silica gel column chromatography (eluent; hexane/ethylacetate =1/1) to obtain 0.45 g of 3-amino-2-(4-methoxyphenyl)pyridine.
¹H-NMR(CDCl₃,TMS)δ(ppm): 3.81(2H,br), 3.85(3H,s), 6.97-7.04(4H,m), 7.61(2H,d,J=8.7Hz), 8.10(1H,t,J=2.9Hz)
0.36 g of 2-(4-chlorophenyl)-2-(2-propynyloxy)acetyl chroride was added dropwise to the mixture of 0.25 g of 3-amino-2-(4-methoxyphenyl) pyridine, 0.19 g of triethylamine and 20 ml of tetrahydrofuran, followed by stirring at room temperature for 2 hours. Thereafter, the reaction mixture was added with ethylacetate and then filtrateed to remove insolubles. After the filtrate was concentrated under reduced pressure, the residue was subjected to a silica gel column chromatography (eluent; hexane/ethylacetate =1/1) to obtain 0.55 g of N-(2-(4-methoxyphenyl)pyridine-3-yl)-2-(2-propynyloxy)-2-(4-c hlorophenyl) acetamide (, which is referred to as the compound of the present invention (I-8) hereinafter).
¹H-NMR(CDCl₃,TMS)δ(ppm): 2.49(1H,t,J=1.6Hz), 3.89(3H,s), 3.99(1H,dd,J=2.4Hz,15.8Hz), 4.18(1H,dd,J=2.4Hz,15.8Hz), 5.07(1H,s), 7.06(2H,d,J=8.7Hz), 7.21-7.26(1H,m), 7.30-7.37(4H,m), 7.54(2H,d,J=8.7Hz), 8.42(1H,dd,J=1.6Hz,4.7Hz), 8.64(1H,dd,J=1.6Hz,8.3Hz), 8.52(1H,s)

### Production Example 9

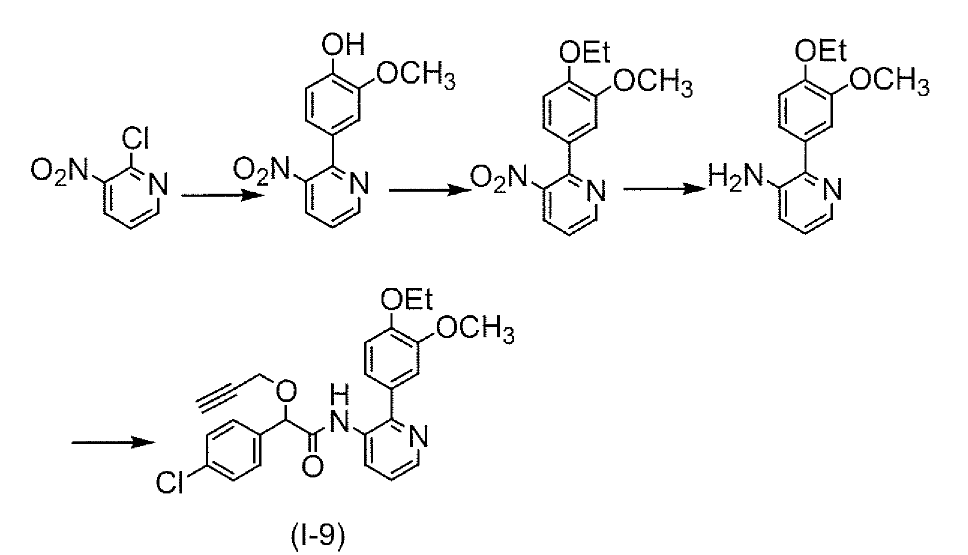

To 100 ml of ethyleneglycoldimethylether, 6.0 g of 2-chloro-3-nitropyridine, 7.9 g of 2-methoxy-4-(4,4,5,5,-tetramethyl-1,3,2-dioxaborane-2-yl)phen ol, 19.8 g of tripotassium phosphate hydrate and 780 mg of {1,1' -bis(diphenylphosphino)ferrocene}dichloropalladium(II) methylenechloride complex were added, followed by stirring under a nitrogen atmosphere at 80°C for 3 hours. Thereafter, the reaction mixture was cooled down to room temperature to be filtrated, followed by concentration of the filtrate under reduced pressure. The residue was subjected to a silica gel column chromatography to obtain 6.38 g of 2-(4-hydroxy-3-methoxyphenyl)-3-nitropyridine.
¹H-NMR(CDCl₃, TMS)δ(ppm): 3.93(3H,s), 6.14(1H,s), 6.96(1H,d,J=7.9Hz), 7.05(1H,dd,J=2.0Hz,7.9Hz), 7.17(1H,d,J=2.0Hz), 7.37(1H,dd,J=4.7Hz,7.9Hz), 8.05(1H,dd,J=1.6Hz,7.9Hz), 8.80(1H,dd,J=1.6Hz,4.7Hz)

To 50 ml of N,N-dimethylformamide, 1.50 g of 2-(4-hydroxy-3-methoxyphenyl)-3-nitropyridine, 1.0 g of ethyl iodide and 0.90 g of potassium carbonate were added, followed by stirring at room temperature for 4 hours. The reaction solution was added with water to be extracted by ethylacetate. The organic layer obtained was concentrated under reduced pressure, followed by subjecting the residue obtained to a silica gel column chromatography to obtain 0.90 g of 2-(4-ethoxy-3-methoxyphenyl)-3-nitropyridine.
¹H-NMR(CDCl₃,TMS)δ(ppm): 1.49(3H,t,J=7.1Hz), 3.92(3H,s), 4.15(2H,q,J=7.1Hz), 6.91(1H,d,J=8.3Hz), 7.09(1H,dd,J=2.0Hz,8.3Hz), 7.18(1H,d,J=2.0Hz), 7.37(1H,dd,J=4.7Hz,7.9Hz), 8.05(1,9,dd,J=1.6Hz,7.9Hz), 8.81(1H,dd,J=1.6Hz,4.7Hz)

To 100 ml of ethanol, 0.90 g of 2-(4-ethoxy-3-methoxyphenyl)-3-nitropyridine and 50 mg of 5 % platinum-carbon were added, followed by stirring under a hydrogen atmosphere for 4 hours. Thereafter, the reaction mixture was filtrated through celite, followed by concentration of the filtrate under reduced pressure. The residue was subjected to a silica gel column chromatography (eluent; hexane/ethylacetate=1/1) to obtain 0.72 g of 3-amino-2-(4-ethoxy-3-methoxyphenyl)pyridine.
¹H-NMR(CDCl₃,TMS)δ(ppm): 1.48(3H,t,J=6.8Hz), 3.88(2H,s), 3.91(3H,s), 4.14(2H,q,J=6.8Hz), 6.94(1H,d,J=8.8Hz), 7.03(2H,d,J=2.8Hz), 7.19-7.25(2H,m), 8.10(1H,t,J=2.8Hz)

The mixture of 0.20 g of 3-amino-2-(4-ethoxy-3-methoxyphenyl)pyridine, 0.15 g of triethylamine and 10 ml of tetrahydrofuran was added dropwise with 0.30 g of 2-(4-chlorophenyl)-2-(2-propynyloxy)acetyl chloride, followed by stirring at room temperature for 2 hours. Thereafter, the reaction mixture was added with ethylacetate and then filtrated to remove insolubles. After the filtrate being concentrated under reduced pressure, the residue was subjected to a silica gel column chromatography (eluent; hexane/ethylacetate =1/1) to obtain 0.30 g of N-(2-(4-ethoxy-3-methoxyphenyl)pyridine-3-yl)-2-(2-propynylox y)-2-(4-chlorophenyl)acetamide (, which is referred to as the compound of the present invention (I-9) hereinafter).
¹H-NMR(CDCl₃,TMS)δ(ppm): 1.52(3H,t,J=7.1Hz), 2.49(1H,t,J=2.4Hz), 3.94(3H,s), 3.97(1H,dd,J=2.4Hz,15.8Hz), 4.18(1H,dd,J=2.4Hz,15.8Hz), 4.19(2H,q,J=7.1Hz), 5.08(1H,s), 7.01(1H,d,J=7.9Hz), 7.11-7.15(2H,m), 7.24(1H,dd,J=4.7Hz,7.9Hz), 7.31(2H,d,J=8.7Hz), 7.35(2H,d,J=8.7Hz), 8.42(1H,dd,J=1.6Hz,4.7Hz), 8.68(1H,dd,J=1.6Hz,7.9Hz), 8.96(1H,s)

### Production Example 10

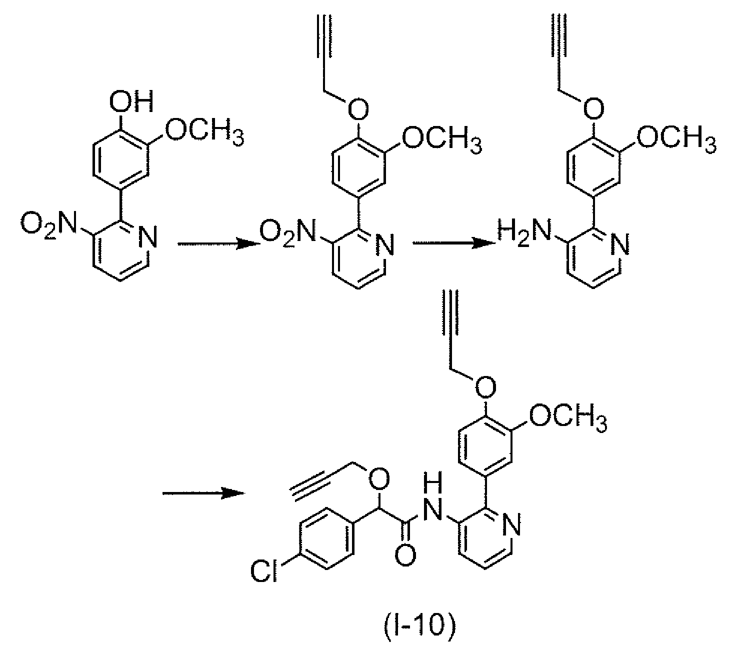

To 100 ml of N,N-dimethylformamide, 3.0 g of 2-(4-hydroxy-3-methoxyphenyl)-3-nitropyridine, 1.5 g of 3-bromo-1-propyne and 1.7 g of potassium carbonate were added, followed by stirring at room temperature for 4 hours. The reaction solution was added with dilute hydrochloric acid to be extracted by ethylacetate. The organic layer was concentrated under reduced pressure, followed by subjecting the residue obtained to a silica gel column chromatography to obtain 1.8 g of 2-(3-methoxy-4-(2-propynyloxy)phenyl)-3-nitropyridine.
¹H-NMR(CDCl₃,TMS)δ(ppm): 2.54(1H,t,J=2.4Hz), 3.92(3H,s), 4.81(2H,d,J=2.4Hz), 7.07-7.13(2H,m), 7.20(1H,d,J=1.6Hz), 7.39(1H,dd,J=4.7Hz,8.3Hz), 8.07(1H,dd,J=1.6Hz,8.3Hz), 8.82(1H,dd,J=1.6Hz,4.7Hz)

The mixture of 5 ml of acetic acid, 30 ml of water and 1.3 g of iron powder was heated to 75 °C and then added dropwise with 1.6 g of solution of 2-(3-methoxy-4-(2-propynyloxy)phenyl)-3-nitropyridine dissolved in 10 ml of ethylacetate. After being heated with reflux for 3 hours, the reaction solution was added with aqueous sodium bicarbonate to be extracted by ethylacetate. The organic layer was dried by magnesium sulfate followed by concentration under reduced pressure; and then the residue was subjected to a silica gel column chromatography to obtain 1.4 g of 3-amino-2-(3-methoxy-4-(2-propynyloxy)phenyl)pyridine.
¹H-NMR(CDCl₃,TMS)δ(ppm): 2.52(1H,t,J=2.4Hz), 3.89(2H,s), 3.91(3H,s), 4.80(2H,d,J=2.4Hz), 7.02-7.05(2H,m), 7.10(1H,d,J=8.8Hz), 7.22-7.26(2H,m), 8.10(1H,ddd,J=0.4Hz,2.4Hz,3.6Hz)
0.25 g of 2-(4-chlorophenyl)-2-(2-propynyloxy)acetyl chloride was added dropwise to the mixture of 0.20 g of 3-amino-2-(3-methoxy-4-(2-propynyloxy)phenyl)pyridine, 0.15 g of triethylamine and 10 ml of tetrahydrofuran, followed by stirring at room temperature for 2 hours. Thereafter, the reaction mixture was added with ethylacetate and then filtrated to remove insolubles. After the filtrate was concentrated under reduced pressure, the concentrated residue was subjected to a silica gel column chromatography (eluent; hexane/ethylacetate =1/1) to obtain 0.30 g of N-(2-(3-methoxy-4-(2-propynyloxy)phenyl)pyridine-3-yl)-2-(2-p ropynyloxy)-2-(4-chlorophenyl)acetamide (, which is referred to as the compound of the present invention (1-10) hereinafter).
¹H-NMR(CDCl₃,TMS)δ(ppm): 2.51(1H,t,J=2.4Hz), 2.55(1H,t,J=2.4Hz), 3.94(3H,s), 3.97(1H,dd,J=2.4Hz,16.2Hz), 4.18(1H,dd,J=2.4Hz,16.2Hz), 4.86(2H,d,J=2.4Hz), 5.08(1H,s), 7.12-7.20(3H,m), 7.25(1H,dd,J=4.7Hz,7.9Hz), 7.31(2H,d,J=8.3Hz), 7.35(2H,d,J=8.3Hz), 8.42(1H,dd,J=1.6Hz,4.7Hz), 8.68(1H,dd,J=1.6Hz,7.9Hz), 8.95(1H,s)

### Production Example 11

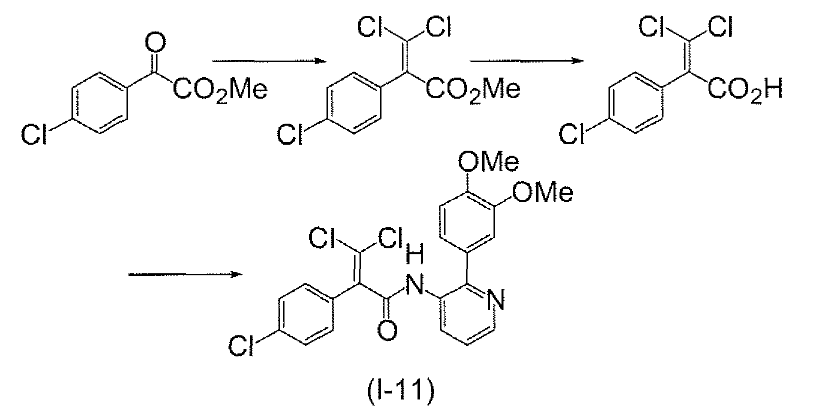

10 g of 4-chlorophenylglyoxylic acid methyl ester was dissolved to 100 ml of acetonitrile and then added with 40 g of triphenylphosphine and 23 g of carbon tetrachloride, followed by stirring at room temperature for 4 hours. The reaction solution was concentrated to subject the residue to a silica gel column chromatography (eluent; hexane/ethylacetate =10/1) to obtain 13.7 g of 2-(chlorophenyl)-3,3-dichloroacrylic acid methyl ester.
¹ H-NMR(CDCl₃,TMS)δ(ppm): 3.79(3H,s), 7.30(2H,d,J=8.3Hz), 7.37(2H,d,J=8.3Hz)
13.5 g of 2-(chlorophenyl)-3,3-dichloroacrylic acid methyl ester was dissolved to 130 ml of methanol and then added dropwise with aqueous potassium hydroxide (4.0 g of potassium hydroxide, 70 ml of water). After being stirred at room temperature for 8 hours, the reaction solution was semi-concentrated, followed by addition of dilute hydrochloric acid to be extracted by ethylacetate. The organic layer was dried by magnesium sulfate, followed by concentration under reduced pressure. The crystal obtained was washed by hexane to obtain 9.0 g of 2-(chlorophenyl)-3,3-dichloroacrylic acid.
¹H-NMR(CDCl₃,TMS)δ(ppm): 7.28(2H,dd,J=1.6Hz,8.3Hz), 7.38(2H,dd,J=1.6Hz,8.3Hz), 10.25(1H,br)
0.63 g of 2-(chlorophenyl)-3,3-dichloroacrylic acid and 0.36 g of thionyl chloride were added to 10 ml of toluene, followed by being heated under stirring for 2 hours. The reaction solution was concentrated under reduced pressure to obtain a crude 2-(chlorophenyl)-3,3-dichloroacryl chloride. The crude 2-(chlorophenyl)-3,3-dichloroacryl chloride obtained was added dropwise to 10 ml of tetrahydrofuran solution containing of 0.50 g of triethylamine and 0.30 g of 3-amino-2-(3,4-dimethoxyphenyl)pyridine, followed by stirring at room temperature for 3 hours; and then the reaction solution was added with ethylacetate and then filtrated to remove insolubles. The filtrate was concentrate under reduced pressure, followed by subjecting the residue to a silica gel column chromatography to obtain 60 mg of N-(2-(3,4-dimethoxyphenyl)-pyridine-3-yl)-2-(chlorophenyl)-3, 3-dichloroacrylamide.
¹H-NMR(CDCl₃,TMS)δ(ppm): 3.83(3H,s), 3.95(3H,s), 6.65-6.70(2H,m), 6.93(1H,s), 7.23-7.31(3H,m), 7.37(2H,d,J=7.6Hz), 7.49(1H,s), 8.41(1H,d,J=4.8Hz), 8.72(1H,d,J=8.4Hz)

### Production Example 12

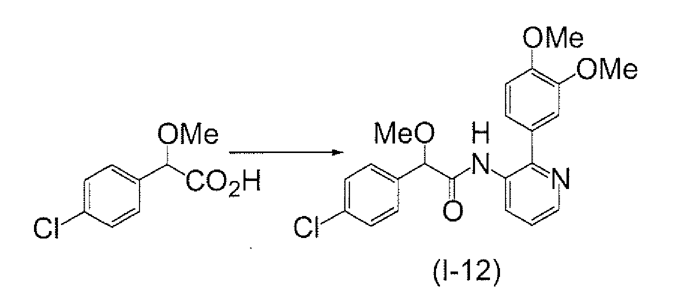

To 10 ml of toluene, 0.19 g of -2-(4-chlorophenyl)-2-methoxyacetic acid and 0.14 g of thionyl chloride were added, followed by being heated under stirring for 2 hours. The reaction solution was concentrated under reduced pressure to obtain a crude 2-(chlorophenyl)-2-methoxyacetyl chloride. The crude 2-(chlorophenyl)-2-methoxyacetyl chloride obtained was added dropwise at 0 °C to 10 ml of tetrahydrofuran solution containing 0.11 g of triethylamine and 0.22 g of 3-amino-2-(3,4-dimethoxyphenyl)pyridine. After being stirred at room temperature for 3 hours, the reaction solution was concentrated under reduced pressure, followed by subjecting the residue to a silica gel column chromatography to obtain 127 mg of N-(2-(3,4-dimethoxyphenyl)-pyridine-3-yl)-2-(4-chlorophenyl)-2-methoxyacetamide.
¹H-NMR(CDCl₃,TMS)δ(ppm): 3.31(3H,s), 3.95(3H,s), 3.98(3H,s), 4.67(1H,s), 7.00(1H,d,J=8.0Hz), 7.10-7.16(2H,m), 7.24(1H,dd,J=4.8Hz,8.4Hz), 7.30(2H,d,J=8.4Hz), 7.35(2H,d,J=8.4Hz), 8.42(1H,dd,J=1.6Hz,4.8Hz), 8.67(1H,dd,J=1.6Hz,8.4Hz), 8.95(1H,s)

Next, the illustrated below is a referential production example producing a production intermediate for the compound of the present invention.

### Referential Production Example 1

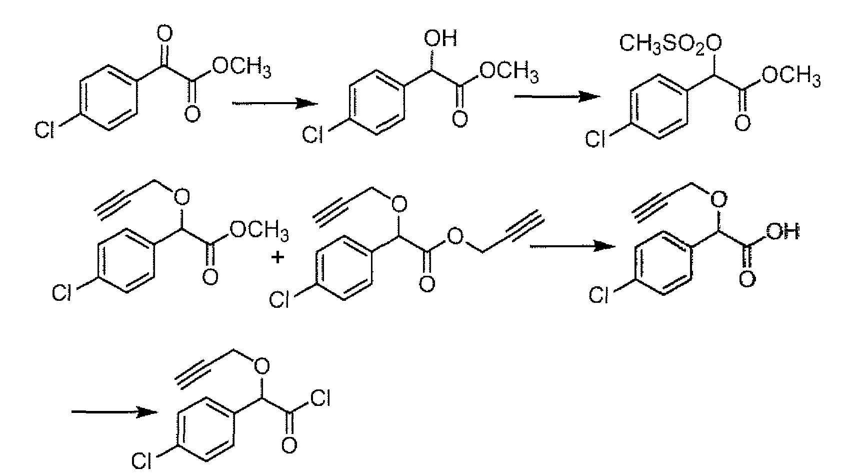

80.2 g of 4-chlorophenylglyoxylic acid methyl ester was dissolved to 500 ml of methanol and then added with 3.8 g of sodium borohydride at the temperature range of from 0 to 5 °C, followed by stirring at room temperature for 2 hours. Thereafter, the reaction mixture was concentrated under reduced pressure, followed by addition of water to the residue to be extracted by ethylacetate. The organic layer was concentrated under reduced pressure, followed by subjecting the concentrated residue to a silica gel column chromatography (eluent; hexane/ethylacetate = 5/1 to 2/1) to obtain 62.4 g of 2-hydroxy-2-(4-chlorophenyl)acetic acid methyl ester.
¹H-NMR(CDCl₃,TMS)δ(ppm): 3.51(1H,d,J=5.3Hz), 3.76(3H,S), 5.15(1H,d,J=5.3Hz), 7.31-7.38(4H,m)

To 200 ml of tetrahydrofuran, 62.4 g of 2-hydroxy-2-(4-chlorophenyl)acetic acid methyl ester and 40.8 g of triethylamine were dissolved and then added with 42.8 g of methanesulfonyl chloride at 0 °C, followed by stirring at room temperature for 3 hours. Thereafter, the reaction mixture was concentrated under reduced pressure, followed by addition of ethylacetate to the residue and then filtrated to remove solid material. After the filtrate being concentrated under reduced pressure, the residue was subjected to a silica gel column chromatography (eluent; hexane/ethylacetate =3/1) to obtain 75.6 g of 2-methanesulfonyloxy-2-(4-chlorophenyl)acetic acid methyl ester.
¹H-NMR(CDCl₃,TMS)δ(ppm): 3.12 (3H,s), 3.78 (3H,s), 5.92(1H,s), 7.36-7.43(4H,m)
70 g of 2-methanesulfonyloxy-2-(4-chlorophenyl) acetic acid methyl ester was mixed with 70 g of 2-propynylalcohol, followed by stirring at 80°C for 1.5 hours. Thereafter, the reaction mixture was added with toluene followed by concentration under reduced pressure. The concentrated residue was subjected to a silica gel column chromatography (eluent; hexane/ethylacetate =10/1) to obtain 64.6 g of a mixture of 2-(2-propynyloxy)-2-(4-chlorophenyl)acetic acid methyl ester and 2-(2-propynyloxy)-2-(4-chlorophenyl)acetic acid (2-propynyl). (2-propynyloxy)-2-(4-chlorophenyl)acetic acid (2-propynyl)
¹H-NMR(CDCl₃,TMS)δ(ppm): 2.47(1H,t,J=2.4Hz), 2.51(1H,t,J=2.4Hz), 4.18(2H,dd,J=2.4Hz,16.2Hz), 4.31(2H,dd,J=2.4Hz,16.2Hz), 4.67(2H,dd,J=2.4Hz,15.4Hz), 4.76(2H,dd,J=2.4Hz,15.4Hz), 5.23(1H,s), 7.33-7.42(4H,m) 2-(2-propynyloxy)-2-(4-chlorophenyl)acetic acid methyl ester
¹H-NMR(CDCl₃,TMS)δ(ppm): 2.50(1H,t,J=2.4Hz), 3.12(3H,s), 4.16(2H,dd,J=2.4Hz,16.2Hz), 4.30(2H,dd,J=2.4Hz,16.2Hz), 5.19(1H,s), 7.33-7.42(4H,m)
64.6 g of the mixture of (2-(2-propynyloxy)-2-(4-chlorophenyl)acetic acid methyl ester and 2-(2-propynyloxy)-2-(4-chlorophenyl)acetic acid (2-propynyl), which mentioned above, were dissolved to 800 ml of tetrahydrofuran and then added dropwise at 0 °C with aqueous lithium hydroxide (a mixture of 6.74 g of lithium hydroxide and 280 ml of water). After being stirred in the range of from 0 °C to room temperature for 3 hours, the reaction solution was added with dilute hydrochloric acid to be extracted by ethylacetate. The organic layer was dried by anhydrous magnesium sulfate, followed by concentration under reduced pressure. The crystal produced was washed by hexane to obtain 43.4 g of 2-(2-propynyloxy)-2-(4-chlorophenyl) acetic acid (Intermediate 5-003).
¹H-NMR(CDCl₃,TMS)δ(ppm): 2.51(1H,t,J=2.4Hz), 4.15(1H,dd,J=2.4Hz,16.0Hz), 4.32(1H,dd,J=2.4Hz,16.0Hz), 7.33-7.44(4H,m), 8.70-9.50(1H,br)
4.7 g of 2-(2-propynyloxy)-2-(4-chlorophenyl)acetic acid and 3.0 g of thionyl chloride were dissolved to 50 ml of toluene, followed by stirring at 100 °C for 2 hours. The reaction solution was concentrated under reduced pressure to obtain 5.0 g of 2-(2-propynyloxy)-2-(4-chlorophenyl)acetic acid chloride.
¹H-NMR(CDCl₃,TMS)δ(ppm): 2.57(1H,t,J=2.0Hz), 4.28(1H,dd,J=2.0Hz,16.2Hz), 4.36(1H,dd,J=2.0Hz,16.2Hz), 5.39(1H,s), 7.37-7.44(4H,m)

Next formulation examples are shown. Parts represent parts by weight.

### Formulation Example 1

Fifty parts of each of the compounds of the present invention (I-1) to (I-12), (II-1), (III-1) and (IV-1), 3 parts of calcium ligninsulfonate, 2 parts of magnesium laurylsulfate and 45 parts of synthetic hydrated silica are pulverized and mixed well to give wettable powders of each compound.

### Formulation Example 2

Twentyparts of each of the compounds of the present invention (I-1) to (I-12), (II-1), (III-1) and (IV-1) and 1.5 parts of sorbitan trioleate aremixedwith 28.5 parts of an aqueous solution containing 2 parts of polyvinyl alcohol, and wet-pulverized finely. To the obtained mixture, 40 parts of an aqueous solution containing 0.05 part of xanthan gum and 0.1 part of aluminium magnesium silicate is added and further 10 parts of propylene glycol are added to give a flowable of each compound.

### Formulation Example 3

Two parts of each of the compounds of the present invention (I-1) to (I-12), (II-1), (III-1) and (IV-1), 88 parts of kaolin clay and 10 parts of talc are pulverized and mixed well to give dusts of each compound.

### Formulation Example 4

Five parts of each of the compounds of the present invention (I-1) to (I-12), (II-1), (III-1) and (IV-1), 14 parts of polyoxyethylenestyryl phenyl ether, 6 parts of calcium dodecylbenzenesulfonate and 75 parts of xylene are mixed well to give emulsifiable concentrates of each compound.

### Formulation Example 5

Two parts of each of the compounds of the present invention (I-1) to (I-12), (II-1), (III-1) and (IV-1), 1 part of synthetic hydrated silica, 2 parts of calcium ligninsulfonate, 30 parts of bentonite and 65 parts of kaolin clay are pulverized and mixed well, and water is added thereto and kneeded, granulated and dried to give granules of each compound.

### Formulation Example 6

Ten parts of each of the compounds of the present invention (I-1) to (1-12), (II-1), (III-1) and (IV-1), 35 parts of white carbon containing 50% by weight of ammonium polyoxyethylenealkyl ether sulfate and 55 parts of water are mixed and wet pulverized finely to give a flowable of each compound.

Next, it is shown that the compound of the present invention are useful for controlling plant diseases.

Additionally, the control effect was evaluated by visually observing the area of a lesion on a sample plant in investigation and comparing the area of a lesion on a non-treatment plant and the area of a lesion on a treated plant with the compound of the present invention.

### Test Example 1

Sand loam was compacted in a plasticpot, andatomato (variety: Ponterosa) was seeded and grown in a green house for 20 days. The compounds of the present invention (I-1), (I-2), (I-4), (1-6) to (I-10), (I-12), (II-1), (III-1) and (IV-1) were formulated to flowable according to Formulation Example 6, then, diluted with water to provide given concentration of 500ppm, and these diluted solutions were sprayed onto stems and leaves so as to give sufficient adhesion on the surface of the tomato leaves. After spraying, the liquid on the stem was air-dried, and a suspension of zoosporangiua of Phytophthora infestans (about 10,000 zoosporangiua were contained in 1ml of the suspension) was inoculated by spraying. The amount of the sprayed suspension was about 2 ml for one plent. After inoculation, the plant was first grown for one day at 23°C under 90 % or more humidity, then further grown for 4 days in the green house, which was 24°C in daytime and 20°C in night-time.

Then, the areas of a lesion of the plants were obserbed. The lesion areas on the plants treated with the compounds of the present invention (I-1), (I-2), (I-4), (1-6) to (I-10), (I-12), (II-1), (III-1) and (IV-1) were not more than 10% of the lesion area on a non-treatment plant.

### Test Example 2

Sand loam was compacted in a plasticpot, andatomato (variety: Ponterosa) was seeded and grown in a green house for 20 days. The compounds of the present invention (I-11) was formulatedto flowable according to Formulation Example 6, then, diluted with water to provide given concentration of 200ppm, and this diluted solution was sprayed onto stems and leaves so as to give sufficient adhesion on the surface of the tomato leaves. After spraying, the liquid on the stem was air-dried, and a suspension of zoosporangiua of Phytophthora infestans (about 10,000 zoosporangiua were contained in 1ml of the suspension) was inoculated by spraying. The amount of the sprayed suspension was about 2 ml for one plent. After inoculation, the plant was first grown for one day at 23°C under 90 % or more humidity, then further grown for 4 days in the green house, which was 24°C in daytime and 20°C in night-time.

Then, the areas of a lesion of the plants were obserbed. The lesion areas on the plants treated with the compounds of the present invention (I-11) was not more than 10% of the lesion area on a non-treatment plant.

### Test Example 3

Sand loam was compacted in a plastic pot, and a grape (variety: Berry A) was seeded and grown in a green house for 40 days. The compounds of the present invention (I-1), (I-2), (I-6) to (I-12), (II-1), (III-1) and (IV-1) were formulated to flowable according to Formulation Example 6, then, diluted with water to provide given concentration of 200ppm, and these diluted solutions were sprayed onto stems and leaves so as to give sufficient adhesion on the surface of grape leaves. After spraying, the liquid on the stem was air-dried, and a suspension of zoosporangiua of Plasmopara viticola (about 10,000 zoosporangiua were contained in 1ml of the suspension) was inoculated by spraying. The amount of the sprayed suspension was about 2 ml for one plant. After inoculation, the plant was first grown for one day at 23°C under 90 % or more humidity, then further grown for 6 days in the green house, which was 24°C in daytime and 20°C in night-time.

Then, the areas of a lesion of the plants were obserbed. The lesion areas on the plants treated with the present compound (I-1), (I-2), (I-6) to (I-12), (II-1), (III-1) and (IV-1) were not more than 10% of the lesion area on a non-treatment plant.

### Test Example 4

Sand loam was compacted in a plasticpot, and a grape (variety: Berry A) was seeded and grown in a green house for 40 days. The compounds of the present invention (I-4) was formulated to flowable according to Formulation Example 6, then, diluted with water to provide given concentration of 200ppm, and this diluted solution was sprayed onto stems and leaves so as to give sufficient adhesion on the surface of grape leaves. After spraying, the liquid on the stem was air-dried, and a suspension of zoosporangiua of Plasmopara viticola (about 10,000 zoosporangiua were contained in 1ml of the suspension) was inoculated by spraying. The amount of the sprayed suspension was about 2 ml for one plant. After inoculation, the plant was first grown for one day at 23°C under 90 % or more humidity, then further grown for 6 days in the green house, which was 24°C in daytime and 20°C in night-time.

Then, the areas of a lesion of the plants were obserbed. The lesion areas on the plants treated with the present compound (I-4) was not more than 10% of the lesion area on a non-treatment plant.

### INDUSTRIAL APPLICABILITY

The compound of the present invention has an excellent controlling activity against plant diseases and is useful as an active ingredient for the agent controlling plant diseases.

## Claims

1. A phenylpyridine compound represented by the formula (1) : 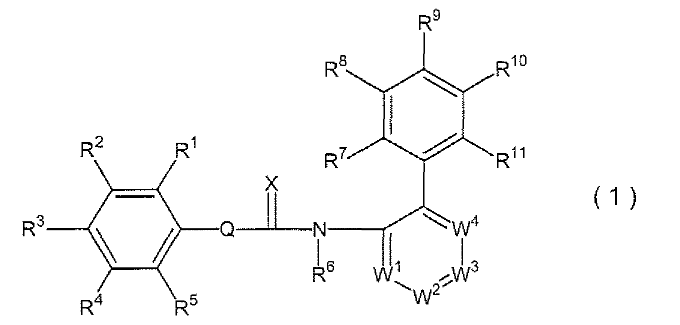 [, wherein, in the formula, R¹, R², R³, R⁴ and R⁵ independently represent a hydrogen atom, a halogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C1-C6 alkylthio group, a C1-C6 haloalkylthio group, a C3-C6 cycloalkyl group, a C3-C6 cycloalkoxy group or a cyano group; both of R² and R³ may be combined to represent a trimethylene, a tetramethylene or -CH=CH-CH=CH-;
R⁶ represents a hydrogen atom or a C1-C3 alkyl group;
R⁷, R⁸ and R¹¹ independently represent a hydrogen atom, a halogen atom or a C1-C3 alkyl group;
R⁹ and R¹⁰ independently represent a hydroxyl group, a halogen atom, a C1-C6 alkyl group, a C1-C6 haloalkyl group, a C2-C6 alkenyl group, a C2-C6 haloalkenyl group, a C2-C6 alkynyl group, a C2-C6 haloalkynyl group, a C2-C6 cyanoalkyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group, a C1-C6 alkylthio group, a C1-C6 haloalkylthio group, a C3-C6 cycloalkyl group, a C3-C6 cycloalkoxy group, a nitro group, a benzyl group or a cyano group;
W¹―W²=W³―W⁴ represents N―CR²¹=CR²²―CR²³, CR²⁴―N=CR²⁵―CR²⁶, CR²⁷ ―CR²⁸=N―CR²⁹ or CR³⁰―CR³¹=CR³²―N
{in which R²¹, R²², R²³, R²⁴, R²⁵, R²⁶, R²⁷, R²⁸, R²⁹, R³⁰, R³¹ and R³² independently represent a hydrogen atom, a halogen atom, a C1-C3 alkyl group, a C1-C3 alkoxy group or a C1-C3 haloalkyl group};
X represents an oxygen atom or a sulfur atom;
Q represents a group illustrated by the following formulas of Q1 or Q2 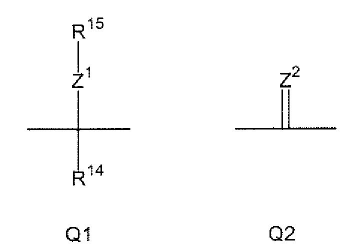 {in which R¹⁴ represents a hydrogen atom or a C1-C3 alkyl group, R¹⁵ represents a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group, a C3-C6 cycloalkyl group, a (C1-C6 alkyl) carbonyl group, a (C1-C6 haloalkyl) carbonyl group, a (C1-C6 alkoxy)carbonyl group, a (C1-C6 haloalkoxy)carbonyl group, a (C3-C6 alkenyloxy) carbonyl group, a (C3-C6 haloalkenyloxy) carbonyl group, a (C3-C6 alkynyloxy)carbonyl group, a (C3-C6 haloalkynyloxy) carbonyl group or a C1-C3 alkylsulfonyl group,
Z¹ represents an oxygen atom or a sulfur atom,
Z² represents an oxygen atom, NOR¹⁶ (in which R¹⁶ represents a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group), CR¹⁷R¹⁸ (in which R¹⁷ represents a halogen atom, a C1-C6 alkyl group, a C1-C6 haloalkyl group, a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group or a C3-C6 cycloalkyloxy group and R¹⁸ represents a hydrogen atom, a halogen atom, a C1-C6 alkyl group or a C1-C6 haloalkyl group) or NNR¹⁹R²⁰ (in which R¹⁹ and R²⁰ independently represent a hydrogen atom, a C1-C6 alkyl group, a C1-C6 haloalkyl group, a C3-C6 alkenyl group, a C3-C6 haloalkenyl group, a C3-C6 alkynyl group or a C3-C6 cycloalkyl group)}].

2. The phenylpyridine compound according to claim 1, wherein X is an oxygen atom.

3. The phenylpyridine compound according to any one of claim 1 or 2, wherein R⁶ is a hydrogen atom.

4. The phenylpyridine compound according to any one of claim 1 to 3, wherein Q is Q1, R¹⁴ is a hydrogen atom and Z¹ is an oxygen atom.

5. The phenylpyridine compound according to claim 4, wherein R¹⁵ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group.

6. The phenylpyridine compound according to any one of claim 1 to 3, wherein Q is Q2 and Z² is NOR¹⁶ (in which R¹⁶ is a hydrogen atom, a C1-C6 alkyl group, a C1-C6 haloalkyl group, a C3-C6 alkenyl group, a C3-C6 haloalkenyl group, a C3-C6 alkynyl group, a C3-C6 haloalkynyl group or a C3-C6 cycloalkyl group).

7. The phenylpyridine compound according to any one of claim 1 to 6, wherein R¹, R⁴ and R⁵ are hydrogen atoms.

8. The phenylpyridine compound according to any one of claim 1 to 6, wherein R¹, R⁴ and R⁵ are hydrogen atoms and R² is a hydrogen atom, a halogen atom, a C1-C6 alkyl group, a C2-C6 alkenyl group, a C3-C6 alkynyl group, a C1-C6 alkoxy group, a C3-C6 alkenyloxy group, a C3-C6 alkynyloxy group, a C1-C6 alkylthio group, a C3-C6 cycloalkyl group, a C3-C6 cycloalkoxy group or a cyano group.

9. The phenylpyridine compound according to any one of claim 1 to 6, wherein R¹, R², R⁴ and R⁵ are hydrogen atoms.

10. The phenylpyridine compound according to any one of claim 1 to 9, wherein each of R⁹ and R¹⁰ is a C1-C6 alkoxy group, a C1-C6 haloalkoxy group, a C3-C6 alkenyloxy group, a C3-C6 haloalkenyloxy group, a C3-C6 alkynyloxy group, a C3-C6 haloalkynyloxy group, a C2-C6 cyanoalkyloxy group or a C3-C6 cycloalkoxy group.

11. The phenylpyridine compound according to any one of claim 1 to 9, wherein each of R⁹ and R¹⁰ is a C1-C4 alkoxy group.

12. The phenylpyridine compound according to any one of claim 1 to 9, wherein R⁹ and R¹⁰ are methoxy groups.

13. The phenylpyridine compound according to any one of claim 1 to 12, wherein R⁷, R⁸ and R¹¹ are hydrogen atoms.

14. The phenylpyridine compound according to any one of claim 1 to 13, wherein W¹―W²=W³―W⁴ is N―CH=CH―CH, CH-N=CH-CH, CH-CH=N―CH or CH―CH=CH―N.

15. A fungicidal composition comprising the phenylpyridine compound according to any one of claim 1 to 14 and a carrier.

16. A method for controlling plant diseases comprising applying of the phenylpyridine compound according to any one of claim 1 to 14 for plants or soils.
